# EUROPEAN PATENT SPECIFICATION

(11) **EP 3 194 612 B1**
(45) Date of publication and mention of the grant of the patent: **03.07.2024**
(21) Application number: 14761762.5
(22) Date of filing: 22.08.2014
(51) Int. Cl.: C12Q 1/6806, C12N 15/10

(54) **METHODS FOR QUANTITATIVE GENETIC ANALYSIS OF CELL FREE DNA**
VERFAHREN ZUR QUANTITATIVEN GENETISCHEN ANALYSE VON ZELLFREIER DNA
PROCÉDÉS PERMETTANT D'EFFECTUER L'ANALYSE GÉNÉTIQUE QUANTITATIVE D'ADN EXEMPT DE CELLULES

(43) Date of publication of application: 26.07.2017
(73) Proprietor: Resolution Bioscience, Inc., Kirkland, WA 98033 (US)
(72) Inventor: RAYMOND, Christopher K., Kirkland, WA 98033 (US); LIM, Lee P., Kirkland, WA 98033 (US); ARMOUR, Christopher D., Kirkland, WA 98033 (US)
(74) Representative: Forresters IP LLP
(86) International application number: PCT/US2014/052317
(87) International publication number: WO 2016/028316

(56) References cited:
- WO-A1-2014/071295
- WO-A1-2014/093330
- WO-A1-2014/122288
- US-A1- 2013 288 915
- HOEIJMAKERS WIETEKE A M ET AL: "Linear amplification for deep sequencing.", NATURE PROTOCOLS, vol. 6, no. 7, July 2011 (2011-07), pages 1026-1036, XP002733471, ISSN: 1750-2799
- YEGNASUBRAMANIAN SRINIVASAN: "Preparation of fragment libraries for next-generation sequencing on the applied biosystems SOLiD platform.", NIH Public Access Author Manuscript , 2013, pages 1-17, XP002733472, Retrieved from the Internet: URL:http://www.ncbi.nlm.nih.gov/pmc/articl es/PMC3991121/ [retrieved on 2014-12-11]
- MCKERNAN KEVIN JUDD ET AL: "Sequence and structural variation in a human genome uncovered by short-read, massively parallel ligation sequencing using two-base encoding.", GENOME RESEARCH, vol. 19, no. 9, September 2009 (2009-09), pages 1527-1541, XP002733473, ISSN: 1549-5469
- MANO HIROYUKI: "Non-solid oncogenes in solid tumors: EML4-ALK fusion genes in lung cancer.", CANCER SCIENCE, vol. 99, no. 12, December 2008 (2008-12), pages 2349-2355, XP002733474, ISSN: 1349-7006

## Description

### BACKGROUND

### Technical Field

The invention relates generally to methods for the quantitative genetic analysis of cell free DNA (cfDNA). In particular, the present invention relates to methods for the genetic characterization and analysis of cfDNA.

### Description of the Related Art

It is becoming increasing clear that most, if not all, of the most common human cancers are diseases of the human genome. The emerging picture is that somatic mutations accumulate during an individual's lifetime, some of which increase the probability that the cell in which they are harbored can develop into a tumor (Vogelstein et al., Science 339(6127): 1546-1558 (2013)). With just the wrong combination of accumulated mutational events, a precancerous growth loses constraints that keep uncontrolled proliferation in check and the resulting cell mass becomes a cancer. The constellations of mutations that are necessary and sufficient to cause cancer are often collectively referred to as "driver mutations." One of the themes that have emerged from recent and intensive molecular analysis is that cancer, once thought of as a single, tissue-specific disease, is in fact a group of related diseases, each with a unique molecular pathology. The human genome project laid the groundwork for genome-wide analysis of cancers.

For example, the introduction of next-generation sequencing technologies (2004-present) has accelerated the discovery pace of causal genomic factors that underlie the diagnosis of NSCLC, making it clear that NSCLC is really a family of related diseases, each of which may be responsive to a different targeted therapy.

The art lacks reliable and robust molecular analysis methods for the analysis of genetic diseases. Traditionally, molecular diagnostics have consisted of antibody-based tests (immunohistochemistry), in-situ hybridization with DNA probes (fluorescence in situ hybridization), and hybridization or PCR-based tests that query specific nucleotide sequences. Until recently, DNA sequencing as a molecular diagnostic tool has been generally limited to the coding exons of one or two genes. While DNA sequencing has been used in the diagnosis and treatment of solid cancers, one of the most significant drawbacks of these methods is that they require direct access to tumor tissues. Such material is often difficult to obtain from the initial biopsy used to diagnose the disease and virtually impossible to obtain in multiple repetitions over time. Similarly, biopsies are not possible in patients with inaccessible tumors and not practical in individuals suffering from metastatic disease.

Thus, the vast potential of molecular diagnostics for genetic diseases; fetal testing; paternity testing; predicting response to drug treatment; diagnosing or monitoring a medical condition; mendelian disorders; genetic mosaicism; pathogen screening; microbiome profiling; and organ transplant monitoring; has yet to be realized. To date, existing molecular diagnostics approaches lack efficient solutions to clone and amplify individual DNA molecules, as well as solutions to efficiently target sequencing to specific genomic loci, with sensitivity sufficient to discriminate true positive test results from false positive signals that arise during sample processing.

Document WO2014093330 discloses a method for generating a tagged DNA library comprising treating fragmented DNA with end-repair enzymes to generate fragmented end-repaired DNA; and ligating a random nucleic acid tag sequence, and optionally a sample code sequence and/or a PCR primer sequence to the fragmented end-repaired DNA to generate the tagged DNA library.

### BRIEF SUMMARY

The invention is a method for targeted genetic analysis of cell-free DNA (cfDNA) as defined in the appended claims. The compositions provided herein are for illustrative purposes and do not fall under the scope of the claimed invention. Any other aspects, configurations, instances or embodiments set forth herein not falling within the scope of the claims are for information only.

The disclosure relates generally to compositions and methods for improved compositions and methods for the genetic analysis of cfDNA.

In accordance with the invention, a method for targeted genetic analysis of cell-free DNA (cfDNA) is provided as recited by the claims, comprising: treating cfDNA with one or more end-repair enzymes to generate end-repaired cfDNA; ligating one or more adaptors to each end of the end-repaired cfDNA to generate a cfDNA library; amplifying the cfDNA library to generate cfDNA library clones; determining the number of genome equivalents in the cfDNA clone library; and performing a quantitative genetic analysis of one or more target genetic loci in the cfDNA library clones.

In a particular embodiment, the method further comprises isolating cfDNA from a biological sample of a subject.

In an additional embodiment, the cfDNA is isolated from a biological sample selected from the group consisting of: amniotic fluid, blood, plasma, serum, semen, lymphatic fluid, cerebral spinal fluid, ocular fluid, urine, saliva, stool, mucous, and sweat.

In a certain embodiment, the one or more adaptors comprise a plurality of adaptor species.

In a particular embodiment, the one or more adaptors each comprise a primer binding site for amplification of the cfDNA library.

In a further embodiment, the one or more adaptors each comprise one or more unique read codes.

In an additional embodiment, the one or more adaptors each comprise one or more sample codes for sample multiplexing.

In another embodiment, the one or more adaptors each comprise one or more sequences for DNA sequencing.

In a particular embodiment, qPCR is performed on the cfDNA clone library and a qPCR measurement is compared to standards of known genome equivalents to determine the genome equivalents of the cfDNA clone library.

In another particular embodiment, qPCR is performed with a primer that binds to an Alu sequence and a primer that binds to a sequence in an adaptor.

In a certain embodiment, the quantitative genetic analysis is performed on a plurality of genetic loci in the cfDNA library clones.

In a further embodiment, the quantitative genetic analysis is performed on a plurality of genetic loci in a plurality of cfDNA clone libraries.

The quantitative genetic analysis comprises hybridizing one or more capture probes to a target genetic locus to form capture probe-cfDNA clone complexes.

The quantitative genetic analysis comprises isolating the capture probe-cfDNA clone complexes.

The quantitative genetic analysis comprises amplification of the cfDNA clone sequence in the isolated hybridized capture probe-cfDNA clone complexes.

The quantitative genetic analysis comprises DNA sequencing to generate a plurality of sequencing reads.

The quantitative genetic analysis comprises bioinformatic analysis of the plurality of sequencing reads.

In a particular embodiment, bioinformatics analysis is used: to quantify the number of genome equivalents analyzed in the cfDNA clone library; to detect genetic variants in a target genetic locus; to detect mutations within a target genetic locus; to detect genetic fusions within a target genetic locus; and to measure copy number fluctuations within a target genetic locus.

In an additional embodiment, the subject does not have a genetic disease.

In a certain embodiment, the subject has not been diagnosed with a genetic disease.

In another certain embodiment, the subject has been diagnosed with a genetic disease.

In another embodiment, the quantitative genetic analysis is used to identify or detect one or more genetic lesions that cause or associated with the genetic disease.

In a certain embodiment, the genetic lesion comprises a nucleotide transition or transversion, a nucleotide insertion or deletion, a genomic rearrangement, a change in copy number, or a gene fusion.

In a particular embodiment, the genetic lesion comprises a genomic rearrangement that fuses the 3' coding region of the ALK gene to another gene.

In a particular embodiment, the 3' coding region of the ALK gene is fused to the EML4 gene.

In another embodiment, the genetic disease is cancer.

In a further embodiment, the subject is pregnant.

In an additional embodiment, the quantitative genetic analysis is used to identify or detect one or more genetic variants or genetic lesions of one or more target genetic loci in fetal cfDNA.

In a particular embodiment, the subject is a transplant recipient.

In an additional embodiment, the quantitative genetic analysis is used to identify or detect donor cfDNA in the subject.

For illustrative purposes, a method (not claimed) of predicting, diagnosing, or monitoring a genetic disease in a subject is provided, comprising: isolating or obtaining cfDNA from a biological sample of a subject; treating the cfDNA with one or more end-repair enzymes to generate end-repaired cfDNA; ligating one or more adaptors to each end of the end-repaired cfDNA to generate a cfDNA library; amplifying the cfDNA library to generate a cfDNA clone library; determining the number of genome equivalents in the cfDNA clone library; and performing a quantitative genetic analysis of one or more target genetic loci associated with the genetic disease in the cfDNA clone library, wherein the identification or detection of one or more genetic lesions in the one or more target genetic loci is prognostic for, diagnostic of, or monitors the progression of the genetic disease.

In an additional embodiment, the cfDNA is isolated from a biological sample selected from the group consisting of: amniotic fluid, blood, plasma, serum, semen, lymphatic fluid, cerebral spinal fluid, ocular fluid, urine, saliva, stool, mucous, and sweat.

In a certain embodiment, the genetic lesion comprises a nucleotide transition or transversion, a nucleotide insertion or deletion, a genomic rearrangement, a change in copy number, or a gene fusion.

In a particular embodiment, the genetic lesion comprises a genomic rearrangement that fuses the 3' coding region of the ALK gene to another gene.

In a further embodiment, the 3' coding region of the ALK gene is fused to the EML4 gene.

In a particular embodiment, the genetic disease is cancer.

For illustrative purposes, a companion diagnostic (not claimed) for a genetic disease is provided comprising: isolating or obtaining cfDNA from a biological sample of a subject; treating the cfDNA with one or more end-repair enzymes to generate end-repaired cfDNA; ligating one or more adaptors to each end of the end-repaired cfDNA to generate a cfDNA library; amplifying the cfDNA library to generate a cfDNA clone library; determining the number of genome equivalents in the cfDNA clone library; and performing a quantitative genetic analysis of one or more biomarkers associated with the genetic disease in the cfDNA clone library, wherein detection of, or failure to detect, at least one of the one or more biomarkers indicates whether the subject should be treated for the genetic disease.

In a particular embodiment, the cfDNA is isolated from a biological sample selected from the group consisting of: amniotic fluid, blood, plasma, serum, semen, lymphatic fluid, cerebral spinal fluid, ocular fluid, urine, saliva, stool, mucous, and sweat.

In an additional embodiment, the biomarker is a genetic lesion.

In a particular embodiment, the genetic lesion comprises a nucleotide transition or transversion, a nucleotide insertion or deletion, a genomic rearrangement, a change in copy number, or a gene fusion.

In an additional embodiment, the genetic lesion comprises a genomic rearrangement that fuses the 3' coding region of the ALK gene to another gene.

In a further embodiment, the 3' coding region of the ALK gene is fused to the EML4 gene.

In a certain embodiment, the genetic disease is cancer.

### BRIEF DESCRIPTION OF THE SEVERAL VIEWS OF THE DRAWINGS

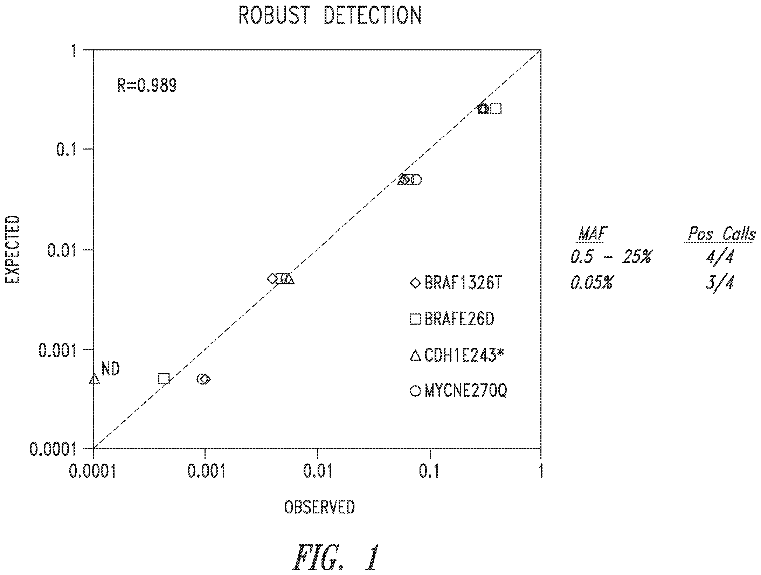

**Figure 1** shows the expected versus observed variant frequencies as a function of admix dilution in the absence of unique read filtering. In the absence of unique read filtering, random base changes at these four selected positions occurred with measurable, non-zero frequencies; thus, demonstrating a lack of sensitivity to detect the particular single nucleotide variants (SNV).

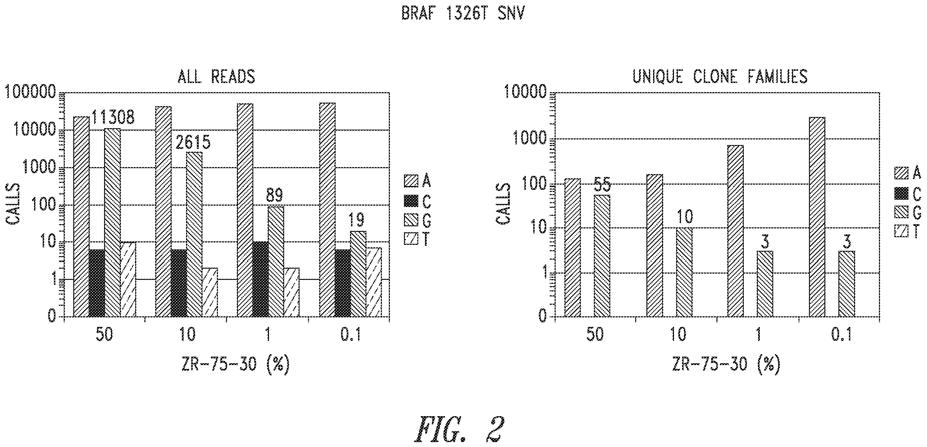

**Figure 2** shows that unique read filtering performed on the data generated in Figure 1. The left hand panel shows the data from Figure 1 on the BRAF I326T SNV without unique read filtering. The right hand panel shows that using unique read filtering of the same data increased the assay sensitivity and allowed the discrimination of true signal from error-prone noise.

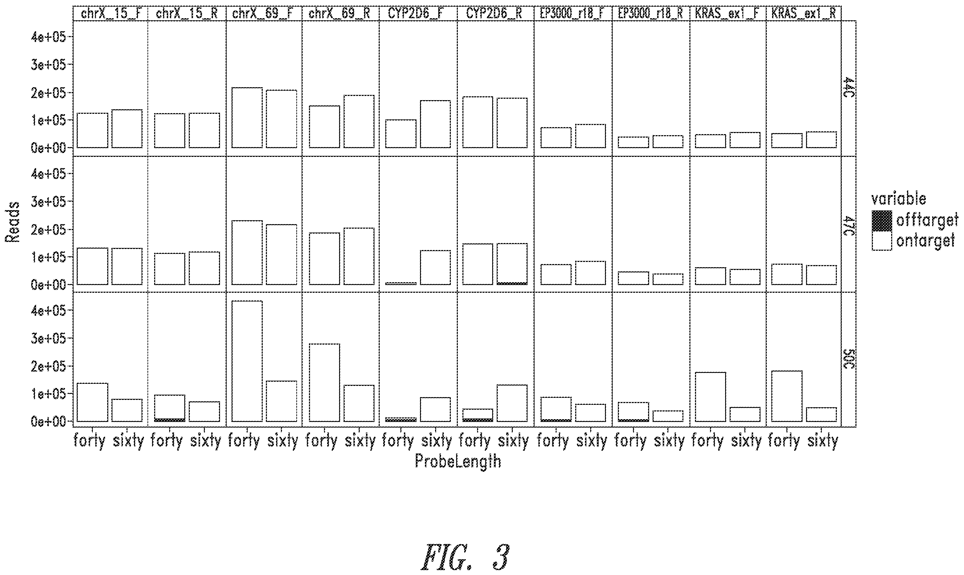

**Figure 3** shows capture probe performance as a function of length and wash temperature. The y-axis shows the total number of reads associated with each capture probe. The bars in the bar chart are broken into two categories, where open bars correspond to on-target reads that align to the intended capture probe targets and solid bars show off-target reads that are associated with a capture probe but that map to unintended regions of the genome. Overall, the 40-mer and 60-mer capture probes perform substantially the same with 44° C and 47° C washes. With the 50° C wash, the 40-mer capture probes perform erratically. These data validate the use of 40-mer capture probes at wash temperatures in the range of about 44° C to about 47° C.

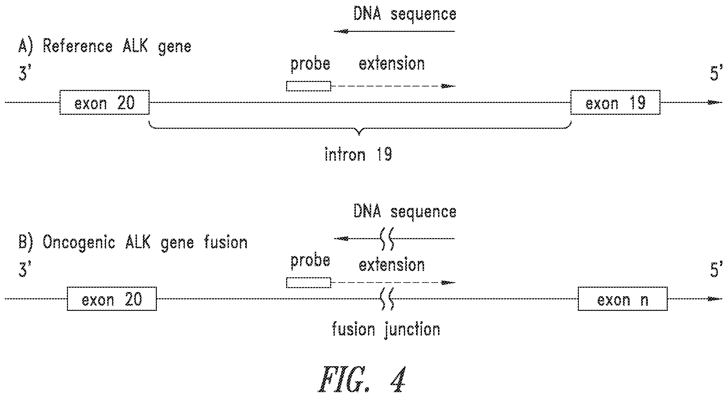

**Figure 4** shows a schematic for the targeted and oriented sequencing of intron 19 of the ALK gene. A) In the "wild-type" reference sequence, antisense-oriented ALK capture probes identify sequences from intron 19. B) In the case of pathogenic fusion genes, some ALK capture probes will identify junction sequences associated with the gene fusion event.

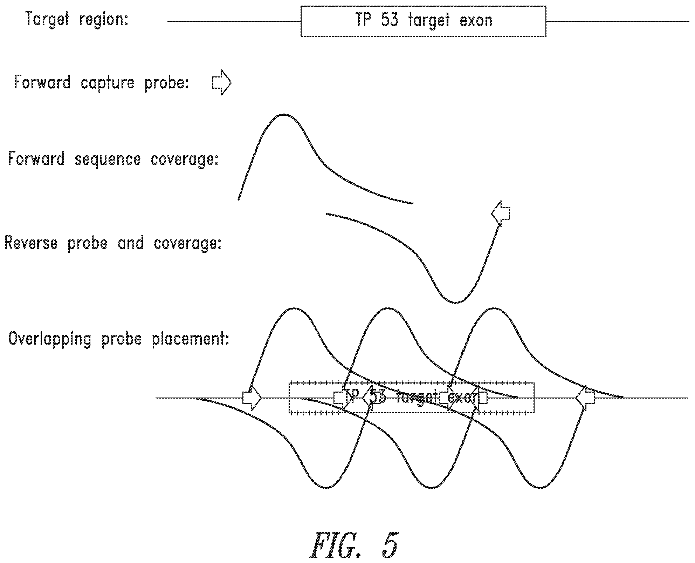

**Figure 5** shows a schematic for high density capture probe placement for complete sequencing of target regions. Each capture probe captures a collection of sequences that provide cumulative coverage at each base position. Here, coverage is represented by a line, and the amplitude of the line denotes depth of coverage derived from a particular capture probe. Overlapping coverage from adjacent capture probes provides complete sequencing of target regions in both possible directions. In addition, the head-to-head placement of opposite strand capture probes ensures that all capture probe binding sites are sequenced.

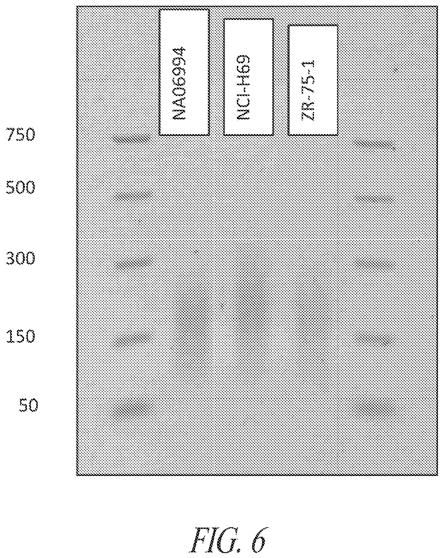

**Figure 6** shows a representative example of the size distribution of fragmented DNA used in library construction.

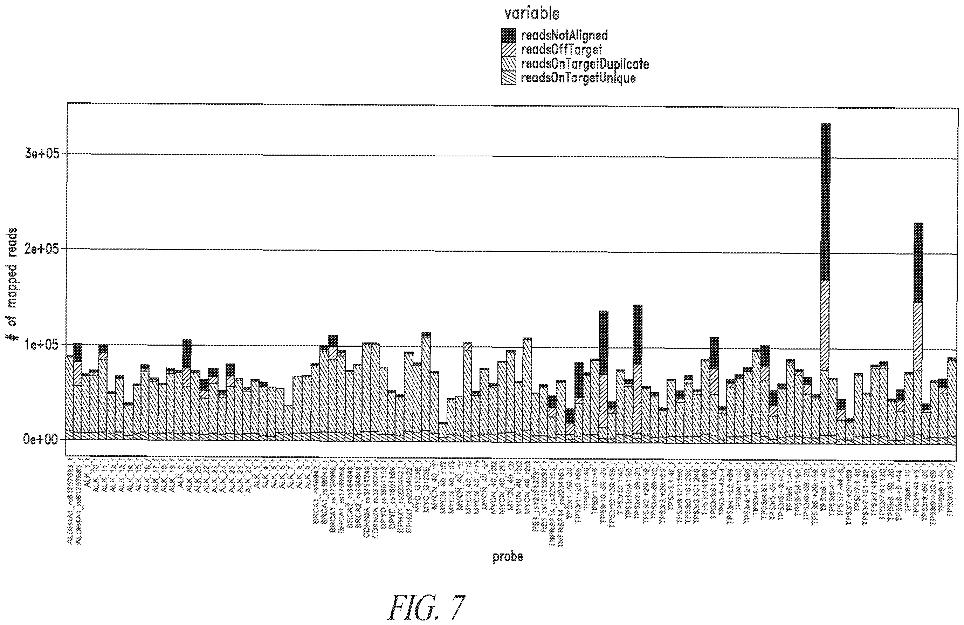

**Figure 7** shows the performance of high-density 40-mer capture probes in a representative experiment. The y-axis shows the total number of reads, which are broken out as on-target reads, off-target reads, and unmappable reads. The x-axis enumerates each of the 105 capture probes used in this experiment for sequence capture.

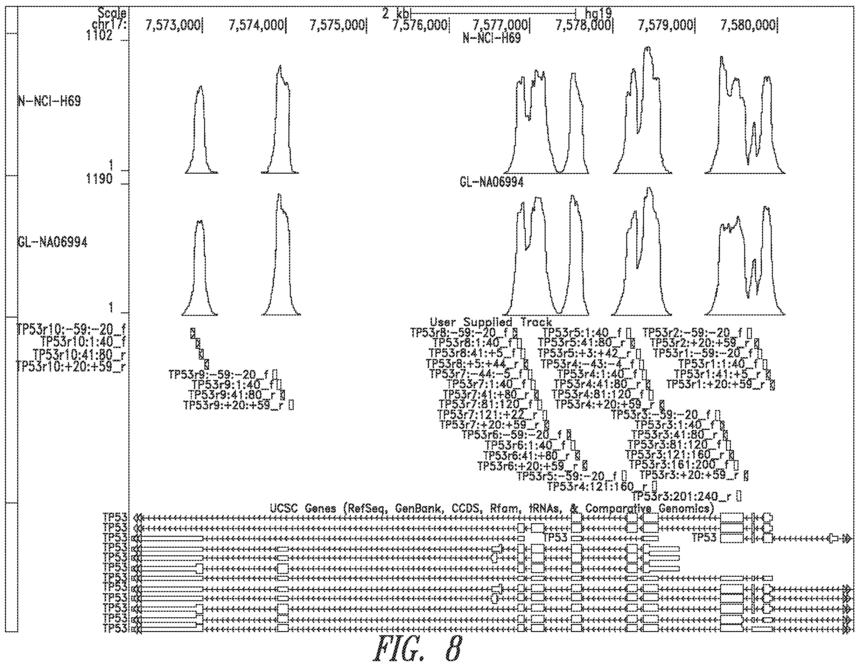

**Figure 8** shows a representative example of the cumulative coverage of a target region using high density 40-mer capture probes. Shown here is the cumulative coverage of TP53 coding exons.

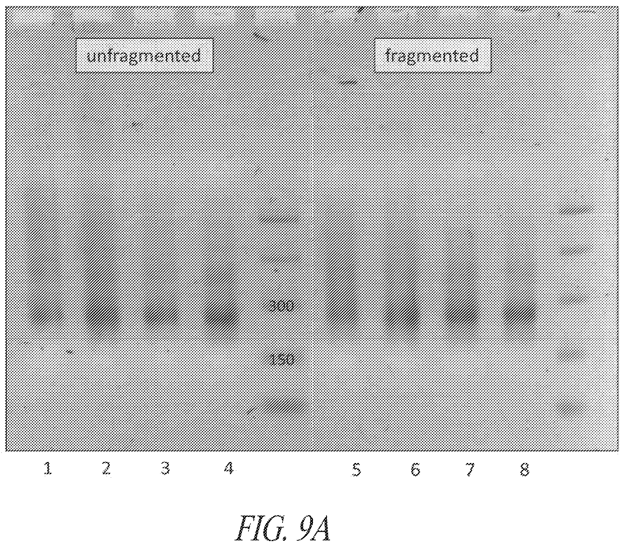

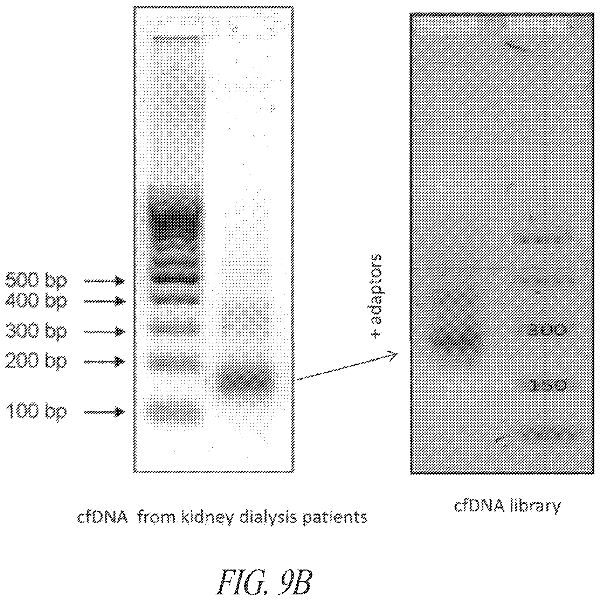

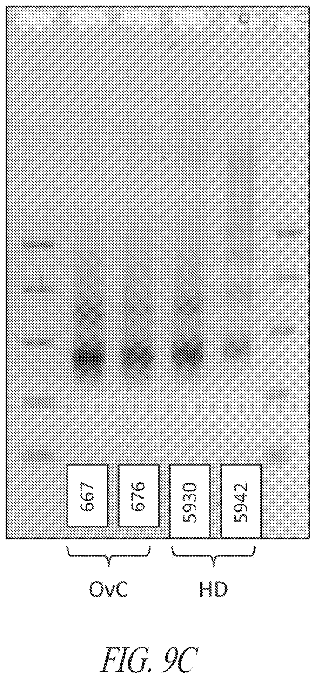

**Figure 9A** shows a representative example of the size distribution of cell-free DNA (cfDNA) libraries. The dominant band is consistent with a collection of 170+10 bp fragments ligated to 90 bp of adaptors. **Figure 9B** shows a published gel image of cfDNA and a representative cfDNA library generated using the methods diclosed and/or contemplated herein. The qualitative "ladder" appearance is conserved in the library, but the library is shifted to higher mass by the addition of 90 bp of adaptor sequences. **Figure 9C** shows a representative example of genomic, plasma-derived cfDNA libraries from Ovarian cancer patients (OvC) and "healthy donors" (HD).

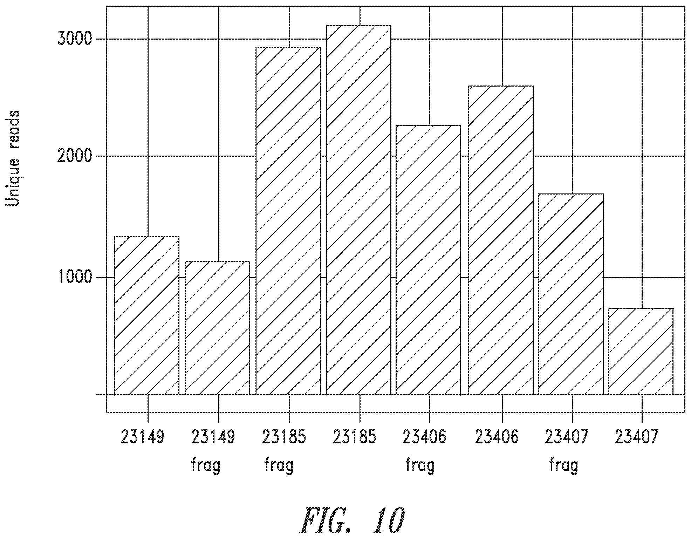

**Figure 10** shows the unique read counts across eight cfDNA libraries derived from four plasma samples. Fragmentation (frag) prior to library construction with this sample 23407 increased the library yield by more than two-fold.

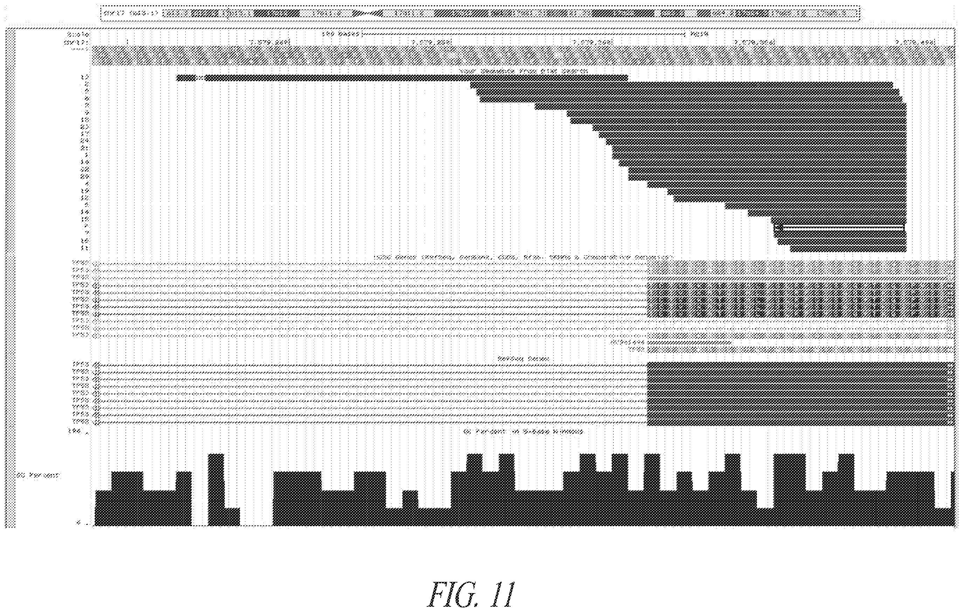

**Figure 11** shows th representative read coverage of cfDNA across a region of the TP53 gene. Twenty four 131 bp reads captured by the "TP53_NM_000546_chr17:7579351:region_3:280nt:41:80:r" capture probe (SEQ ID NO:201) were chosen at random and aligned using the BLAT algorithm within the UCSC genome browser. Twenty one reads map to the target region, and they do so in a pattern of overlapping coverage. The probe used to capture these reads is marked with an arrow.

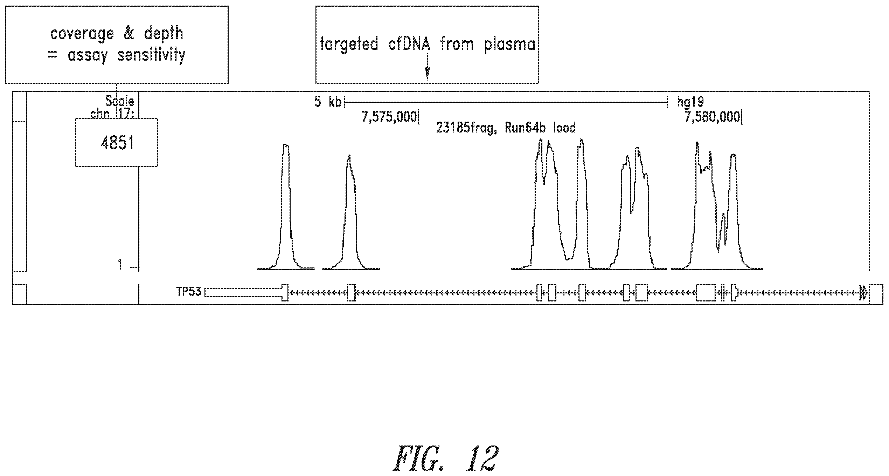

**Figure 12** shows an overview of targeted DNA sequencing of the coding regions of the TP53 gene from a cfDNA genomic library. The coverage (horizontal axis) extends across all 10 coding regions and includes intronic regions involved in mRNA splicing. The sequencing depth (vertical axis) reaches a maximum of 4851 and is uniform across all coding exons.

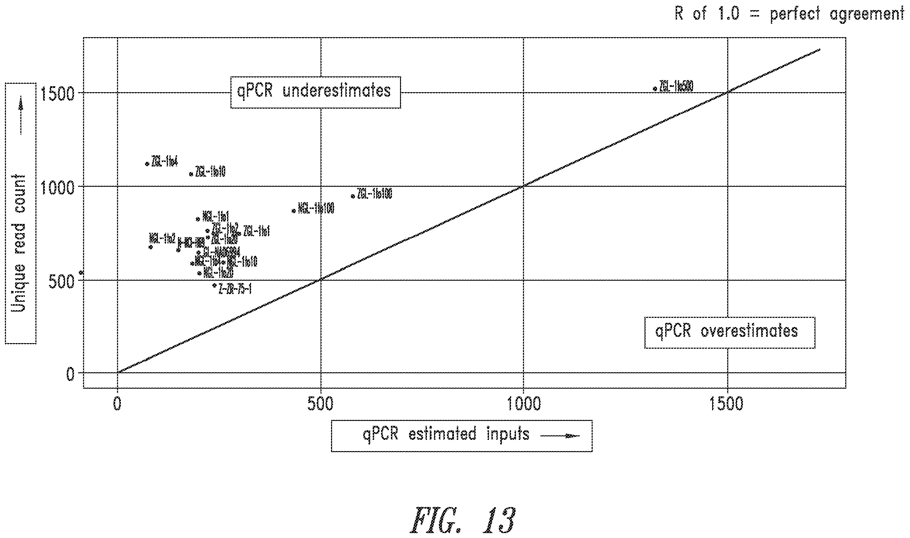

**Figure 13** shows a plot of unique read counts versus qPCR estimated genome equivalents in an ACA2-based assay. qPCR measurements are shown on the X-axis versus read counts on the Y-axis. Perfect agreement between these measurements is shown as the diagonal. There is very poor, if any, correlation between measurements, especially at lower genomic inputs. These data show that the ACA2-based qPCR assay chronically underestimates library complexity and is inadequate for measuring genome equivalents.

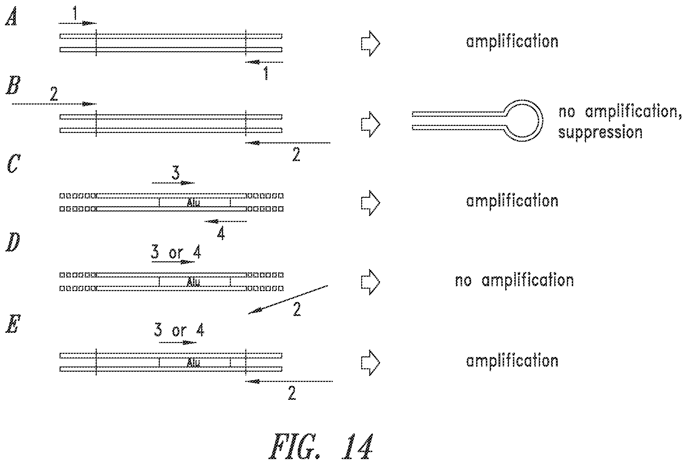

**Figure 14** shows a schematic of the core elements of a qPCR genome equivalent measurement assay that couples an genomic repeat specific primer (e.g., Alu) and a long adaptor-specific primer. (A) Standard library amplification using a single, 25 nt primer named ACA2 (primer 1). (B) Longer, 58 nt versions of the ACA2 primer (primer 2) do not amplify genomic libraries because of stem-loop suppression. (C) Forward and reverse primers directed to a consensus human Alu repeat element (primers 3 and 4) recognize 1000's of loci and readily amplify genomic DNA. (D) A single Alu primer alone, either forward or reverse (primer 3 or primer 4), coupled with the long ACA2 primer (primer 2) do not amplify genomic DNA. (E) The same primer pair as in (D) readily amplifies genomic cfDNA library clones that contain Alu sequences.

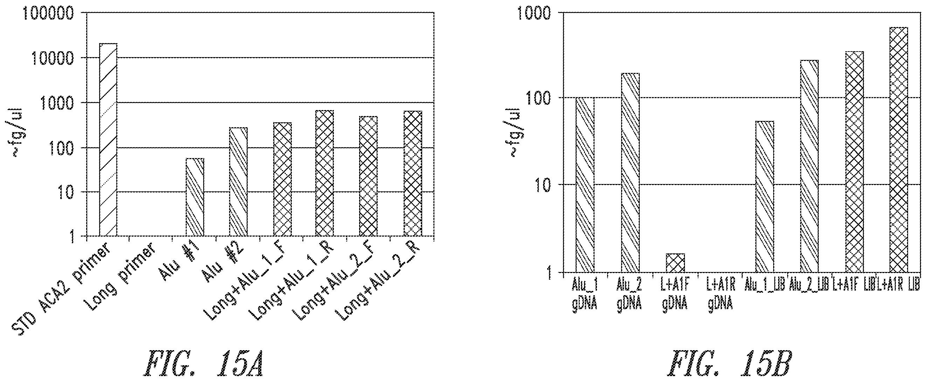

**Figure 15** shows proof-of-concept data for an Alu plus adaptor-based qPCR assay of genome equivalents. (A) Amplification of 10 pg of a standard genomic library with various PCR primers. The x-axis specifies PCR primers used for amplification and the Y-axis (log scale) indicates the PCR signal measured in units of fg/µL. The standard ACA2 primer produced a strong signal, as expected. The ACA2 long primer failed to produce signal owing to PCR suppression. The two Alu primer pairs both produced signal at 1% the amount of ACA2, suggesting that 1% of clones possess an amplifiable Alu sequence. The combination of any Alu primer with the long ACA2 primer also produced signal in ~1% of clones. (B) Validation against 10 pg of genomic DNA (left four samples) or 10 pg of library DNA (right four clones). Alu primer pairs amplify comparable signal from genomic DNA or a genomic library. In contrast, primer pairs consisting of an Alu primer and a long ACA2 primer amplify genomic DNA poorly (L+A1F) or not at all (L+A1R). These same pairs exhibit amplification of library that slightly exceeds the signal from Alu primer pairs.

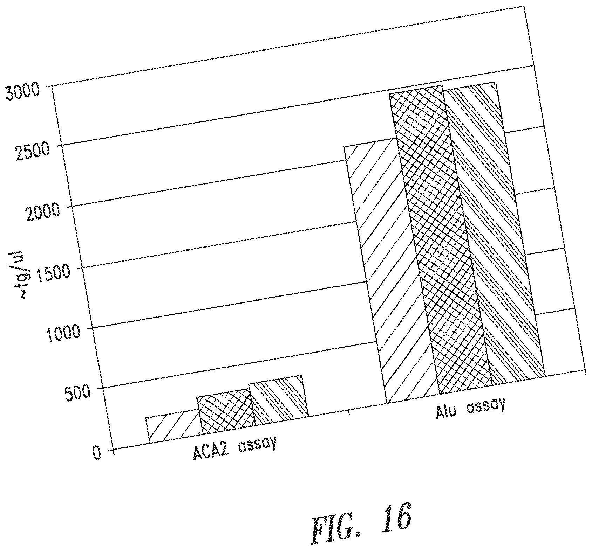

**Figure 16** shows a direct comparison of ACA2 primer qPCR assay with the Alu-ACA2 long-primer qPCR assay. The Alu ACA2 long-primer qPCR assay shows an 8-fold increase in detectable genome equivalents, which is more consistent with unique read counts derived from sequencing data.

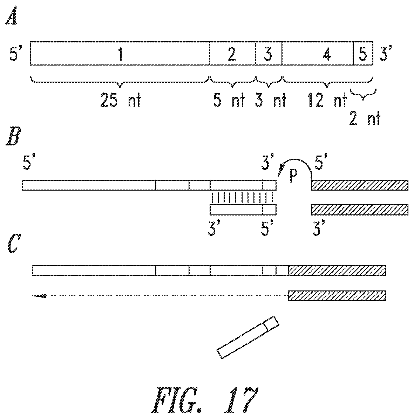

**Figure 17** shows a representative example of adaptor structure and function for high sensitivity, quantitative genetic assays that provide accurate determinations of genome equivalents analyzed. (A) Fine structure of the adaptor ligation strand. Details relating to each numbered element are provided in Example 4. (B) The duplex formed between 45 nt ligation strands and 12 nt partner oligo strand creates a blunt-end ligation substrate compatible with end-repaired cfDNA fragments (solid bars). (C) Following ligation, the complement to the ligation strand is created by a DNA polymerase-mediated fill-in reaction.

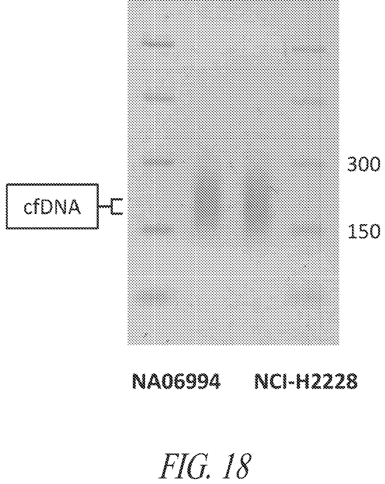

**Figure 18** shows a representative example of the size distribution of two DNA samples (NA06994 & NCI-H2228) processed to mimic cfDNA.

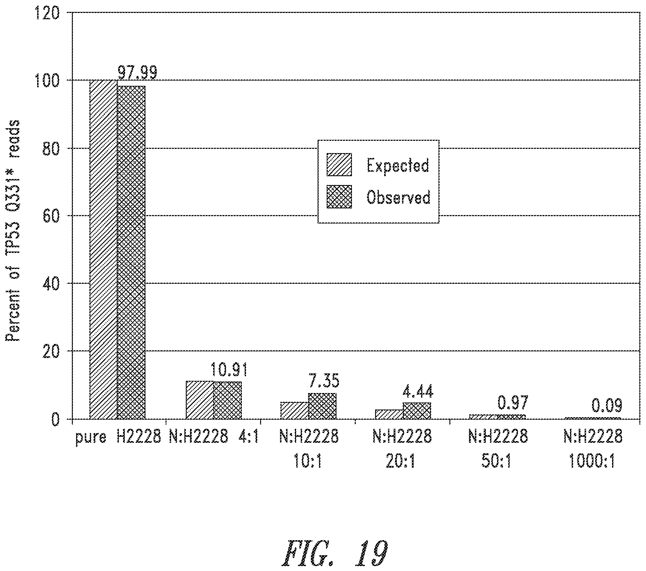

**Figure 19** shows a representative example of the sensitivity of detection of the TP53 point mutation Q331 * in tumor sample DNA (H2228) admixed with normal genomic DNA (N). The most sensitive detection corresponds to ~ 1 mutant copy of TP53 among 1000 normal copies of the gene.

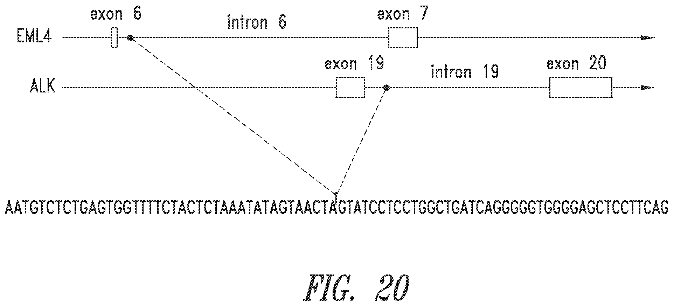

**Figure 20** shows the precise determination of the junction sequence for the EML4-ALK fusion gene harbored in cell line NCI-H2228 using the compositions and methods contemplated herein.

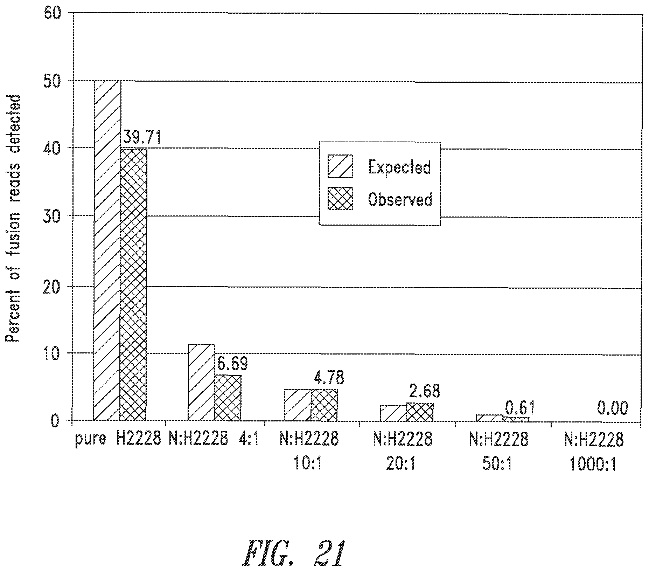

**Figure 21** shows the detection of the EML4-ALK fusion gene tumor sample DNA (H2228) admixed with normal genomic DNA (N). Because the fusion is present as a heterozygote in the NCI-H2228 cell line, the most sensitive detection corresponds to one gene fusion among ~100 normal chromosomal copies of the ALK gene (50 genome equivalents).

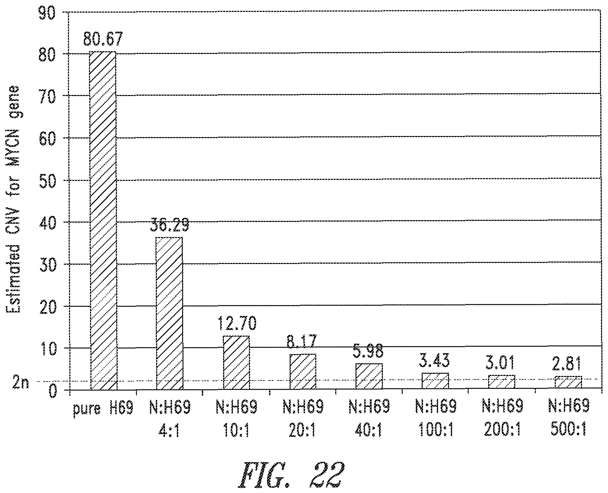

**Figure 22** shows the detection of the MYCN gene amplification in admixtures of cell line NCI-H69 (H69) diluted into normal human DNA (N). The threshold value of two normal diploid copies is shown as a dashed red line.

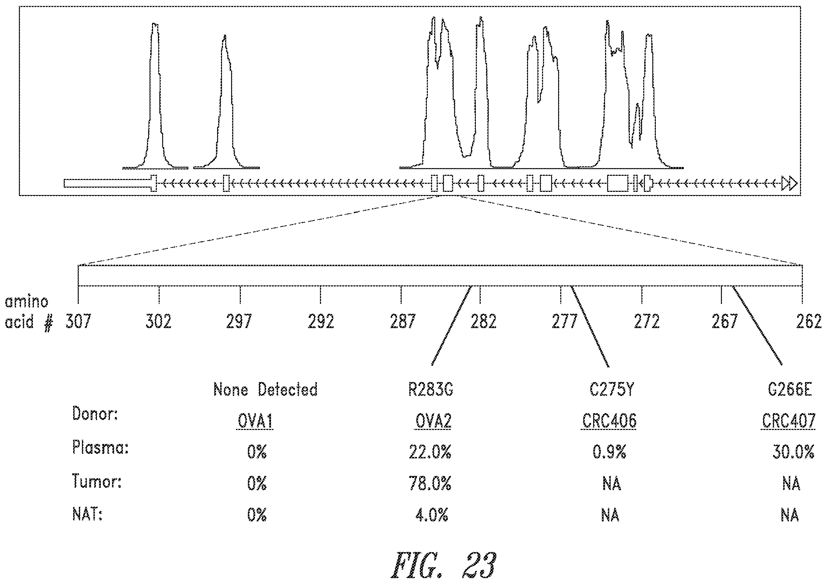

**Figure 23** shows the DNA mutations detected in the TP53 gene of three different cancer patients. The canonical gene model is shown at the top of the figure. The peaks represent DNA sequence coverage (X-axis) and depth (Y-axis). Sequencing depth was >4000 genome equivalents for all sample analyzed. An expanded view of exon 7 below the gene model shows where all detected mutations were localized. The frequency of mutant detection in cfDNA (plasma), tumor tissue, and normal adjacent tissue is shown, where available (NA - not available). OVA1 and OVA2 are ovarian cancer patients; CRC406 and CRC407 are colorectal cancer patients. No mutations in TP53 were found in any of the OVA1 samples.

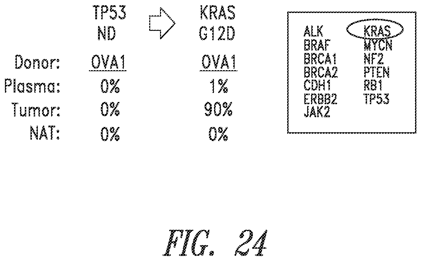

**Figure 24** shows the DNA sequencing of a larger, thirteen gene panel (boxed). The sequencing identified a KRAS mutation in cfDNA and tumor from ovarian cancer patient OVA1.

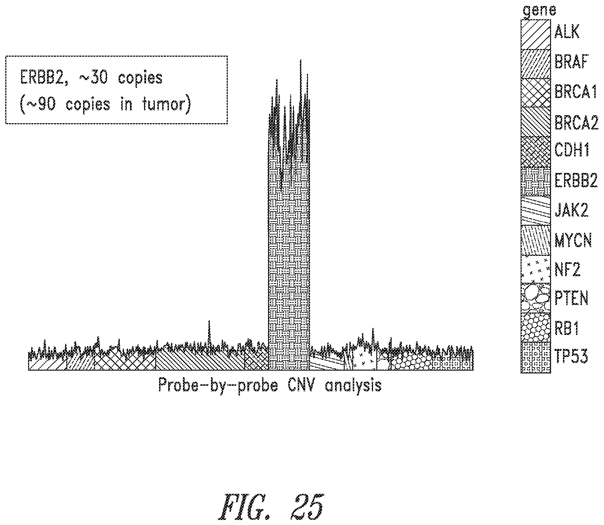

**Figure 25** shows the DNA sequencing of a larger, twelve gene panel. The sequencing identified an ERBB2 gene amplification in the plasma of colorectal cancer patient CRC407.

### DETAILED DESCRIPTION

### A. OVERVIEW

The present invention provides a method for targeted genetic analysis of cell-free DNA (cfDNA) as defined by the claim set. Also described herein for illustrative purposes are compositions for the quantitative genetic analysis of the genetic state of an individual using cell-free DNA (cfDNA); such compositions are not claimed.

As used herein, the term "genetic state" refers to the sequence of one or more target genome sequences in the genome in relation to a non-causal normal sequence or in relation to a sequence that is causal for a genetic condition or disease. In one embodiment, analyzing the genetic state refers to identifying, quantifying, or monitoring a genetic variant in a target genetic locus, wherein the variant varies with respect to a reference sequence (*e.g.*, a normal or mutated sequence). The present inventors have provided solutions to the molecular diagnostic problems of genetic conditions or diseases associated with lack of sensitivity to discriminate true positives from false positives, inefficient cloning and amplification of individual DNA molecules, and inefficient targeted sequencing to specific genomic loci. The solutions contemplated herein comprise compositions and methods for reliable and robust quantitative genetic analysis with sensitivity sufficient to discriminate true positive test results from false positive signals that arise during sample processing.

Next-generation sequencing technology has afforded the opportunity to add broad genomic surveys to molecular diagnosis in a variety of scenarios including cancers, fetal diagnostics, paternity testing, pathogen screening and organ transplant monitoring. In the context of genetic diseases, next-generation sequencing information is being used in a clinical setting to identify mutations within genes that are likely to alter gene function, to identify the gain or loss of genetic material within cells, and to identify genomic rearrangements that are not found in normal, healthy cells. The results of these broad diagnostic surveys are often used to guide patient treatment.

However, the potential benefits of DNA sequencing in diagnosis and treatment of the genetic state of an individual or genetic conditions or diseases is outweighed by the need to directly access affected tissues to obtain samples. Such material is often difficult to obtain from the initial biopsy used to diagnose the disease and virtually impossible to obtain in multiple repetitions over time. Similarly, in cancer patients, biopsies are not possible in patients with inaccessible tumors and not practical in individuals suffering from metastatic disease. In contrast, the present inventors' approach derives from the fact that all tissues require access to the vasculature to survive and as a consequence these masses deposit DNA into bodily fluids. One major depot of bodily fluid in which the DNA of diseased cells is found is the plasma of human blood.

In contrast to testing methods that rely on shallow, genome-wide sequence coverage, molecular diagnostics contemplated herein for the genetic state of an individual; genetic diseases; mendelian disorders; genetic mosaicism; fetal testing; paternity testing; predicting response to drug treatment; diagnosing or monitoring a medical condition; pathogen screening; microbiome profiling; and organ transplant monitoring leverage the availability of cfDNA to provide deep sequence coverage of select target genes. In addition, the cfDNA-based cancer diagnostics contemplated herein possess the ability to detect a variety of genetic changes including somatic sequence variations that alter protein function, large-scale chromosomal rearrangements that create chimeric gene fusions, and copy number variation that includes loss or gain of gene copies. Using the contemplated compositions and methods, these changes are detectable and quantifiable in the face of significant dilution, or admixture, of normal sequences within cfDNA that are contributed by the normal turnover processes that happen within healthy tissues. The compositions and methods contemplated herein also successfully address the major challenges associated with detecting rare genetic changes causal of disease; namely, that cfDNA is highly fragmented, that cfDNA levels vary substantially between different individuals, and that the degree of admixture of diseased versus normal sequences is highly variable among patients, even within individuals suffering from the same molecular disease and stage.

In various embodiments, compositions (not claimed) and methods for genetic analysis of comprise interrogating the DNA fraction within biological fluid samples and stool samples. The methods contemplated herein provide a novel comprehensive framework address molecular genetic analysis using cfDNA available from a variety of biological sources. Cloning of purified cfDNA introduces tagged cfDNA sequences that inform downstream analysis and enable amplification of the resulting clone libraries. Hybrid capture with target specific oligonucleotides is used to retrieve specific sequences for subsequent analysis. Independent measurements of the number of genomes present in the library are applied to each sample, and these assays provide a means to estimate the assay's sensitivity. The assays contemplated herein provide reliable, reproducible, and robust methods for the analysis, detection, diagnosis, or monitoring of genetic states, conditions, or disease.

The practice of particular embodiments of the invention will employ, unless indicated specifically to the contrary, conventional methods of chemistry, biochemistry, organic chemistry, molecular biology, microbiology, recombinant DNA techniques, genetics, immunology, and cell biology that are within the skill of the art, many of which are described below for the purpose of illustration. Such techniques are explained fully in the literature. See, *e.g.,* Sambrook, et al., Molecular Cloning: A Laboratory Manual (3rd Edition, 2001); Sambrook, et al., Molecular Cloning: A Laboratory Manual (2nd Edition, 1989); Maniatis et al., Molecular Cloning: A Laboratory Manual (1982); Ausubel et al., Current Protocols in Molecular Biology (John Wiley and Sons, updated July 2008); Short Protocols in Molecular Biology: A Compendium of Methods from Current Protocols in Molecular Biology, Greene Pub. Associates and Wiley-Interscience; Glover, DNA Cloning: A Practical Approach, vol. I & II (IRL Press, Oxford, 1985); Anand, Techniques for the Analysis of Complex Genomes, (Academic Press, New York, 1992); Transcription and Translation (B. Hames & S. Higgins, Eds., 1984); Perbal, A Practical Guide to Molecular Cloning (1984); and Harlow and Lane, Antibodies, (Cold Spring Harbor Laboratory Press, Cold Spring Harbor, N.Y., 1998).

### B. DEFINITIONS

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred embodiments of compositions, methods and materials are described herein. For the purposes of the present invention, the following terms are defined below.

The articles "a, " "an, " and "the" are used herein to refer to one or to more than one (*i.e.* to at least one) of the grammatical object of the article. By way of example, "an element" means one element or more than one element.

The use of the alternative (*e.g.,* "or") should be understood to mean either one, both, or any combination thereof of the alternatives.

The term "and/or" should be understood to mean either one, or both of the alternatives.

As used herein, the term "about" or "approximately" refers to a quantity, level, value, number, frequency, percentage, dimension, size, amount, weight or length that varies by as much as 15%, 10%, 9%, 8%, 7%, 6%, 5%, 4%, 3%, 2% or 1% to a reference quantity, level, value, number, frequency, percentage, dimension, size, amount, weight or length. In one embodiment, the term "about" or "approximately" refers a range of quantity, level, value, number, frequency, percentage, dimension, size, amount, weight or length ± 15%, ± 10%, ± 9%, ± 8%, ± 7%, ± 6%, ± 5%, ± 4%, ± 3%, ± 2%, or ± 1% about a reference quantity, level, value, number, frequency, percentage, dimension, size, amount, weight or length.

Throughout this specification, unless the context requires otherwise, the words "comprise", "comprises" and "comprising" will be understood to imply the inclusion of a stated step or element or group of steps or elements but not the exclusion of any other step or element or group of steps or elements. In particular embodiments, the terms "include," "has," "contains," and "comprise" are used synonymously.

By "consisting of" is meant including, and limited to, whatever follows the phrase "consisting of." Thus, the phrase "consisting of" indicates that the listed elements are required or mandatory, and that no other elements may be present.

By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that no other elements are optional and may or may not be present depending upon whether or not they affect the activity or action of the listed elements.

Reference throughout this specification to "one embodiment," "an embodiment," "a particular embodiment," "a related embodiment," "a certain embodiment," "an additional embodiment," or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments.

As used herein, the term "isolated" means material that is substantially or essentially free from components that normally accompany it in its native state. In particular embodiments, the term "obtained" or "derived" is used synonymously with isolated.

As used herein, the term "DNA" refers to deoxyribonucleic acid. In various embodiments, the term DNA refers to genomic DNA, recombinant DNA, synthetic DNA, or cDNA. In one embodiment, DNA refers to genomic DNA or cDNA. In particular embodiments, the DNA comprises a "target region." DNA libraries contemplated herein include genomic DNA libraries and cDNA libraries constructed from RNA, *e.g.,* an RNA expression library. In various embodiments, the DNA libraries comprise one or more additional DNA sequences and/or tags.

A "target genetic locus" or "DNA target region" refers to a region of interest within a DNA sequence. In various embodiments, targeted genetic analyses are performed on the target genetic locus. In particular embodiments, the DNA target region is a region of a gene that is associated with a particular genetic state, genetic condition, genetic diseases; fetal testing; genetic mosaicism, paternity testing; predicting response to drug treatment; diagnosing or monitoring a medical condition; microbiome profiling; pathogen screening; or organ transplant monitoring.

As used herein, the terms "circulating DNA," "circulating cell-free DNA" and "cell-free DNA" are often used interchangeably and refer to DNA that is extracellular DNA, DNA that has been extruded from cells, or DNA that has been released from necrotic or apoptotic cells.

A "subject," "individual," or "patient" as used herein, includes any animal that exhibits a symptom of a condition that can be detected or identified with compositions contemplated herein. Suitable subjects include laboratory animals (such as mouse, rat, rabbit, or guinea pig), farm animals (such as horses, cows, sheep, pigs), and domestic animals or pets (such as a cat or dog). In particular embodiments, the subject is a mammal. In certain embodiments, the subject is a non-human primate and, in preferred embodiments, the subject is a human.

### C. METHODS OF GENETIC ANALYSIS OF CELL FREE DNA

The invention provides a method for genetic analysis of cfDNA, as recited by the claim set.

The method comprises: generating and amplifying a cfDNA library, determining the number of genome equivalents in the cfDNA library; and performing a quantitative genetic analysis of one or more genomic target loci.

In particular, the method comprises treating cfDNA with one or more end-repair enzymes to generate end-repaired cfDNA and ligating one or more adaptors to each end of the end-repaired cfDNA to generate a cfDNA library; amplifying the cfDNA library to generate cfDNA library clones; determining the number of genome equivalents of cfDNA library clones; and performing a quantitative genetic analysis of one or more target genetic loci in the cfDNA library clones.

### 1. GENERATING A CFDNA LIBRARY

The methods of genetic analysis contemplated herein comprise generating a cfDNA library comprising treating cfDNA with one or more end-repair enzymes to generate end-repaired cfDNA and ligating one or more adaptors to each end of the end-repaired cfDNA to generate the cfDNA library.

### (a) Cell-Free DNA (cfDNA)

The methods and compositions contemplated herein are designed to efficiently analyze, detect, diagnose, and/or monitor genetic states, genetic conditions, genetic diseases, genetic mosaicism, fetal diagnostics, paternity testing, microbiome profiling, pathogen screening, and organ transplant monitoring using cell-free DNA (cfDNA) as an analyte. The size distribution of cfDNA ranges from about 150 bp to about 180 bp fragments. Fragmentation may be the result of endonucleolytic and/or exonucleolytic activity and presents a formidable challenge to the accurate, reliable, and robust analysis of cfDNA. Another challenge for analyzing cfDNA is its short half-life in the blood stream, on the order of about 15 minutes. Without wishing to be bound to any particular theory, the present invention contemplates, in part, that analysis of cfDNA is like a "liquid biopsy" and is a real-time snapshot of current biological processes.

Moreover, because cfDNA is not found within cells and may be obtained from a number of suitable sources including, but not limited to, biological fluids and stool samples, it is not subject to the existing limitations that plague next generation sequencing analysis, such as direct access to the tissues being analyzed..

Illustrative examples of biological fluids that are suitable sources from which to isolate cfDNA in particular embodiments include, but are not limited to amniotic fluid, blood, plasma, serum, semen, lymphatic fluid, cerebral spinal fluid, ocular fluid, urine, saliva, mucous, and sweat.

In particular embodiments, the biological fluid is blood or blood plasma.

In certain embodiments, commercially available kits and other methods known to the skilled artisan can used used to isolate cfDNA directly from the biological fluids of a patient or from a previously obtained and optionally stabilized biological sample, *e.g.,* by freezing and/or addition of enzyme chelating agents including, but not limited to EDTA, EGTA, or other chelating agents specific for divalent cations.

### (b) Generating End-Repaired cfDNA

Generating a cfDNA library comprises the end-repair of isolated cfDNA. The fragmented cfDNA is processed by end-repair enzymes to generate end-repaired cfDNA with blunt ends, 5'-overhangs, or 3'-overhangs. In some embodiments, the end-repair enzymes can yield for example. In some embodiments, the end-repaired cfDNA contains blunt ends. In some embodiments, the end-repaired cfDNA is processed to contain blunt ends. In some embodiments, the blunt ends of the end-repaired cfDNA are further modified to contain a single base pair overhang. In some embodiments, end-repaired cfDNA containing blunt ends can be further processed to contain adenine (A)/thymine (T) overhang. In some embodiments, end-repaired cfDNA containing blunt ends can be further processed to contain adenine (A)/thymine (T) overhang as the single base pair overhang. In some embodiments, the end-repaired cfDNA has non-templated 3' overhangs. In some embodiments, the end-repaired cfDNA is processed to contain 3' overhangs. In some embodiments, the end-repaired cfDNA is processed with terminal transferase (TdT) to contain 3' overhangs. In some embodiments, a G-tail can be added by TdT. In some embodiments, the end-repaired cfDNA is processed to contain overhang ends using partial digestion with any known restriction enzymes (*e*.*g*., with the enzyme Sau3A, and the like.

### (c) Ligating Adaptor Molecules to End-Repaired cfDNA

Generating a cfDNA library comprises ligating one or more adaptors to each end of the end-repaired cfDNA. The present invention contemplates, in part, an adaptor module designed to accommodate large numbers of genome equivalents in cfDNA libraries. Adaptor modules are configured to measure the number of genome equivalents present in cfDNA libraries, and, by extension, the sensitivity of sequencing assays used to identify sequence mutations.

As used herein, the term "adaptor module" refers to a polynucleotide comprising at least five elements: (i) a first element comprising a PCR primer binding site for the single-primer library amplification; (ii) a second element comprising a 5 nucleotide read code that acts to uniquely identified each sequencing read; (iii) a third element comprising a 3 nucleotide sample code to identify different samples and enable sample multiplexing within a sequencing run; (iv) a fourth element comprising a 12 nucleotide anchor sequence that enables calibration of proper base calls in sequencing reads and acts as an anchor for hybridization to a partner oligonucleotide; and (v) a fifth element comprising the two 3' terminal nucleotides of Element 4 (Figure 17 and Tables 12-16). The adaptor module is hybridized to a partner oligonucleotide that is complementary to Element 4 to form an adaptor suitable for ligating to the ends of cfDNA, optionally end-repaired blunt-ended cfDNA.

In particular embodiments, an adaptor module comprises one or more PCR primer sequences, one or more read codes, one or more sample codes, one or more anchor sequences, and two or more 3'nucleotides that are efficient ligation substrates. In additional embodiments, the adaptor module further comprises one or more sequencing primer binding sites.

In particular embodiments, an adaptor module comprises a first element that comprises one or more PCR primer binding sequences for single-primer amplification of a cfDNA library. In one embodiment, the PCR primer binding sequence is about 12 to about 40 nucleotides, about 18 to about 40 nucleotides, about 20 to about 35 nucleotides, or about 20 to about 30 nucleotides. In another embodiment, the PCR primer binding sequence is about 12 nucleotides, about 13 nucleotides, about 14 nucleotides, about 15 nucleotides, about 16 nucleotides, about 17 nucleotides, about 18 nucleotides, about 19 nucleotides, about 20 nucleotides, about 21 nucleotides, about 22 nucleotides, about 23 nucleotides, about 24 nucleotides, about 25 nucleotides, about 26 nucleotides, about 27 nucleotides, about 28 nucleotides, about 29 nucleotides, about 30 nucleotides, about 31 nucleotides, about 32 nucleotides, about 33 nucleotides, about 34 nucleotides, about 35 nucleotides, about 36 nucleotides, about 37 nucleotides, about 38 nucleotides, about 39 nucleotides, or about 40 nucleotides or more.

In one embodiment, the PCR primer binding sequence is about 25 nucleotides.

In particular embodiments, an adaptor module comprises a second element that comprises one or more read code sequences. As used herein, the term "read code" refers to a polynucleotide that is used to identify unique sequencing reads. In one embodiment, the read code is a random sequence of nucleotides. In one embodiment, the read code is about 1 nucleotide, about 2 nucleotides, about 3 nucleotides, about 4 nucleotides, about 5 nucleotides, about 6 nucleotides, about 7 nucleotides, about 8 nucleotides, about 9 nucleotides, about 10 nucleotides, or more.

By way of a non-limiting example, a 5 nucleotide codes consists of 256 possible unique sequences where each code chosen is 2 nucleotides different from every other code in the set. This feature enables unique and distinct reads to be differentiated from reads that appear to be unique owing to a sequencing error in the code region. In particular embodiments, codes that have been empirically determined to interfere with adaptor function, owing to particular sequence combinations, may be excluded from use, *e.g.,* seven codes of the 256 had an overrepresentation of G nucleotides and were excluded.

In other embodiments, each read code of 5, 6, 7, 8, 9, 10 or more nucleotides may differ by 2, 3, 4, or 5 nucleotides from every other read code.

In one embodiment, the read code is about 5 nucleotides and differs from every other read code by 2 nucleotides.

In particular embodiments, an adaptor module comprises a third element that comprises one or more sample code sequences. As used herein, the term "sample code" refers to a polynucleotide that is used to identify the sample. The sample code is also useful in establishing multiplex sequencing reactions because each sample code is unique to the sample and thus, can be used to identify a read from a particular sample within a multiplexed sequencing reaction.

In one embodiment, the sample code comprises sequence that is about 1, about 2 nucleotides, about 3 nucleotides, about 4 nucleotides, or about 5 nucleotides, or more. In another embodiment, each sample code of 2, 3, 4, 5 or more nucleotides may differ from every other sample code by 2, 3, 4, or 5 nucleotides.

In one embodiment, the sample code is about three nucleotides and differs from every other sample code used in other samples by two nucleotides.

In particular embodiments, an adaptor module comprises a fourth element that comprises one or more anchor sequences. As used herein, an "anchor sequence" refers to a nucleotide sequence of at least 8 nucleotides, at least 10 nucleotides, at least 12 nucleotides, at least 14 nucleotides, or at least 16 nucleotides that hybridizes to a partner oligonucleotide and that comprises the following three properties: (1) each anchor sequence is part of a family of four anchor sequences that collectively represent each of the four possible DNA bases at each site within extension; this feature, balanced base representation, is useful to calibrate proper base calling in sequencing reads in particular embodiments; (2) each anchor sequence is composed of only two of four possible bases, and these are specifically chosen to be either and equal number of A + C or an equal number of G + T; an anchor sequence formed from only two bases reduces the possibility that the anchor sequence will participate in secondary structure formation that would preclude proper adaptor function; and (3) because each anchor sequence is composed of equal numbers of A+C or G+T, each anchor sequence shares roughly the same melting temperature and duplex stability as every other anchor sequence in a set of four.

In particular embodiments, an adaptor module comprises a fifth element that is comprised of the two 3' terminal nucleotides of Element 4. These two bases at the 3' end of each anchor are chosen based on an empirical determination that shows that these two nucleotides are efficient substrates for ligation to the cfDNA. In particular embodiments, Element 5 comprises the sequences selected from the group consisting of: AA, CC, TT and GG. In particular embodiments, Element 5 does not comprise the dinucleotide combination CG or TG as the inventors have determined that these combinations are not efficient ligation substrates.

In particular embodiments, a ligation step comprises ligating an adaptor module to the end-repaired cfDNA to generate a "tagged" cfDNA library. In some embodiments, a single adaptor module is employed. In some embodiments, two, three, four or five adaptor modules are employed. In some embodiments, an adaptor module of identical sequence is ligated to each end of the fragmented end-repaired DNA.

In one embodiment, a plurality of adaptor species is ligated to an end-repaired cfDNA library. Each of the plurality of adaptors may comprise one or more primer binding site for amplification of the cfDNA library, one or more read code sequences, one or more sequences for sample multiplexing, and one or more sequences for DNA sequencing.

Ligation of one or more adaptors contemplated herein may be carried out by methods known to those of ordinary skill in the art. In particular embodiments, one or more adaptors contemplated herein are ligated to end-repaired cfDNA that comprises blunt ends. In certain embodiments, one or more adaptors contemplated herein are ligated to end-repaired cfDNA that comprises complementary ends appropriate for the ligation method employed. In certain embodiments, one or more adaptors contemplated herein are ligated to end-repaired cfDNA that comprises a 3' overhang.

### 2. CFDNA LIBRARY AMPLIFICATION

Methods of genetic analysis contemplated herein comprise amplification of a cfDNA library to generate a cfDNA clone library or a library of cfDNA clones. Each molecule of the cfDNA library comprises an adaptor ligated to each end of an end-repaired cfDNA, and each adaptor comprises one or more PCR primer binding sites. In one embodiment, different adaptors are ligated to different ends of the end-repaired cfDNA.

In a preferred embodiment, the same adaptor is ligated to both ends of the cfDNA. Ligation of the same adaptor to both ends of end-repaired cfDNA allows for PCR amplification with a single primer sequence. In particular embodiments, a portion of the adaptor ligated-cfDNA library will be amplified using standard PCR techniques with a single primer sequence driving amplification. In one embodiment, the single primer sequence is about 25 nucleotides, optionally with a projected Tm of ≥ 55° C under standard ionic strength conditions.

In particular embodiments, picograms of the initial cfDNA library are amplified into micrograms of cfDNA clones, implying a 10,000-fold amplification. The amount of amplified product can be measured using methods known in the art, *e.g.,* quantification on a Qubit 2.0 or Nanodrop instrument.

### 3. DETERMINING THE NUMBER OF GENOME EQUIVALENTS

A method for genetic analysis of cfDNA comprises determining the number of genome equivalents in the cfDNA clone library. As used herein, the term "genome equivalent" refers to the number of genome copies in each library. An important challenge met by the compositions and methods contemplated herein is achieving sufficient assay sensitivity to detect and analysis rare genetic mutations or differences in genetic sequence. To determine assay sensitivity value on a sample-by-sample basis, the numbers of different and distinct sequences that are present in each sample are measured, by measuring the number of genome equivalents that are present in a sequencing library. To establish sensitivity, the number of genome equivalents must be measured for each sample library.

The number of genome equivalents can be determined by qPCR assay or by using bioinformatics-based counting after sequencing is performed. In the process flow of clinical samples, qPCR measurement of genome equivalents is used as a QC step for cfDNA libraries. It establishes an expectation for assay sensitivity prior to sequence analysis and allows a sample to be excluded from analysis if its corresponding cfDNA clone library lacks the required depth of genome equivalents. Ultimately, the bioinformatics-based counting of genome equivalents is also used to identify the genome equivalents - and hence the assay sensitivity and false negative estimates - for each given cfDNA clone library.

The empirical qPCR assay and statistical counting assays should be well correlated. In cases where sequencing fails to reveal the sequence depth in a cfDNA clone library, reprocessing of the cfDNA clone library and/or additional sequencing may be required.

In one embodiment, the genome equivalents in a cfDNA clone library are determined using a quantitative PCR (qPCR) assay. In a particular embodiment, a standard library of known concentration is used to construct a standard curve and the measurements from the qPCR assay are fit to the resulting standard curve and a value for genome equivalents is derived from the fit. Surprisingly, the present inventors have discovered that a qPCR "repeat-based" assay comprising one primer that specifically hybridizes to a common sequence in the genome, *e.g.,* a repeat sequence, and another primer that binds to the primer binding site in the adaptor, measured an 8-fold increase in genome equivalents compared to methods using just the adaptor specific primer (present on both ends of the cfDNA clone). The number of genome equivalents measured by the repeat-based assays provides a more consistent library-to-library performance and a better alignment between qPCR estimates of genome equivalents and bioinformatically counted tag equivalents in sequencing runs.

Illustrative examples of repeats suitable for use in the repeat-based genome equivalent assays contemplated herein include, but not limited to: short interspersed nuclear elements (SINEs), *e.g.,* Alu repeats; long interspersed nuclear elements (LINEs), *e.g.,* LINE1, LINE2, LINE3; microsatellite repeat elements, *e.g.,* short tandem repeats (STRs), simple sequence repeats (SSRs); and mammalian-wide interspersed repeats (MIRs).

In one embodiment, the repeat is an Alu repeat.

### 4. QUANTITATIVE GENETIC ANALYSIS

A method for genetic analysis of cfDNA comprises quantitative genetic analysis of one or more target genetic loci of the cfDNA library clones. Quantitative genetic analysis comprises one or more of, or all of, the following steps: capturing cfDNA clones comprising a target genetic locus; amplification of the captured targeted genetic locus; sequencing of the amplified captured targeted genetic locus; and bioinformatic analysis of the resulting sequence reads.

### (a) Capture of Target Genetic Locus

The present invention contemplates, in part, a capture probe module designed to retain the efficiency and reliability of larger probes but that minimizes uninformative sequence generation in a cfDNA clone library. A "capture probe module" refers to a polynucleotide that comprises a capture probe sequence and a tail sequence. In particular embodiments, the capture probe module sequence or a portion thereof serves as a primer binding site for one or more sequencing primers.

In particular embodiments, a capture probe module comprises a capture probe. As used herein a "capture probe" refers to a region capable of hybridizing to a specific DNA target region. Because the average size of cfDNA is about 150 to about 170 bp and is highly fragmented the compositions and methods contemplated herein comprise the use of high density and relatively short capture probes to interrogate DNA target regions of interest.

One particular concern with using high density capture probes is that generally capture probes are designed using specific "sequence rules." For example, regions of redundant sequence or that exhibit extreme base composition biases are generally excluded in designing capture probes. However, the present inventors have discovered that the lack of flexibility in capture probe design rules does not substantially impact probe performance. In contrast, capture probes chosen strictly by positional constraint provided on-target sequence information; exhibit very little off-target and unmappable read capture; and yield uniform, useful, on-target reads with only few exceptions. Moreover, the high redundancy at close probe spacing more than compensates for occasional poor-performing capture probes.

In particular embodiments, a target region is targeted by a plurality of capture probes, wherein any two or more capture probes are designed to bind to the target region within 10 nucleotides of each other, within 15 nucleotides of each other, within 20 nucleotides of each other, within 25 nucleotides of each other, within 30 nucleotides of each other, within 35 nucleotides of each other, within 40 nucleotides of each other, within 45 nucleotides of each other, or within 50 nucleotides or more of each other, as well as all intervening nucleotide lengths.

In one embodiment, the capture probe is about 25 nucleotides, about 26 nucleotides, about 27 nucleotides, about 28 nucleotides, about 29 nucleotides, about 30 nucleotides, about 31 nucleotides, about 32 nucleotides, about 33 nucleotides, about 34 nucleotides, about 35 nucleotides, about 36 nucleotides, about 37 nucleotides, about 38 nucleotides, about 39 nucleotides, about 40 nucleotides, about 41 nucleotides, about 42 nucleotides, about 43 nucleotides, about 44 nucleotides, or about 45 nucleotides.

In a particular embodiment, the capture probe is not 60 nucleotides.

In another embodiment, the capture probe is substantially smaller than 60 nulceotides but hybridizes comparably, as well as, or better than a 60 nucleotide capture probe targeting the same DNA target region.

In a certain embodiment, the capture probe is 40 nucleotides.

In certain embodiments, a capture probe module comprises a tail sequence. As used herein, the term "tail sequence" refers to a polynucleotide at the 5' end of the capture probe module, which in particular embodiments can serve as a primer binding site. In particular embodiments, a sequencing primer binds to the primer binding site in the tail region.

In particular embodiments, the tail sequence is about 5 to about 100 nucleotides, about 10 to about 100 nucleotides, about 5 to about 75 nucleotides, about 5 to about 50 nucleotides, about 5 to about 25 nucleotides, or about 5 to about 20 nucleotides. In certain embodiments, the third region is from about 10 to about 50 nucleotides, about 15 to about 40 nucleotides, about 20 to about 30 nucleotides or about 20 nucleotides, or any intervening number of nucleotides.

In particular embodiments, the tail sequence is about 30 nucleotides, about 31 nucleotides, about 32 nucleotides, about 33 nucleotides, about 34 nucleotides, about 35 nucleotides, about 36 nucleotides, about 37 nucleotides, about 38 nucleotides, about 39 nucleotides, or about 40 nucleotides.

In various embodiments, the capture probe module comprises a specific member of a binding pair to enable isolation and/or purification of one or more captured fragments of a tagged and or amplified cfDNA library that hybridizes to the capture probe. In particular embodiments, the capture probe module is conjugate to biotin or another suitable hapten, *e*.*g*., dinitrophenol, digoxigenin.

In various embodiments, the capture probe module is hybridized to a tagged and optionally amplified cfDNA library to form a complex. In some embodiments, the multifunctional capture probe module substantially hybridizes to a specific genomic target region in the cfDNA library.

Hybridization or hybridizing conditions can include any reaction conditions where two nucleotide sequences form a stable complex; for example, the tagged cfDNA library and capture probe module forming a stable tagged cfDNA library -capture probe module complex. Such reaction conditions are well known in the art and those of skill in the art will appreciated that such conditions can be modified as appropriate, *e*.*g*., decreased annealing temperatures with shorter length capture probes, and within the scope of the present invention. Substantial hybridization can occur when the second region of the capture probe complex exhibits 100%, 99%, 98%, 97%, 96%, 95%, 94%, 93%, 92% 91%, 90%, 89%, 88%, 85%, 80%, 75%, or 70% sequence identity, homology or complementarity to a region of the tagged cfDNA library.

In particular embodiments, the capture probe is about 40 nucleotides and has an optimal annealing temperature of about 44° C o about 47° C.

In certain embodiments, the methods contemplated herein comprise isolating a tagged cfDNA library-capture probe module complex. In particular embodiments, methods for isolating DNA complexes are well known to those skilled in the art and any methods deemed appropriate by one of skill in the art can be employed with the methods of the present invention (Ausubel et al., Current Protocols in Molecular Biology, 2007-2012). In particular embodiments, the complexes are isolated using biotin-streptavidin isolation techniques.

In particular embodiments, removal of the single stranded 3'-ends from the isolated tagged cfDNA library- capture probe module complex is contemplated. In certain embodiments, the methods comprise 3'-5' exonuclease enzymatic processing of the isolated tagged DNA library-multifunctional capture probe module complex to remove the single stranded 3' ends.

In certain other embodiments, the methods comprise performing 5'-3' DNA polymerase extension of multifunctional capture probe utilizing the isolated tagged DNA library fragments as template.

In certain other embodiments, the methods comprise creating a hybrid capture probe-isolated tagged cfDNA target molecule through the concerted action of a 5' FLAP endonuclease, DNA polymerization and nick closure by a DNA ligase.

A variety of enzymes can be employed for the 3'-5' exonuclease enzymatic processing of the isolated tagged cfDNA library-multifunctional capture probe module complex. Illustrative examples of suitable enzymes, which exhibit 3'-5' exonuclease enzymatic activity, that can be employed in particular embodiments include, but are not limited to: T4 or Exonucleases I, III, V (*see also,* Shevelev IV, Hübscher U., "The 3' 5' exonucleases," Nat Rev Mol Cell Biol. 3(5):364-76 (2002)). In particular embodiments, the enzyme comprising 3'-5' exonuclease activity is T4 polymerase. In particular embodiments, an enzyme which exhibits 3'-5' exonuclease enzymatic activity and is capable of primer template extension can be employed, including for example T4 or Exonucleases I, III, V. *Id.*

In some embodiments, the methods contemplated herein comprise performing sequencing and/or PCR on the 3'-5' exonuclease enzymatically processed complex discussed *supra* and elsewhere herein. In particular embodiments, a tail portion of a capture probe molecule is copied in order to generate a hybrid nucleic acid molecule. In one embodiment, the hybrid nucleic acid molecule generated comprises the target region capable of hybridizing to the capture probe module and the complement of the capture probe module tail sequence.

In a particular embodiment, genetic analysis comprises a) hybridizing one or more capture probe modules to one or more target genetic loci in a plurality of cfDNA library clones to form one or more capture probe module-cfDNA library clone complexes; b) isolating the one or more capture probe module-cfDNA library clone complexes from a); c) enzymatically processing the one or more isolated capture probe module-cfDNA library clone complexes from step b); d) performing PCR on the enzymatically processed complex from c) wherein the tail portion of the capture probe molecule is copied in order to generate amplified hybrid nucleic acid molecules, wherein the amplified hybrid nucleic acid molecules comprise a target sequence in the target genomic locus capable of hybridizing to the capture probe and the complement of the capture probe module tail sequence; and e) performing quantitative genetic analysis on the amplified hybrid nucleic acid molecules from d).

In a particular embodiment, methods for determining copy number of a specific target genetic locus are contemplated comprising: a) hybridizing one or more capture probe modules to one or more target genetic loci in a plurality of cfDNA library clones to form one or more capture probe module-cfDNA library clone complexes; b) isolating the one or more capture probe module-cfDNA library clone complexes from a); c) enzymatically processing the one or more isolated capture probe module-cfDNA library clone complexes from step b); d) performing PCR on the enzymatically processed complex from c) wherein the tail portion of the capture probe molecule is copied in order to generate amplified hybrid nucleic acid molecules, wherein the amplified hybrid nucleic acid molecules comprise a target sequence in the the target genetic locus capable of hybridizing to the capture probe and the complement of the capture probe module tail sequence; e) performing PCR amplification of the amplified hybrid nucleic acid molecules in d); and f) quantitating the PCR reaction in e), wherein the quantitation allows for a determination of copy number of the specific target region.

In one embodiment, the enzymatic processing of step c) comprises performing 3'-5' exonuclease enzymatic processing on the one or more capture probe module-cfDNA library clone complexes from b) using an enzyme with 3'-5' exonuclease activity to remove the single stranded 3' ends; creating one or more hybrid capture probe module-cfDNA library clone molecules through the concerted action of a 5' FLAP endonuclease, DNA polymerization and nick closure by a DNA ligase; or performing 5'-3' DNA polymerase extension of the capture probe using the isolated cfDNA clone in the complex as a template.

In one embodiment, the enzymatic processing of step c) comprises performing 5'-3' DNA polymerase extension of the capture probe using the isolated cfDNA clone in the complex as a template.

In particular embodiments, PCR can be performed using any standard PCR reaction conditions well known to those of skill in the art. In certain embodiments, the PCR reaction in e) employs two PCR primers. In one embodiment, the PCR reaction in e) employs a first PCR primer that hybridizes to a repeat within the target genetic locus. In a particular embodiment, the PCR reaction in e) employs a second PCR primer that hybridizes to the hybrid nucleic acid molecules at the target genetic locus/tail junction. In certain embodiments, the PCR reaction in e) employs a first PCR primer that hybridizes to the target genetic locus and a second PCR primer hybridizes to the amplified hybrid nucleic acid molecules at the target genetic locus/tail junction. In particular embodiments, the second primer hybridizes to the target genetic locus/tail junction such that at least one or more nucleotides of the primer hybridize to the target genetic locus and at least one or more nucleotides of the primer hybridize to the tail sequence.

In certain embodiments, the amplified hybrid nucleic acid molecules obtained from step e) are sequenced and the sequences aligned horizontally, i.e., aligned to one another but not aligned to a reference sequence. In particular embodiments, steps a) through e) are repeated one or more times with one or more capture probe modules. The capture probe modules can be the same or different and designed to target either cfDNA strand of a target genetic locus. In some embodiments, when the capture probes are different, they hybridize at overlapping or adjacent target sequences within a target genetic locus in the tagged cfDNA clone library. In one embodiment, a high density capture probe strategy is used wherein a plurality of capture probes hybridize to a target genetic locus, and wherein each of the plurality of capture probes hybridizes to the target genetic locus within about 5, 10, 15, 20, 25, 30, 35, 40, 45, 50, 100, 200, bp of any other capture probe that hybridizes to the target genetic locus in a tagged cfDNA clone library, including all intervening distances.

In some embodiments, the method can be performed using two capture probe modules per target genetic locus, wherein one hybridizes to the "Watson" strand (noncoding or template strand) upstream of the target region and one hybridizes to the "Crick" strand (coding or non-template strand) downstream of the target region.

In particular embodiments, the methods contemplated herein can further be performed multiple times with any number of capture probe modules, for example 2, 3, 4, 5, 6, 7, 8, 9, or 10 or more capture probe modules per target genetic locus any number of which hybridize to the Watson or Crick strand in any combination. In some embodiments, the sequences obtained can be aligned to one another in order to identify any of a number of differences.

In certain embodiments, a plurality of target genetic loci are interrogated, *e*.*g*., 100, 200, 300, 400, 500, 600, 700, 800, 900, 1000, 1500, 2000, 2500, 3000, 3500, 4000, 4500, 5000, 10000, 50000, 100000, 500000 or more in a single reaction, using one or more capture probe modules.

### (b) Sequencing

The quantitative genetic analysis comprises sequencing a plurality of hybrid nucleic acid molecules, as discussed elsewhere herein, *supra,* to generate sufficient sequencing depths to obtain a plurality of unique sequencing reads. A unique read is defined as the single consensus read from a "family" of reads that all share the same read code and sequence start point within cfDNA. Each capture probe yields a set of unique reads that are computationally distilled from total reads by grouping into families. The unique reads for a given sample are then computed as the average of all the unique reads observed on a probe-by-probe basis. Cases where there is an obvious copy number change are excluded from the data set used to compute the average. Unique reads are important because each unique read must be derived from a unique cfDNA clone. Each unique read represents the input and analysis of a haploid equivalent of genomic DNA. The sum of unique reads is the sum of haploid genomes analyzed. The number of genomes analyzed, in turn, defines the sensitivity of the sequencing assay. By way of a non-limiting example, if the average unique read count is 100 genome equivalents, then that particular assay has a sensitivity of being able to detect one mutant read in 100, or 1%. Any observation less than this is not defensible.

In particular embodiments, the quantitative genetic analysis comprises multiplex sequencing of hybrid nucleic acid molecules derived from a plurality of samples.

In various embodiments, the quantitative genetic analysis comprises obtaining one or more or a plurality of tagged DNA library clones, each clone comprising a first DNA sequence and a second DNA sequence, wherein the first DNA sequence comprises a sequence in a targeted genetic locus and the second DNA sequence comprises a capture probe sequence; performing a paired end sequencing reaction on the one or more clones and obtaining one or more sequencing reads or performing a sequencing reaction on the one or more clones in which a single long sequencing read of greater than about 100, 200, 300, 400, 500 or more nucleotides is obtained, wherein the read is sufficient to identify both the first DNA sequence and the second DNA sequence; and ordering or clustering the sequencing reads of the one or more clones according to the probe sequences of the sequencing reads.

### (c) Bioinformatics Analysis

The quantitative genetic analysis further comprises bioinformatic analysis of the sequencing reads. Bioinformatic analysis excludes any purely mental analysis performed in the absence of a composition or method for sequencing. In certain embodiments, bioinformatics analysis includes, but is not limited to: sequence alignments; genome equivalents analysis; single nucleotide variant (SNV) analysis; gene copy number variation (CNV) analysis; and detection of genetic lesions. In particular embodiments, bioinformatics analysis is usefulto quantify the number of genome equivalents analyzed in the cfDNA clone library; to detect the genetic state of a target genetic locus; to detect genetic lesions in a target genetic locus; and to measure copy number fluctuations within a target genetic locus.

Sequence alignments may be performed between the sequence reads and one or more human reference DNA sequences. In particular embodiments, sequencing alignments can be used to detect genetic lesions in a target genetic locus including, but not limited to detection of a nucleotide transition or transversion, a nucleotide insertion or deletion, a genomic rearrangement, a change in copy number, or a gene fusion. Detection of genetic lesions that are causal or prognostic indicators may be useful in the diagnosis, prognosis, treatment, and/or monitoring of a particular genetic condition or disease.

Also contemplated herein, are methods for sequence alignment analysis that can be performed without the need for alignment to a reference sequence, referred to herein as horizontal sequence analysis. Such analysis can be performed on any sequences generated by the methods contemplated herein or any other methods. In particular embodiments, the sequence analysis comprises performing sequence alignments on the reads obtained by the methods contemplated herein.

In one embodiment, the genome equivalents in a cfDNA clone library are determined using bioinformatics-based counting after sequencing is performed. Each sequencing read is associated with a particular capture probe, and the collection of reads assigned to each capture probe is parsed into groups. Within a group, sets of individual reads share the same read code and the same DNA sequence start position within genomic sequence. These individual reads are grouped into a "family" and a single consensus representative of this family is carried forward as a "unique read." All of the individual reads that constituted a family are derived from a single ligation event and thus, they are amplification-derived "siblings" of one another. Each unique read is considered a unique ligation event and the sum of unique reads is considered equivalent to the number of genome equivalents analyzed.

As the number of unique clones approaches the total number of possible sequence combinations, probability dictates that the same code and start site combinations will be created by independent events and that these independent events will be inappropriately grouped within single families. The net result will be an underestimate of genome equivalents analyzed, and rare mutant reads may be discarded as sequencing errors because they overlap with wild-type reads bearing the same identifiers.

In particular embodiments, to provide an accurate analysis for cfDNA clone libraries, the number of genome equivalents analyzed is about 1/10, about 1/12, about 1/14, about 1/16, about 1/18, about 1/20, about 1/25 or less the the number of possible unique clones. It should be understood that the procedure outlined above is merely illustrative and not limiting.

In some embodiments, the number of genome equivalents to be analyzed may need to be increased. To expand the depth of genome equivalents, at least two solutions are contemplated. The first solution is to use more than one adaptor set per sample. By combining adaptors, it is possible to multiplicatively expand the total number of possible clones and therefore, expand the comfortable limits of genomic input. The second solution is to expand the read code by 1, 2, 3, 4, or 5 or more bases. The number of possible read codes that differ by at least 2 bases from every other read code scales as 4⁽ⁿ⁻¹⁾ where n is the number of bases within a read code. Thus, in a non-limiting example, if a read code is 5 nucleotides and 4⁽⁵⁻¹⁾ = 256; therefore, the inclusion of additional bases expands the available repertoire by a factor of four for each additional base.

In one embodiment, quantitative genetic analysis comprises bioinformatic analysis of sequencing reads to identify rare single nucleotide variants (SNV).

Next-generation sequencing has an inherent error rate of roughly 0.02-0.02%, meaning that anywhere from 1/200 to 1/500 base calls are incorrect. To detect variants and other mutations that occur at frequencies lower than this, for example at frequencies of 1 per 1000 sequences, it is necessary to invoke molecular annotation strategies. By way of a non-limiting example, analysis of 5000 unique molecules using targeted sequence capture technology would generate - at sufficient sequencing depths of >50,000 reads - a collection of 5000 unique reads, with each unique read belonging to a "family" of reads that all possess the same read code. A SNV that occurs within a family is a candidate for being a rare variant. When this same variant is observed in more than one family, it becomes a very strong candidate for being a rare variant that exists within the starting sample. In contrast, variants that occur sporadically within families are likely to be sequencing errors and variants that occur within one and only one family are either rare or the result of a base alteration that occurred *ex vivo* (*e.g.,* oxidation of a DNA base or PCR-introduced errors).

In one embodiment, the methods of detecting SNVs comprise introducing 10-fold more genomic input (genomes or genome equivalents) as the desired target sensitivity of the assay. In one non-limiting example, if the desired sensitivity is 2% (2 in 100), then the experimental target is an input of 2000 genomes.

In particular embodiments, bioinformatics analysis of sequencing data is used to detect or identify SNV associated with a genetic state, condition or disease, genetic mosaicism, fetal testing, paternity testing, predicting response to drug treatment, diagnosing or monitoring a medical condition, microbiome profiling, pathogen screening, and monitoring organ transplants.

In various embodiments, a method for copy number determination analysis is provided comprising obtaining one or more or a plurality of clones, each clone comprising a first DNA sequence and a second DNA sequence, wherein the first DNA sequence comprises a sequence in a targeted genetic locus and the second DNA sequence comprises a capture probe sequence. In related embodiments, a paired end sequencing reaction on the one or more clones is performed and one or more sequencing reads are obtained. In another embodiment, a sequencing reaction on the one or more clones is performed in which a single long sequencing read of greater than about 100 nucleotides is obtained, wherein the read is sufficient to identify both the first DNA sequence and the second DNA sequence. The sequencing reads of the one or more clones can be ordered or clustered according to the probe sequence of the sequencing reads.

Copy number analyses include, but are not limited to analyses, that examine the number of copies of a particular gene or mutation that occurs in a given genomic DNA sample and can further include quantitative determination of the number of copies of a given gene or sequence differences in a given sample. In particular embodiments, copy number analysis is used to detect or identify gene amplification associated with genetic states, conditions, or diseases, fetal testing, genetic mosaicism, paternity testing, predicting response to drug treatment, diagnosing or monitoring a medical condition, microbiome profiling, pathogen screening, and monitoring organ transplants.

In particular embodiments, bioinformatics analysis of sequencing data is used to detect or identify one or more sequences or genetic lesions in a target locus including, but not limited to detection of a nucleotide transition or transversion, a nucleotide insertion or deletion, a genomic rearrangement, a change in copy number, or a gene fusion. Detection of genetic lesions that are causal or prognostic indicators may be useful in the diagnosis, prognosis, treatment, and/or monitoring of a particular genetic condition or disease. In one embodiment, genetic lesions are associated with genetic states, conditions, or diseases, fetal testing, genetic mosaicism, paternity testing, predicting response to drug treatment, diagnosing or monitoring a medical condition, microbiome profiling, pathogen screening, and monitoring organ transplants.

### D. CLINICAL APPLICATIONS OF QUANTITATIVE GENETIC ANALYSIS

For illustrative purposes only and not recited by the claim set, there is provided a method of detecting, identifying, predicting, diagnosing, or monitoring a condition or disease in a subject.

In particular embodiments, a method of detecting, identifying, predicting, diagnosing, or monitoring a genetic state, condition or disease in a subject comprises performing a quantitative genetic analysis of one or more target genetic loci in a cfDNA clone library to detect or identify a change in the sequence at the one or more target genetic loci.

In one embodiment, a method of detecting, identifying, predicting, diagnosing, or monitoring a genetic state, condition or disease comprises isolating or obtaining cfDNA from a biological sample of a subject; treating the cfDNA with one or more end-repair enzymes to generate end-repaired cfDNA; ligating one or more adaptors to each end of the end-repaired cfDNA to generate a cfDNA library; amplifying the cfDNA library to generate a cfDNA clone library; determining the number of genome equivalents in the cfDNA clone library; and performing a quantitative genetic analysis of one or more target genetic loci in a cfDNA clone library to detect or identify a change in the sequence at the one or more target genetic loci.

In particular embodiments, a method of detecting, identifying, predicting, diagnosing, or monitoring a genetic state, or genetic condition or disease selected from the group consisting of: genetic diseases; genetic mosaicism; fetal testing; paternity testing; paternity testing; predicting response to drug treatment; diagnosing or monitoring a medical condition; microbiome profiling; pathogen screening; and organ transplant monitoring comprising isolating or obtaining cfDNA from a biological sample of a subject; treating the cfDNA with one or more end-repair enzymes to generate end-repaired cfDNA; ligating one or more adaptors to each end of the end-repaired cfDNA to generate a cfDNA library; amplifying the cfDNA library to generate a cfDNA clone library; determining the number of genome equivalents in the cfDNA clone library; and performing a quantitative genetic analysis of one or more target genetic loci in a cfDNA clone library to detect or identify a nucleotide transition or transversion, a nucleotide insertion or deletion, a genomic rearrangement, a change in copy number, or a gene fusion in the sequence at the one or more target genetic loci.

Illustrative examples of genetic diseases that can be detected, identified, predicted, diagnosed, or monitored with the compositions and methods contemplated herein include, but are not limited to cancer, Alzheimer's disease (APOE1), Charcot-Marie-Tooth disease, Leber hereditary optic neuropathy (LHON), Angelman syndrome (UBE3A, ubiquitin-protein ligase E3A), Prader-Willi syndrome (region in chromosome 15), β-Thalassaemia (HBB, β-Globin), Gaucher disease (type I) (GBA, Glucocerebrosidase), Cystic fibrosis (CFTR Epithelial chloride channel), Sickle cell disease (HBB, β-Globin), Tay-Sachs disease (HEXA, Hexosaminidase A), Phenylketonuria (PAH, Phenylalanine hydrolyase), Familial hypercholesterolaemia (LDLR, Low density lipoprotein receptor), Adult polycystic kidney disease (PKD1, Polycystin), Huntington disease (HDD, Huntingtin), Neurofibromatosis type I (NF1, NF1 tumour suppressor gene), Myotonic dystrophy (DM, Myotonin), Tuberous sclerosis (TSC1, Tuberin), Achondroplasia (FGFR3, Fibroblast growth factor receptor), Fragile X syndrome (FMR1, RNA-binding protein), Duchenne muscular dystrophy (DMD, Dystrophin), Haemophilia A (F8C, Blood coagulation factor VIII), Lesch-Nyhan syndrome (HPRT1, Hypoxanthine guanine ribosyltransferase 1), and Adrenoleukodystrophy (ABCD1).

Illustrative examples of cancers that can be detected, identified, predicted, diagnosed, or monitored with the compositions and methods contemplated herein include, but are not limited to: B cell cancer, e.g., multiple myeloma, melanomas, breast cancer, lung cancer (such as non-small cell lung carcinoma or NSCLC), bronchus cancer, colorectal cancer, prostate cancer, pancreatic cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematological tissues, adenocarcinomas, inflammatory myofibroblastic tumors, gastrointestinal stromal tumor (GIST), colon cancer, multiple myeloma (MM), myelodysplastic syndrome (MDS), myeloproliferative disorder (MPD), acute lymphocytic leukemia (ALL), acute myelocytic leukemia (AML), chronic myelocytic leukemia (CML), chronic lymphocytic leukemia (CLL), polycythemia Vera, Hodgkin lymphoma, non-Hodgkin lymphoma (NHL), soft-tissue sarcoma, fibrosarcoma, myxosarcoma, liposarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, seminoma, embryonal carcinoma, Wilms' tumor, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma, follicular lymphoma, diffuse large B-cell lymphoma, mantle cell lymphoma, hepatocellular carcinoma, thyroid cancer, gastric cancer, head and neck cancer, small cell cancers, essential thrombocythemia, agnogenic myeloid metaplasia, hypereosinophilic syndrome, systemic mastocytosis, familiar hypereosinophilia, chronic eosinophilic leukemia, neuroendocrine cancers, carcinoid tumors, and the like.

In one embodiment, the genetic lesion is a lesion annotated in the Cosmic database (the lesions and sequence data can be downloaded from cancer.sanger.ac.uk/cosmic/census) or a lesion annotated in the Cancer Genome Atlas (the lesions and sequence data can be downloaded from tcga-data.nci.nih.gov/tcga/tcgaDownload.jsp).

Illustrative examples of genes that harbor one or more genetic lesions associated with cancer that can be detected, identified, predicted, diagnosed, or monitored with the compositions and methods contemplated herein include, but are not limited to ABCB1, ABCC2, ABCC4, ABCG2, ABL1, ABL2, AKT1, AKT2, AKT3, ALDH4A1, ALK, APC, AR, ARAF, ARFRP1, ARID1A, ATM, ATR, AURKA, AURKB, BCL2, BCL2A1, BCL2L1, BCL2L2, BCL6, BRAF, BRCA1, BRCA2, Clorf144, CARD11, CBL, CCND1, CCND2, CCND3, CCNE1, CDH1, CDH2, CDH20, CDH5, CDK4, CDK6, CDK8, CDKN2A, CDKN2B, CDKN2C, CEBPA, CHEK1, CHEK2, CRKL, CRLF2, CTNNB1, CYP1B1, CYP2C19, CYP2C8, CYP2D6, CYP3A4, CYP3A5, DNMT3A, DOT1L, DPYD, EGFR, EPHA3, EPHA5, EPHA6, EPHA7, EPHB1, EPHB4, EPHB6, EPHX1, ERBB2, ERBB3, ERBB4, ERCC2, ERG, ESR1, ESR2, ETV1, ETV4, ETV5, ETV6, EWSR1, EZH2, FANCA, FBXW7, FCGR3A, FGFR1, FGFR2, FGFR3, FGFR4, FLT1, FLT3, FLT4, FOXP4, GATA1, GNA11, GNAQ, GNAS, GPR124, GSTP1, GUCY1A2, HOXA3, HRAS, HSP90AA1, IDH1, IDH2, IGF1R, IGF2R, IKBKE, IKZF1, INHBA, IRS2, ITPA, JAK1, JAK2, JAK3, JUN, KDR, KIT, KRAS, LRP1B, LRP2, LTK, MAN1B1, MAP2K1, MAP2K2, MAP2K4, MCL1, MDM2, MDM4, MEN1, MET, MITF, MLH1, MLL, MPL, MRE11A, MSH2, MSH6, MTHFR, MTOR, MUTYH, MYC, MYCL1, MYCN, NF1, NF2, NKX2-1, NOTCH1, NPM1, NQO1, NRAS, NRP2, NTRK1, NTRK3, PAK3, PAX5, PDGFRA, PDGFRB, PIK3CA, PIK3R1, PKHD1, PLCG1, PRKDC, PTCH1, PTEN, PTPN11, PTPRD, RAF1, RARA, RB1, RET, RICTOR, RPTOR, RUNX1, SLC19A1, SLC22A2, SLCO1B3, SMAD2, SMAD3, SMAD4, SMARCA4, SMARCB1, SMO, SOD2, SOX10, SOX2, SRC, STK11, SULT1A1, TBX22, TET2, TGFBR2, TMPRSS2, TNFRSF14, TOP1, TP53, TPMT, TSC1, TSC2, TYMS, UGT1A1, UMPS, USP9X, VHL, and WT1.

In particular embodiments, the genetic lesion comprises a nucleotide transition or transversion, a nucleotide insertion or deletion, a genomic rearrangement, a change in copy number, or a gene fusion.

In one embodiment, the genetic lesion is a gene fusion that fuses the 3' coding region of the ALK gene to another gene.

In one embodiment, the genetic lesion is a gene fusion that fuses the 3' coding region of the ALK gene to the EML4 gene.

Illustrative examples of conditions suitable for fetal testing that can be detected, identified, predicted, diagnosed, or monitored with the compositions and methods contemplated herein include but are not limited to: Down Syndrome (Trisomy 21), Edwards Syndrome (Trisomy 18), Patau Syndrome (Trisomy 13), Klinefelter's Syndrome (XXY), Triple X syndrome, XYY syndrome, Trisomy 8, Trisomy 16, Turner Syndrome (XO), Robertsonian translocation, DiGeorge Syndrome and Wolf-Hirschhorn Syndrome.

Illustrative examples of alleles suitable for paternity testing that can be detected, identified, predicted, diagnosed, or monitored with the compositions and methods contemplated herein include but are not limited to 16 or more of: D20S1082, D6S474, D12ATA63, D22S1045, D10S1248, D1S1677, D11S4463, D4S2364, D9S1122, D2S1776, D10S1425, D3S3053, D5S2500, D1S1627, D3S4529, D2S441, D17S974, D6S1017, D4S2408, D9S2157, Amelogenin, D17S1301, D1GATA113, D18S853, D20S482, and D14S1434.

Illustrative examples of genes suitable for predicting the response to drug treatment that can be detected, identified, predicted, diagnosed, or monitored with the compositions and methods contemplated herein include, but are not limited to, one or more of the following genes: ABCB1 (ATP-binding cassette, sub-family B (MDR/TAP), member 1), ACE (angiotensin I converting enzyme), ADH1A (alcohol dehydrogenase 1A (class I), alpha polypeptide), ADH1B (alcohol dehydrogenase IB (class I), beta polypeptide), ADH1C (alcohol dehydrogenase 1C (class I), gamma polypeptide), ADRB1 (adrenergic, beta-1-, receptor), ADRB2 (adrenergic, beta-2-, receptor, surface), AHR (aryl hydrocarbon receptor), ALDH1A1 (aldehyde dehydrogenase 1 family, member A1), ALOX5 (arachidonate 5-lipoxygenase), BRCA1 (breast cancer 1, early onset), COMT (catechol-O-methyltransferase), CYP2A6 (cytochrome P450, family 2, subfamily A, polypeptide 6), CYP2B6 (cytochrome P450, family 2, subfamily B, polypeptide 6), CYP2C9 (cytochrome P450, family 2, subfamily C, polypeptide 9), CYP2C19 (cytochrome P450, family 2, subfamily C, polypeptide 19), CYP2D6 (cytochrome P450, family 2, subfamily D, polypeptide 6), CYP2J2 (cytochrome P450, family 2, subfamily J, polypeptide 2), CYP3A4 (cytochrome P450, family 3, subfamily A, polypeptide 4), CYP3A5 (cytochrome P450, family 3, subfamily A, polypeptide 5), DPYD (dihydropyrimidine dehydrogenase), DRD2 (dopamine receptor D2), F5 (coagulation factor V), GSTP1 (glutathione S-transferase pi), HMGCR (3-hydroxy-3-methylglutaryl-Coenzyme A reductase), KCNH2 (potassium voltage-gated channel, subfamily H (eag-related), member 2), KCNJ11 (potassium inwardly-rectifying channel, subfamily J, member 11), MTHFR (5,10-methylenetetrahydrofolate reductase (NADPH)), NQO1 (NAD(P)H dehydrogenase, quinone 1), P2RY1 (purinergic receptor P2Y, G-protein coupled, 1), P2RY12 (purinergic receptor P2Y, G-protein coupled, 12), PTGIS (prostaglandin I2 (prostacyclin) synthase), SCN5A (sodium channel, voltage-gated, type V, alpha (long QT syndrome 3)), SLC19A1 (solute carrier family 19 (folate transporter), member 1), SLCO1B1 (solute carrier organic anion transporter family, member 1B1), SULT1A1 (sulfotransferase family, cytosolic, 1A, phenol-preferring, member 1), TPMT (thiopurine S-methyltransferase), TYMS (thymidylate synthetase), UGT1A1 (UDP glucuronosyltransferase 1 family, polypeptide A1), VDR (vitamin D (1,25- dihydroxyvitamin D3) receptor), VKORC1 (vitamin K epoxide reductase complex, subunit 1).

Illustrative examples of medical conditions that can be detected, identified, predicted, diagnosed, or monitored with the compositions and methods contemplated herein include, but are not limited to: stroke, transient ischemic attack, traumatic brain injury, heart disease, heart attack, angina, atherosclerosis, and high blood pressure.

Illustrative examples of pathogens that can be screened for with the compositions and methods contemplated herein include, but are not limited to: bacteria fungi, and viruses.

Illustrative examples of bacterial species that can be screened for with the compositions and methods contemplated herein include, but are not limited to: a Mycobacterium spp., a Pneumococcus spp., an Escherichia spp., a Campylobacter spp., a Corynebacterium spp., a Clostridium spp., a Streptococcus spp., a Staphylococcus spp., a Pseudomonas spp., a Shigella spp., a Treponema spp., or a Salmonella spp.

Illustrative examples of fungal species that can be screened for with the compositions and methods contemplated herein include, but are not limited to: an Aspergillis spp., a Blastomyces spp., a Candida spp., a Coccicioides spp., a Cryptococcus spp., dermatophytes, a Tinea spp., a Trichophyton spp., a Microsporum spp., a Fusarium spp., a Histoplasma spp., a Mucoromycotina spp., a Pneumocystis spp., a Sporothrix spp., an Exserophilum spp., or a Cladosporium spp.

Illustrative examples of viruses that can be screened for with the compositions and methods contemplated herein include, but are not limited to: Influenza A such as H1N1, H1N2, H3N2 and H5N1 (bird flu), Influenza B, Influenza C virus, Hepatitis A virus, Hepatitis B virus, Hepatitis C virus, Hepatitis D virus, Hepatitis E virus, Rotavirus, any virus of the Norwalk virus group, enteric adenoviruses, parvovirus, Dengue fever virus, Monkey pox, Mononegavirales, Lyssavirus such as rabies virus, Lagos bat virus, Mokola virus, Duvenhage virus, European bat virus 1 & 2 and Australian bat virus, Ephemerovirus, Vesiculovirus, Vesicular Stomatitis Virus (VSV), Herpesviruses such as Herpes simplex virus types 1 and 2, varicella zoster, cytomegalovirus, Epstein-Bar virus (EBV), human herpesviruses (HHV), human herpesvirus type 6 and 8, Moloney murine leukemia virus (M-MuLV), Moloney murine sarcoma virus (MoMSV), Harvey murine sarcoma virus (HaMuSV), murine mammary tumor virus (MuMTV), gibbon ape leukemia virus (GaLV), feline leukemia virus (FLV), spumavirus, Friend murine leukemia virus, Murine Stem Cell Virus (MSCV) and Rous Sarcoma Virus (RSV), HIV (human immunodeficiency virus; including HIV type 1, and HIV type 2), visna-maedi virus (VMV) virus, the caprine arthritis-encephalitis virus (CAEV), equine infectious anemia virus (EIAV), feline immunodeficiency virus (FIV), bovine immune deficiency virus (BIV), and simian immunodeficiency virus (SIV), papilloma virus, murine gammaherpesvirus, Arenaviruses such as Argentine hemorrhagic fever virus, Bolivian hemorrhagic fever virus, Sabia-associated hemorrhagic fever virus, Venezuelan hemorrhagic fever virus, Lassa fever virus, Machupo virus, Lymphocytic choriomeningitis virus (LCMV), Bunyaviridiae such as Crimean-Congo hemorrhagic fever virus, Hantavirus, hemorrhagic fever with renal syndrome causing virus, Rift Valley fever virus, Filoviridae (filovirus) including Ebola hemorrhagic fever and Marburg hemorrhagic fever, Flaviviridae including Kaysanur Forest disease virus, Omsk hemorrhagic fever virus, Tick-borne encephalitis causing virus and Paramyxoviridae such as Hendra virus and Nipah virus, variola major and variola minor (smallpox), alphaviruses such as Venezuelan equine encephalitis virus, eastern equine encephalitis virus, western equine encephalitis virus, SARS-associated coronavirus (SARS-CoV), West Nile virus, and any encephaliltis causing virus.

Illustrative examples of genes suitable for monitoring an organ transplant in a transplant recipient that can be detected, identified, predicted, diagnosed, or monitored with the compositions and methods contemplated herein include, but are not limited to, one or more of the following genes: HLA-A, HLA-B, HLA-C, HLA-DR, HLA-DP, and HLA-DQ.

In particular embodiments, a bioinformatic analysis is used to quantify the number of genome equivalents analyzed in the cfDNA clone library; detect genetic variants in a target genetic locus; detect mutations within a target genetic locus; detect genetic fusions within a target genetic locus; or measure copy number fluctuations within a target genetic locus.

### E. COMPANION DIAGNOSTICS

For illustrative purposes only and not recited by the claim set, there is provided a companion diagnostic for a genetic disease, comprising: isolating or obtaining cfDNA from a biological sample of a subject; treating the cfDNA with one or more end-repair enzymes to generate end-repaired cfDNA; ligating one or more adaptors to each end of the end-repaired cfDNA to generate a cfDNA library; amplifying the cfDNA library to generate a cfDNA clone library; determining the number of genome equivalents in the cfDNA clone library; and performing a quantitative genetic analysis of one or more biomarkers associated with the genetic disease in the cfDNA clone library, wherein detection of, or failure to detect, at least one of the one or more biomarkers indicates whether the subject should be treated for the genetic disease.

As used herein, the term "companion diagnostic" refers to a diagnostic test that is linked to a particular anti-cancer therapy. In a particular embodiment, the diagnostic methods comprise detection of genetic lesion in a biomarker associated with in a biological sample, thereby allowing for prompt identification of patients should or should not be treated with the anti-cancer therapy.

Anti-cancer therapy includes, but is not limited to surgery, radiation, chemotherapeutics, anti-cancer drugs, and immunomodulators.

Illustrative examples of anti-cancer drugs include, but are not limited to: alkylating agents such as thiotepa and cyclophosphamide (CYTOXAN^{™}); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine resume; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin and its pegylated formulations, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK^{®}; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxoids, *e*.*g*., paclitaxel (TAXOL^{®}, Bristol-Myers Squibb Oncology, Princeton, N.J.) and doxetaxel (TAXOTERE^{®}., Rhne-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; aminopterin; xeloda; ibandronate; CPT-11; topoisomerase inhibitor RFS 2000; difluoromethylomithine (DMFO); retinoic acid derivatives such as Targretin^{™} (bexarotene), Panretin^{™} (alitretinoin); ONTAK^{™} (denileukin diftitox) ; esperamicins; capecitabine; and pharmaceutically acceptable salts, acids or derivatives of any of the above. Also included in this definition are anti-hormonal agents that act to regulate or inhibit hormone action on cancers such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and antiandrogens such as flutamide, nilutamide, bicalutamide, leuprolide, and goserelin; and pharmaceutically acceptable salts, acids or derivatives of any of the above.

Illustrative examples of immunomodulators include, but are not limited to: cyclosporine, tacrolimus, tresperimus, pimecrolimus, sirolimus, verolimus, laflunimus, laquinimod and imiquimod, as well as analogs, derivatives, salts, ions and complexes thereof.

### EXAMPLES

### EXAMPLE 1

### ACCURATE DETECTION OF RARE MUTATIONS USING TARGETED SEQUENCE CAPTURE TECHNOLOGY

### PURPOSE

The purpose of this experiment was to provide a direct proof-of-principle demonstration of rare variant detection using targeted sequence capture technology.

### BACKGROUND

Target sequence capture technology provides quantitative, sequence-based genetic analysis of nucleic acids and can be exploited to perform a combined mutational and copy number analysis of drug metabolism genes. The present inventors used targeted sequence capture technology and subsequence genetic analysis to detect rare sequence variants.

Genomic DNA inputs play a central role in rare variant detection, but quantitative analysis and control of genomic inputs places bounds on the estimated sensitivity of rare variant analysis. A genomic qPCR assay was used by the present inventors to estimate genomic inputs.

One experimental goal for rare variant analysis is to introduce 10-fold more genomic input as the target sensitivity of the assay. In other words, to measure variants with a sensitivity of 1% (1 in 100), then the experimental target is to input 1000 genomes. Downstream of sequencing, bioinformatics analysis reveals the number of unique reads, and this has the desirable quality of being both an orthogonal and a more direct measure of genomic inputs.

### SUMMARY

A cell line (ZR75-30) with known SNVs was admixed with a germ line DNA sample (NA12878) in a dilution series ranging from 1-to-1 through 1-to-1000. Target regions corresponding to known sequence differences were retrieved using targeted sequence capture technology and sequenced. Sequence variants that occur at a frequency of less than 1 per 1000 sequences were detected.

### METHODS

### Capture probes

The following table shows a collection of 62 capture probes that were used in this experiment.

**Table 1. 60 base probe sequences used in the admix proof-of-concept study**

| **Target** | **SEQ ID NO:** | **Probe Sequence** |
|---|---|---|
| BRAF | 1 | TAAACATTGGAAAGGTTTCTAATTAACCAGGAGATCCAAAAGA AAGCGGTTCAAGTAGCA |
| | 2 | GATCTCAGTTTTTTTGGTTAACTATGTATTTTGGTATATGAAGCT TCTGGGTTTTGCACA |
| MYCN | 3 | GACAGATAAGCATACATATTAACATGGATATATATGTGAATTTC ATTCAAATGGTTCTCA |
| | 4 | AGCTCTTAGCCTTTGGGGGGATGACACTCTTGAGCGGACGTGGG GACGCCTCGCTCTTTA |
| BRAF | 5 | AAGCCCCCACCGCCGCCTCTTTCCAAAATAAACACCAGCCAGC CGCCGAGCCCGGAGTCG |
| | 6 | GCCTCCCTTCCCCCTCCCCGCCCGACAGCGGCCGCTCGGGCCCC GGCTCTCGGTTATAAG |
| CDH1 | 7 | GGTGTGGCAGCCAGGGGGGCGCACTCTGCTCTGGCTGGGCCCC TTCTCCCATGTTTTCTT |
| | 8 | TTACACAACCTTTGGGCTTGGACAACACTTTGGGGTCCAAAGAA CCTAAGAGTCTTTCTG |
| EPHX2 | 9 | TGATGAAACTTGGGCTGGATGGGGCACAGGTAGGGTGCTTGTT GCTTTCAGTCAGATGAA |
| | 10 | AATGAAAGAAAAGGAGGCCAGATTGCTACTCCTGGTCCCTGCC ACACACTAGGTACCCTA |
| BRCA1 | 11 | ATTGACAATACCTACATAAAACTCTTTCCAGAATGTTGTTAAGT CTTAGTCATTAGGGAG |
| | 12 | GGATTTCCACCAACACTGTATTCATGTACCCATTTTTCTCTTAAC CTAACTTTATTGGTC |
| BRCA2 | 13 | CAAAGGGGAAAACCATCAGGACATTATTTAACAACGGAAATA TCTAACTGAAAGGCAAA |
| | 14 | CAGGCAGACCAACCAAAGTCTTTGTTCCACCTTTTAAAACTAAA TCACATTTTCACAGAG |
| MYCN | 15 | CCCCAGCCAGCGGTCCGCAACCCTTGCCGCATCCACGAAACTTT GCCCATAGCAGCGGGC |
| MYC_r1_F1 | 16 | CGACTCATCTCAGCATTAAAGTGATAAAAAAATAAATTAAAAG GCAAGTGGACTTCGGTG |
| MYC_r1_R1 | 17 | CTGTGGCGCGCACTGCGCGCTGCGCCAGGTTTCCGCACCAAGA CCCCTTTAACTCAAGAC |
| MYC_r2_F1 | 18 | TTCTACTGCGACGAGGAGGAGAACTTCTACCAGCAGCAGCAGC AGAGCGAGCTGCAGCCC |
| MYC_r2_F2 | 19 | ACCGAGCTGCTGGGAGGAGACATGGTGAACCAGAGTTTCATCT GCGACCCGGACGACGAG |
| MYC_r2_F3 | 20 | GCCGCCGCCTCAGAGTGCATCGACCCCTCGGTGGTCTTCCCCTA CCCTCTCAACGACAGC |
| MYC_r2_F4 | 21 | GGCGGCTAGGGGACAGGGGCGGGGTGGGCAGCAGCTCGAATTT CTTCCAGATATCCTCGC |
| MYC_r2_R1 | 22 | AGACGAGCTTGGCGGCGGCCGAGAAGCCGCTCCACATACAGTC CTGGATGATGATGTTTT |
| MYC_r2_R2 | 23 | AGGAGAGCAGAGAATCCGAGGACGGAGAGAAGGCGCTGGAGT CTTGCGAGGCGCAGGACT |
| MYC_r2_R3 | 24 | TAAGAGTGGCCCGTTAAATAAGCTGCCAATGAAAATGGGAAAG GTATCCAGCCGCCCACT |
| MYC_r2_R4 | 25 | TTGTATTTGTACAGCATTAATCTGGTAATTGATTATTTTAATGTA ACCTTGCTAAAGGAG |
| MYC_r3_F1 | 26 | GAGGCCACAGCAAACCTCCTCACAGCCCACTGGTCCTCAAGAG GTGCCACGTCTCCACAC |
| MYC_r3_F2 | 27 | AGAGGAGGAACGAGCTAAAACGGAGCTTTTTTGCCCTGCGTGA CCAGATCCCGGAGTTGG |
| MYC_r3_F3 | 28 | TCCAACTTGACCCTCTTGGCAGCAGGATAGTCCTTCCGAGTGGA GGGAGGCGCTGCGTAG |
| MYC_r3_R1 | 29 | GCTTGGACGGACAGGATGTATGCTGTGGCTTTTTTAAGGATAAC TACCTTGGGGGCCTTT |
| MYC_r3_R2 | 30 | GCATTTGATCATGCATTTGAAACAAGTTCATAGGTGATTGCTCA GGACATTTCTGTTAGA |
| MYC_r3_R3 | 31 | CGCCCCGCGCCCTCCCAGCCGGGTCCAGCCGGAGCCATGGGGC CGGAGCCGCAGTGAGCA |
| ERBB2rlr | 32 | CTCTGGCCCCGCCGGCCGCGGGACCTCGGCGGGGCATCCACAG GGCAGGGTCCCGCCGCT |
| ERBB2r2f | 33 | GGCATGACTTGGAGTGAGTTTGGATGGGGTGGCCAGGTCTGAG AAGGTCCCCCGCCAGTG |
| ERBB2r2r | 34 | GCAGGGCACCTTCTTCTGCCACCCACCTGTAAACAGAGGGCTCA GCCCAGCTGGAGGCAG |
| ERBB2r3f | 35 | CCCAAGATCTCCAAGTACTGGGGAACCCCAGGGAGGCCCTGGG GGGTGGCAGTGTTCCTA |
| ERBB2r3r | 36 | CTAATGCACACAAAGCCTCCCCCTGGTTAGCAGTGGCCCTGGTC AGCTCTGAATAACCAA |
| ERBB2r4f | 37 | CTGCTCCTCTTTTAGAAGGCAGGAGGGCCCCAAGGGAAGCAGA AGGTGACAGAAGGGGAA |
| ERBB2r4r | 38 | TGGGGCAGTGGCGGGCAGGCACTGGGTTGTAAGTTGGGAGTTT GCGGCTGGGGTCAGGCT |
| ERBB2r5f | 39 | TCTGCTGCTGTTTGTGCCTCTCTCTGTTACTAACCCGTCCTCTCG CTGTTAGACATCTCT |
| ERBB2r5r | 40 | CCCACCCCTCCCATGTCACCTGTATGACACCTGCATTCCACCCG GCCCCAGCCCTCCCCT |
| ERBB2r6f | 41 | TGGGCCAGGTAGTCTCCCTAGAAGGTGATGCTGATGAGGGTCT GGTGCCCAGGGCGCCAC |
| ERBB2r6r | 42 | GGTGCCCACCCCTTGCATCCTGGGGGGTAGAGCACATTGGGCA CAAAGCAGAGGCACATA |
| ERBB2r7f | 43 | CACCCTGCCTGGTACTGCCCTATTGCCCCTGGCACACCAGGGCA AAACAGCACAGTGAAA |
| ERBB2r7r | 44 | CCATTTACAGAAACAAACCTCCCCACCAAAATGAGAAAACTGT GTTTCTCCCTGGCACTC |
| ERBB2r8f | 45 | TTATTCTTCTTGTGCCTGGGCACGGTAATGCTGCTCATGGTGGT GCACGAAGGGCCAGGG |
| ERBB2r8r | 46 | GAAGGATAGGACAGGGTGGGCTGGGCCAGGCTGCATGCGCAG AGGGACAGGAACTGCAGC |
| ERBB2r9f | 47 | GGGCCCGGACCCTGATGCTCATGTGGCTGTTGACCTGTCCCGGT ATGAAGGCTGAGACGG |
| ERBB2r9r | 48 | TCTGTCTCCTGCCATCCCCAAGAGATGCTGCCACATCTGGATCC TCAGGACTCTGTCTGC |
| TYMSr2f | 49 | TCACGTCCCAGGCAGTTTCTTCCCTGAAGAAAGTTGGATGGC ATGATCTGTCTTCCCA |
| TYMSr2r | 50 | GTGTTGAGAACAGACTACTGACTTCTAATAGCAGCGACTTCTTT ACCTTGATAAACCACA |
| TYMSr3f | 51 | AAAAAAAGGATGGGTTCCATATGGGTGGTGTCAAGTGCCCACC TCCTAGCAAGTCAGCAG |
| TYMSr3r | 52 | CCCTCACAAGGTCAAAGCTATACATCAGCTCCTGTGACATTGAC TCATCCCCCAGACCTT |
| TYMSr4f | 53 | AACCCACCGAGATCTGCAAACTTTGCAGGATGCACCAGATGTC TTGTAGCCATGGGTCAA |
| TYMSr4r | 54 | TGCCTCCCTCAGGTGCCTCTGCACAAAACCAGATTGCTTCCCTC TAAGAGTATGGTTAGT |
| TYMSr5f | 55 | GTTTTACTTTGCCTTTAGCTGTGGTCTTTCAAACCACCATCCCTC CTTATCTTCCTCTGC |
| TYMSr5r | 56 | CTCTGCAATTTGTTTTCCCATATTAAAGAACTGAAGAGCTCAGT GTGGTAGGCTGGCAAG |
| TYMSr6f | 57 | TTTTAAATGATGTTTTAAAGAATTGAAACTAACATACTGTTCTG CTTTCTCCCCGGGTT |
| TYMSr6r | 58 | CCTGCCCACCACTTCTCCCTAAACTGAAGCCCCACATTTGGAGC AGTCATCTTTATCTTG |
| TYMSr7f | 59 | GGTTGCGCTCCAATCATGTTACATAACCTACGGCAAGGTATCGA CAGGATCATACTCCTG |
| TYMSr7r | 60 | GCACAGTTACATTTGCCAGTGGCAACATCCTTAAAAATTAATAA CTGATAGGTCACGGAC |
| TYMSr1f | 61 | CGTCCCGCCGCGCCACTTGGCCTGCCTCCGTCCCGCCGCGCCAC TTCGCCTGCCTCCGTC |
| TYMSr1r | 62 | CTGTAAGGCGAGGAGGACGATGCGTCCCCTCCCTCGCAGGATT GAGGTTAGGACTAAACG |

Capture probe modules were pooled from stock plates, combined with partner oligo # 138 (SEQ ID NO:63) (GTGAAAACCAGGATCAACTCCCGTGCCAGTCACAT/3BioTEG/) and diluted to a final working concentration of 1 nM.

### Genomic samples

Commercially-purchased genomic DNA from germ line sample NA12878 and cell line ZR75-30 was fragmented at a concentration of 10-20 ng/µl to a target fragment size of 500 bp on a Covaris sonication instrument. The DNA was purified with a 1:1 concentration of DNA purification beads and end-repaired using the New England Biolabs (NEB) Quick blunt kit at a final concentration of 15 - 30 ng/µL. The germ line and cell line DNAs were blended at ratios of 1: 1, 10: 1, 100:1 and 1000: 1, respectively. Libraries were constructed, purified and quantified. The sample codes, library quantitation and inputs used for library construction are shown in Table 2.

**Table 2. Adaptors and genomic analysis of libraries used as inputs**

| **Admix** | **Adaptor code** | **genomes/µl** | **# desired genomes** | **µL into PCR** |
|---|---|---|---|---|
| 1:1 | NNNNNNNNCATGGCCGCAGG (SEQ ID NO:64) | 55 | 200 | 4 |
| 10:1 | NNNNNNNNATCTTAGTGGCA (SEQ ID NO:65) | 66 | 200 | 3 |
| 100:1 | NNNNNNNNCGGAACTCGGAG (SEQ ID NO:66) | 64 | 1000 | 16 |
| 1000:1 | NNNNNNNNGACTCCGATCCC (SEQ ID NO:67) | 77 | 10000 | 130 |

Genomic libraries were pooled, denatured, combined with probe, hybridized and washed. The washed capture probe-tagged genomic library **complexes** were amplified with **forward and reverse full-length primers,** purified, and size-selected for 225-600 bp fragments on a Pippin-prep instrument. Finally, the captured material was sequenced using a 150-V3 Illumina sequencing kit.

### RESULTS

The paired capture probes that target BRAF (in two loci), MYCN and CDH1 were used to analyze the SNVs in these loci. The results are shown in Table 3.

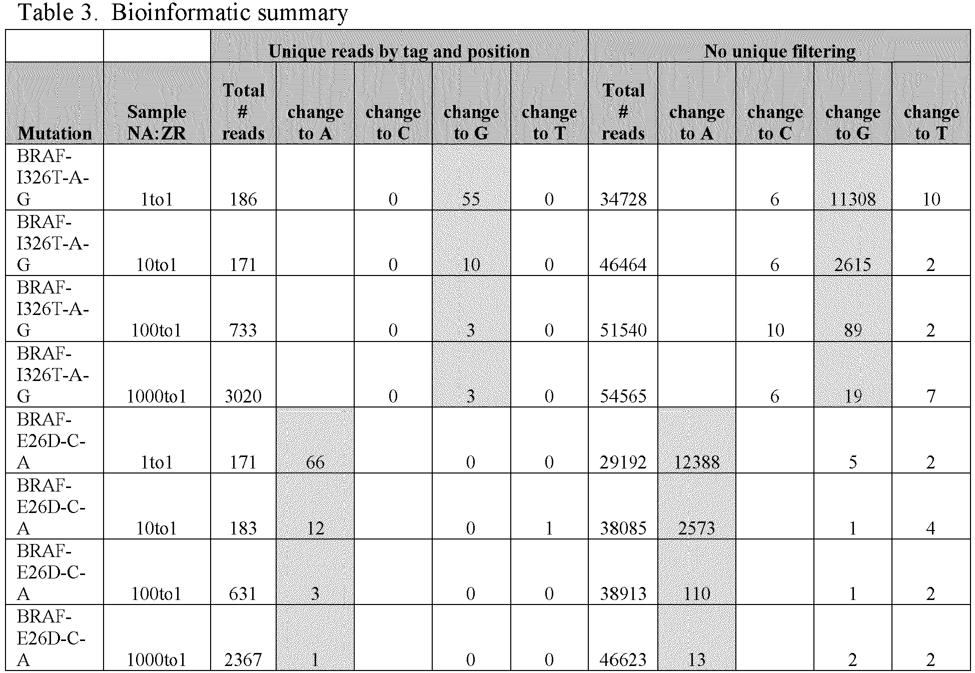

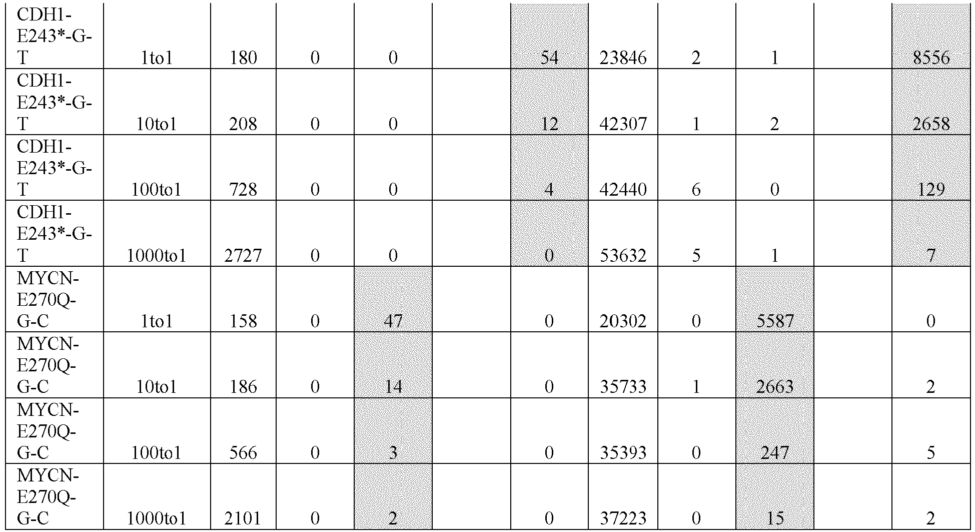

Column 3 shows the total number unique read counts, which in turn provide bounds on the sensitivity of the assay. The estimated and measured genomic inputs were well within range of one another. The lightly shaded boxes highlight the SNV where the cell line sequence differed from the germ line sequence. In the absence of unique read filtering - shown on the right portion of the table - random base changes at these four selected positions occurred with measurable, non-zero frequencies. Figure 1. By requiring that changes occur within unique read families, it became possible to sort true signal from error-prone noise. Figure 2.

### EXAMPLE 2

### A NOVEL PROBE DESIGN EFFECTIVE FOR COMPREHENSIVE SEQUENCING OF TARGET REGIONS IN HIGHLY FRAGMENTED GDNA

### PURPOSE

The purpose of these experiments is to develop an assay system to reliably and reproducibly interrogate circulating DNAs.

### BACKGROUND

Analysis of circulating DNA from body fluids represents an exciting, but as yet, unrealized opportunity in molecular diagnostics. Genomic DNA is highly intact. Literature suggest that the average size of circulating DNA is about 150 bp, which correlates well to the size of DNAs wrapped around a single nucleosomal histone complex.

### SUMMARY

The technical parameters of targeted sequence capture technology contemplated herein were designed to accommodate highly fragmented DNA and to retain the ability to generate comprehensive sequence coverage of targeted DNA. Capture probe density was increased and the length of capture probe sequences was reduced from 60 nucleotide to 40 nucleotide to minimize uninformative sequence generation in the clone library. The human genome is littered with repetitive sequences and drastic fluctuations in base composition, thus, the suitability of implementing higher capture probe densities and shorter capture probes could not be conceded but required empirical validation of the new assay.

Conditions were established in which the shorter 40 mer capture probe sequences exhibit reliable and robust assay performance. In a first set of experiments, the assays were used to query two large regions - the coding regions for the tumor suppressor gene TP53 and the long, contiguous, intron 19 of the ALK oncogene, both of which are central to cancer diagnostics. In a second set of experiments, several high density pairwise capture probes that possess shorter 40 nucleotide capture probe sequences were used to interrogate known SNVs that reside in the NCI-H69 cell line.

The new high density shorter capture probes were successfully used to query short fragmented DNAs and the results indicated that the assay design is well suited to sequencing of circulating DNAs that are found in the plasma fraction of blood.

### METHODS - MODIFIED 40 MER CAPTURE PROBES

The capture probe sequences used to empirically validate the performance of the 40 mer capture probes are shown in Table 4.

| **60 mer Capture Probes** | | |
|---|---|---|
| **Name** | **SEQ ID NO:** | **Sequence** |
| PLP1_ex2 _F | 68 | ATGTGACTGGCACGGGAGTTGATCCTGGTTTTCACGGGTTTGAGT GGCATGAGCTACCTACTGGATGTGCCTGACTGTTTCCCCTTCTTCT TCCC |
| PLP1_ex2 _R | 69 | ATGTGACTGGCACGGGAGTTGATCCTGGTTTTCACCTATCTCCAG GATGGAGAGAGGGAAAAAAAAGATGGGTCTGTGTGGGAGGGCA7 0GGTACTT |
| PLP1_ex3 _F | 70 | ATGTGACTGGCACGGGAGTTGATCCTGGTTTTCACGAAAGAAGCC AGGTCTTCAATTAATAAGATTCCCTGGTCTCGTTTGTCTACCTGTT AATG |
| PLP1_ex3 _M | 71 | ATGTGACTGGCACGGGAGTTGATCCTGGTTTTCACCAGACTCGCG CCCAATTTTCCCCCACCCCTTGTTATTGCCACAAAATCCTGAGGAT GATC |
| CYP2D6_ F | 72 | ATGTGACTGGCACGGGAGTTGATCCTGGTTTTCACAAGCACCTAG CCCCATTCCTGCTGAGCAGGAGGTGGCAGGTACCCCAGACTGGGA GGTAA |
| CYP2D6_ R | 73 | ATGTGACTGGCACGGGAGTTGATCCTGGTTTTCACAGTCGGTGGG GCCAGGATGAGGCCCAGTCTGTTCACACATGGCTGCTGCCTCTCA GCTCT |
| chrX_15_ F | 74 | ATGTGACTGGCACGGGAGTTGATCCTGGTTTTCACCCTGGCCCTC AGCCAGTACAGAAAGTCATTTGTCAAGGCCTTCAGTTGGCAGACG TGCTC |
| chrX_15_ R | 75 | ATGTGACTGGCACGGGAGTTGATCCTGGTTTTCACAGAATTCATT GCCAGCTATAAATCTGTGGAAACGCTGCCACACAATCTTAGCACA CAAGA |
| chrX_69_ F | 76 | ATGTGACTGGCACGGGAGTTGATCCTGGTTTTCACTTACTTCCCTC CAGTTTTGTTGCTTGCAAAACAACAGAATCTTCTCTCCATGAAATC ATG |
| chrX_69_ R | 77 | ATGTGACTGGCACGGGAGTTGATCCTGGTTTTCACCAGGGGTATC TATTATCCCCATTTTCTCACAAAGGAAACCAAGATAAAAGGTTTA AATGG |
| KRAS_ex 1_F | 78 | ATGTGACTGGCACGGGAGTTGATCCTGGTTTTCACTGTTACCTTTA AAAGACATCTGCTTTCTGCCAAAATTAATGTGCTGAACTTAAACT TACC |
| KRAS_ex 1_R | 79 | ATGTGACTGGCACGGGAGTTGATCCTGGTTTTCACTTCCCAGTAA ATTACTCTTACCAATGCAACAGACTTTAAAGAAGTTGTGTTTTAC AATGC |
| KRAS_ex 2_F | 80 | ATGTGACTGGCACGGGAGTTGATCCTGGTTTTCACTAAATGACAT AACAGTTATGATTTTGCAGAAAACAGATCTGTATTTATTTCAGTGT TACT |
| KRAS_ex 2_R | 81 | ATGTGACTGGCACGGGAGTTGATCCTGGTTTTCACGACAGGTTTT GAAAGATATTTGTGTTACTAATGACTGTGCTATAACTTTTTTTTCT TTCC |
| MYC_r2_ F1 | 82 | ATGTGACTGGCACGGGAGTTGATCCTGGTTTTCACCTGTGGCGCG CACTGCGCGCTGCGCCAGGTTTCCGCACCAAGACCCCTTTAACTC AAGAC |
| MYC_r2_ R1 | 83 | ATGTGACTGGCACGGGAGTTGATCCTGGTTTTCACGGCGGCTAGG GGACAGGGGCGGGGTGGGCAGCAGCTCGAATTTCTTCCAGATATC CTCGC |
| MYC_r2_ F3 | 84 | ATGTGACTGGCACGGGAGTTGATCCTGGTTTTCACACCGAGCTGC TGGGAGGAGACATGGTGAACCAGAGTTTCATCTGCGACCCGGAC GACGAG |
| MYC_r2_ R3 | 85 | ATGTGACTGGCACGGGAGTTGATCCTGGTTTTCACAGGAGAGCAG AGAATCCGAGGACGGAGAGAAGGCGCTGGAGTCTTGCGAGGCGC AGGACT |
| SRY_r1_F | 86 | ATGTGACTGGCACGGGAGTTGATCCTGGTTTTCACCTGTAAGTTA TCGTAAAAAGGAGCATCTAGGTAGGTCTTTGTAGCCAATGTTACC CGATT |
| SRY_r1_ M3 | 87 | ATGTGACTGGCACGGGAGTTGATCCTGGTTTTCACAATGGCCATT CTTCCAGGAGGCACAGAAATTACAGGCCATGCACAGAGAGAAAT ACCCGA |
| VHL_r3_F | 88 | ATGTGACTGGCACGGGAGTTGATCCTGGTTTTCACCTTGTTCGTTC CTTGTACTGAGACCCTAGTCTGCCACTGAGGATTTGGTTTTTGCCC TTC |
| VHL_r3_ R | 89 | ATGTGACTGGCACGGGAGTTGATCCTGGTTTTCACATCAAGACTC ATCAGTACCATCAAAAGCTGAGATGAAACAGTGTAAGTTTCAACA GAAAT |
| UGT1A1_ r_4F | 90 | ATGTGACTGGCACGGGAGTTGATCCTGGTTTTCACTGTGTCCAGC TGTGAAACTCAGAGATGTAACTGCTGACATCCTCCCTATTTTGCAT CTCA |
| UGT1A1_ r_4R | 91 | ATGTGACTGGCACGGGAGTTGATCCTGGTTTTCACATTTGAAACA ATTTTATCATGAATGCCATGACCAAAGTATTCTTCTGTATCTTCTT TCTT |
| TNFRSF1 4_r3_F | 92 | ATGTGACTGGCACGGGAGTTGATCCTGGTTTTCACTGATGGGTGG GCTCCCGAAGGGGCCTCCCGCAGACTTGCGAAGTTCCCACTCTCT GGGCG |
| TNFRSF1 4_r3_R | 93 | ATGTGACTGGCACGGGAGTTGATCCTGGTTTTCACCAGGGTGCGG GGGCATCCAGGCTGCCCAAGCGGAGGCTGGGCCGGCTGTGCTGG CCTCTT |
| RUNX1_r 4_F | 94 | ATGTGACTGGCACGGGAGTTGATCCTGGTTTTCACTTTTGAAATGT GGGTTTGTTGCCATGAAACGTGTTTCAAGCATAGTTTTGACAGAT AACG |
| RUNX1_r 4_R | 95 | ATGTGACTGGCACGGGAGTTGATCCTGGTTTTCACTGCCCTAAAA GTGTATGTATAACATCCCTGATGTCTGCATTTGTCCTTTGACTGGT GTTT |
| RHD_r5_ F | 96 | ATGTGACTGGCACGGGAGTTGATCCTGGTTTTCACAACCCCTCGA GGCTCAGACCTTTGGAGCAGGAGTGTGATTCTGGCCAACCACCCT CTCTG |
| RHD_r5_ R | 97 | ATGTGACTGGCACGGGAGTTGATCCTGGTTTTCACCATAAATATG TGTGCTAGTCCTGTTAGACCCAAGTGCTGCCCAAGGGCAGCGCCC TGCTC |
| PTEN_r5_ F | 98 | ATGTGACTGGCACGGGAGTTGATCCTGGTTTTCACTACTTGTTAAT TAAAAATTCAAGAGTTTTTTTTTCTTATTCTGAGGTTATCTTTTTAC CA |
| PTEN_r5_ R | 99 | ATGTGACTGGCACGGGAGTTGATCCTGGTTTTCACCCAAAATCTG TTTTCCAATAAATTCTCAGATCCAGGAAGAGGAAAGGAAAAACA TCAAAA |
| EP300_r1 8_F | 100 | ATGTGACTGGCACGGGAGTTGATCCTGGTTTTCACATACTCCATCT CCCGTAAAAATAGTGAGACTTGAGTAATGTTTGATGTCACTTGTC TTTC |
| EP300_r1 8_R | 101 | ATGTGACTGGCACGGGAGTTGATCCTGGTTTTCACCAGTCACCAC TATATTATTCTAGGTATCCCAGAAAAGTTAAAGTCAAATCTGAAA CACAT |
| VHL_r1_F | 102 | ATGTGACTGGCACGGGAGTTGATCCTGGTTTTCACCGCCCCGCGT CCGACCCGCGGATCCCGCGGCGTCCGGCCCGGGTGGTCTGGATCG CGGAG |
| VHL_r1_ R | 103 | ATGTGACTGGCACGGGAGTTGATCCTGGTTTTCACCCATACGGGC AGCACGACGCGCGGACTGCGATTGCAGAAGATGACCTGGGAGGG CTCGCG |
| VHL_r1_ M1 | 104 | ATGTGACTGGCACGGGAGTTGATCCTGGTTTTCACTAGAGGGGCT TCAGACCGTGCTATCGTCCCTGCTGGGTCGGGCCTAAGCGCCGGG CCCGT |
| VHL_r1_ M2 | 105 | ATGTGACTGGCACGGGAGTTGATCCTGGTTTTCACGGCGCCGAGG AGGAGATGGAGGCCGGGCGGCCGCGGCCCGTGCTGCGCTCGGTG AACTCG |
| VHL_r2_F | 106 | ATGTGACTGGCACGGGAGTTGATCCTGGTTTTCACGGTGTGGGCC ACCGTGCCCAGCCACCGGTGTGGCTCTTTAACAACCTTTGCTTGTC CCGA |
| VHL_r2_ R | 107 | ATGTGACTGGCACGGGAGTTGATCCTGGTTTTCACAAGTGGTCTA TCCTGTACTTACCACAACAACCTTATCTTTTTAAAAAGTAAAACGT CAGT |
| VHL_r3_F | 108 | ATGTGACTGGCACGGGAGTTGATCCTGGTTTTCACCTTGTTCGTTC CTTGTACTGAGACCCTAGTCTGCCACTGAGGATTTGGTTTTTGCCC TTC |
| VHL_r3_ R | 109 | ATGTGACTGGCACGGGAGTTGATCCTGGTTTTCACATCAAGACTC ATCAGTACCATCAAAAGCTGAGATGAAACAGTGTAAGTTTCAACA GAAAT |

| **40 mers** | | |
|---|---|---|
| **Name** | **SEQ ID NO:** | **Sequence** |
| PLP1_ex2 F 40 | 110 | ATGTGACTGGCACGGGAGTTGATCCTGGTTTTCACACCTACTGGA TGTGCCTGACTGTTTCCCCTTCTTCTTCCC |
| PLP1_ex2 R 40 | 111 | ATGTGACTGGCACGGGAGTTGATCCTGGTTTTCACGGGAAAAAAA AGATGGGTCTGTGTGGGAGGGCAGGTACTT |
| PLP1_ex3 F 40 | 112 | ATGTGACTGGCACGGGAGTTGATCCTGGTTTTCACTTAATAAGAT TCCCTGGTCTCGTTTGTCTACCTGTTAATG |
| PLP1_ex3 M 40 | 113 | ATGTGACTGGCACGGGAGTTGATCCTGGTTTTCACCCCCACCCCTT GTTATTGCCACAAAATCCTGAGGATGATC |
| CYP2D6_ F 40 | 114 | ATGTGACTGGCACGGGAGTTGATCCTGGTTTTCACGCTGAGCAGG AGGTGGCAGGTACCCCAGACTGGGAGGTAA |
| CYP2D6_ R 40 | 115 | ATGTGACTGGCACGGGAGTTGATCCTGGTTTTCACGGCCCAGTCT GTTCACACATGGCTGCTGCCTCTCAGCTCT |
| chrX_15_ F 40 | 116 | ATGTGACTGGCACGGGAGTTGATCCTGGTTTTCACGAAAGTCATT TGTCAAGGCCTTCAGTTGGCAGACGTGCTC |
| chrX_15_ R 40 | 117 | ATGTGACTGGCACGGGAGTTGATCCTGGTTTTCACAATCTGTGGA AACGCTGCCACACAATCTTAGCACACAAGA |
| chrX_69_ F 40 | 118 | ATGTGACTGGCACGGGAGTTGATCCTGGTTTTCACTGCTTGCAAA ACAACAGAATCTTCTCTCCATGAAATCATG |
| chrX_69_ R 40 | 119 | ATGTGACTGGCACGGGAGTTGATCCTGGTTTTCACATTTTCTCACA AAGGAAACCAAGATAAAAGGTTTAAATGG |
| KRAS_ex 1_F_40 | 120 | ATGTGACTGGCACGGGAGTTGATCCTGGTTTTCACTGCTTTCTGCC AAAATTAATGTGCTGAACTTAAACTTACC |
| KRAS_ex 1_R_40 | 121 | ATGTGACTGGCACGGGAGTTGATCCTGGTTTTCACCCAATGCAAC AGACTTTAAAGAAGTTGTGTTTTACAATGC |
| KRAS_ex 2 F 40 | 122 | ATGTGACTGGCACGGGAGTTGATCCTGGTTTTCACATTTTGCAGA AAACAGATCTGTATTTATTTCAGTGTTACT |
| KRAS_ex 2 R 40 | 123 | ATGTGACTGGCACGGGAGTTGATCCTGGTTTTCACTGTGTTACTA ATGACTGTGCTATAACTTTTTTTTCTTTCC |
| MYC_r2_ F1_40 | 124 | ATGTGACTGGCACGGGAGTTGATCCTGGTTTTCACTGCGCCAGGT TTCCGCACCAAGACCCCTTTAACTCAAGAC |
| MYC_r2_ R1 40 | 125 | ATGTGACTGGCACGGGAGTTGATCCTGGTTTTCACGGGGTGGGCA GCAGCTCGAATTTCTTCCAGATATCCTCGC |
| MYC_r2_ F3 40 | 126 | ATGTGACTGGCACGGGAGTTGATCCTGGTTTTCACCATGGTGAAC CAGAGTTTCATCTGCGACCCGGACGACGAG |
| MYC_r2_ R3 40 | 127 | ATGTGACTGGCACGGGAGTTGATCCTGGTTTTCACGACGGAGAGA AGGCGCTGGAGTCTTGCGAGGCGCAGGACT |
| SRY_r1_F 40 | 128 | ATGTGACTGGCACGGGAGTTGATCCTGGTTTTCACGAGCATCTAG GTAGGTCTTTGTAGCCAATGTTACCCGATT |
| SRY_r1_ M3_40 | 129 | ATGTGACTGGCACGGGAGTTGATCCTGGTTTTCACGCACAGAAAT TACAGGCCATGCACAGAGAGAAATACCCGA |
| VHL_r3_F 40 | 130 | ATGTGACTGGCACGGGAGTTGATCCTGGTTTTCACAGACCCTAGT CTGCCACTGAGGATTTGGTTTTTGCCCTTC |
| VHL_r3_ R 40 | 131 | ATGTGACTGGCACGGGAGTTGATCCTGGTTTTCACTCAAAAGCTG AGATGAAACAGTGTAAGTTTCAACAGAAAT |
| UGT1A1_r_4F_40 | 132 | ATGTGACTGGCACGGGAGTTGATCCTGGTTTTCACAGAGATGTAA |
| | | CTGCTGACATCCTCCCTATTTGCATCTCA |
| UGT1A1_ r_4R_40 | 133 | ATGTGACTGGCACGGGAGTTGATCCTGGTTTTCACGAATGCCATG ACCAAAGTATTCTTCTGTATCTTCTTTCTT |
| TNFRSF1 4_r3_F_40 | 134 | ATGTGACTGGCACGGGAGTTGATCCTGGTTTTCACGGGCCTCCCG CAGACTTGCGAAGTTCCCACTCTCTGGGCG |
| TNFRSF1 4_r3_R_4 0 | 135 | ATGTGACTGGCACGGGAGTTGATCCTGGTTTTCACGCTGCCCAAG CGGAGGCTGGGCCGGCTGTGCTGGCCTCTT |
| RUNX1_r 4_F_40 | 136 | ATGTGACTGGCACGGGAGTTGATCCTGGTTTTCACGCCATGAAAC GTGTTTCAAGCATAGTTTTGACAGATAACG |
| RUNX1_r 4_R_40 | 137 | ATGTGACTGGCACGGGAGTTGATCCTGGTTTTCACAACATCCCTG ATGTCTGCATTTGTCCTTTGACTGGTGTTT |
| RHD_r5_ F_40 | 138 | ATGTGACTGGCACGGGAGTTGATCCTGGTTTTCACTTTGGAGCAG GAGTGTGATTCTGGCCAACCACCCTCTCTG |
| RHD_r5_ R_40 | 139 | ATGTGACTGGCACGGGAGTTGATCCTGGTTTTCACCTGTTAGACC CAAGTGCTGCCCAAGGGCAGCGCCCTGCTC |
| PTEN_r5_ F_40 | 140 | ATGTGACTGGCACGGGAGTTGATCCTGGTTTTCACAAGAGTTTTTT TTTCTTATTCTGAGGTTATCTTTTTACCA |
| PTEN_r5_ R_40 | 141 | ATGTGACTGGCACGGGAGTTGATCCTGGTTTTCACAATTCTCAGA TCCAGGAAGAGGAAAGGAAAAACATCAAAA |
| EP300_r1 8_F_40 | 142 | ATGTGACTGGCACGGGAGTTGATCCTGGTTTTCACATAGTGAGAC TTGAGTAATGTTTGATGTCACTTGTCTTTC |
| EP300_r1 8_R_40 | 143 | ATGTGACTGGCACGGGAGTTGATCCTGGTTTTCACTAGGTATCCC AGAAAAGTTAAAGTCAAATCTGAAACACAT |
| VHL_r1_F 40 | 144 | ATGTGACTGGCACGGGAGTTGATCCTGGTTTTCACGATCCCGCGG CGTCCGGCCCGGGTGGTCTGGATCGCGGAG |
| VHL_r1_ R_40 | 145 | ATGTGACTGGCACGGGAGTTGATCCTGGTTTTCACGCGGACTGCG ATTGCAGAAGATGACCTGGGAGGGCTCGCG |
| VHL_r1_ M1_40 | 146 | ATGTGACTGGCACGGGAGTTGATCCTGGTTTTCACCTATCGTCCCT GCTGGGTCGGGCCTAAGCGCCGGGCCCGT |
| VHL_r1_ M2 40 | 147 | ATGTGACTGGCACGGGAGTTGATCCTGGTTTTCACGGCCGGGCGG CCGCGGCCCGTGCTGCGCTCGGTGAACTCG |
| VHL_r2_F 40 | 148 | ATGTGACTGGCACGGGAGTTGATCCTGGTTTTCACGCCACCGGTG TGGCTCTTTAACAACCTTTGCTTGTCCCGA |
| VHL_r2_ R_40 | 149 | ATGTGACTGGCACGGGAGTTGATCCTGGTTTTCACACCACAACAA CCTTATCTTTTTAAAAAGTAAAACGTCAGT |
| VHL_r3_F 40 | 150 | ATGTGACTGGCACGGGAGTTGATCCTGGTTTTCACAGACCCTAGT CTGCCACTGAGGATTTGGTTTTTGCCCTTC |
| VHL_r3_ R_40 | 151 | ATGTGACTGGCACGGGAGTTGATCCTGGTTTTCACTCAAAAGCTG AGATGAAACAGTGTAAGTTTCAACAGAAAT |

The performance of 40 mer capture probes was compared to that of 60 mer capture probes. The 40 mer was designed from the 60 mer by removing 20 nucleotides from the 5' end of the 60 mer. Although the 3' end of both capture probe sets are identical with respect to the sequences that are copied from captured genomic clones, the probe sequence signature (Read 2 of the paired end read) is different between the 60 mer and 40 mer probe sets. This design is useful because it allows the capture probes to be multiplexed during sequencing and their performance subsequently analyzed during downstream bioinformatics deconvolution.

### Genomic samples

A pool of 12 genomic DNA samples (chosen from a Coriell human panel of 112 human genomic DNAs) was used as the target DNA. The 12 samples were broken into four sets of four samples each, as shown in detail in Table 5.

| **Capture probes** | **Wash temp.** | **Sample Code** | **Samples** | | | |
|---|---|---|---|---|---|---|
| 60mer | 50C | AAT | GM20291 | M | Americas | AFRICAN ANCESTRY IN SOUTHWEST USA |
| | | CTA | GM19373 | M | WAfrican | LUHYA IN WEBUYE, KENYA |
| | | GGG | HG00428 | F | AsianE | HAN CHINESE SOUTH |
| | | TCC | HG01624 | M | Euro | IBERIAN POPULATIONS IN SPAIN |

| | | | | | | |
|---|---|---|---|---|---|---|
| 40mer | 50C | AAT | GM20291 | M | Americas | AFRICAN ANCESTRY IN SOUTHWEST USA |
| | | CTA | GM19373 | M | WAfrican | LUHYA IN WEBUYE, KENYA |
| | | GGG | HG00428 | F | AsianE | HAN CHINESE SOUTH |
| | | TCC | HG01624 | M | Euro | IBERIAN POPULATIONS IN SPAIN |

| | | | | | | |
|---|---|---|---|---|---|---|
| 60mer | 47C | AGA | HG02489 | M | Americas | AFRICAN CARIBBEAN IN BARBADOS |
| | | CCT | HG01108 | F | Americas | PUERTO RICAN IN PUERTO RICO |
| | | GAC | GM19011 | F | AsianE | JAPANESE IN TOKYO, JAPAN |
| | | TTG | GM18946 | F | AsianE | JAPANESE IN TOKYO, JAPAN |

| | | | | | | |
|---|---|---|---|---|---|---|
| 40mer | 47C | AGA | HG02489 | M | Americas | AFRICAN CARIBBEAN IN BARBADOS |
| | | CCT | HG01108 | F | Americas | PUERTO RICAN IN PUERTO RICO |
| | | GAC | GM19011 | F | AsianE | JAPANESE IN TOKYO, JAPAN |
| | | TTG | GM18946 | F | AsianE | JAPANESE IN TOKYO, JAPAN |

| | | | | | | |
|---|---|---|---|---|---|---|
| 60mer | 44C | ATC | NA13783 | F | NA13783 | GM13783 |
| | | CAG | HG03700 | F | AsianS | PUNJABI IN LAHORE, PAKISTAN |
| | | GCA | HG03367 | M | WAfrican | ESAN FROM NIGERIA |
| | | TGT | NA22991 | F | NA22991 | GM22991 |

| | | | | | | |
|---|---|---|---|---|---|---|
| 40mer | 44C | ATC | NA13783 | F | NA13783 | GM13783 |
| | | CAG | HG03700 | F | AsianS | PUNJABI IN LAHORE, PAKISTAN |
| | | GCA | HG03367 | M | WAfrican | ESAN FROM NIGERIA |
| | | TGT | NA22991 | F | NA22991 | GM22991 |

### Hybridization, washing and sequencing

Six different hybridization conditions were used to hybridize the 60 mer and 40 mer probes to the genomic target DNA:
1) 60mer probes washed at 50° C.
2) 40mer probes washed at 50° C.
3) 60mer probes washed at 47° C.
4) 40mer probes washed at 47° C.
5) 60mer probes washed at 44° C.
6) 40mer probes washed at 44° C.
For each experiment, the capture probe oligos were combined with partner oligo; the final concentration of duplex capture probe was 1 nM for each capture probe.

Each hybridization reaction had ~ 2.5 µg of genomic library in 40 µl total volume. Each sample was heated to 98° C for 2 min then cooled on ice. 20 µl of capture probe and 90 µl of hybridization buffer were added and the hybridization mix was incubated for 24 hours starting at 80° C and decreasing one degree every 48 minutes to 50° C. The complexes were bound to 20 ul of streptavidin beads in 1 mL total volume of TEzero buffer + 0.05% Tween20 (TT). The beads were washed 3 times, 5 min each with 200 ul of TT, and once at 45° C for 5 min in wash buffer. The beads were then washed with TEzero and each reaction was resuspended in 20 µl TEzero. The complexes were then PCR amplified with full length forward (ACA2 FLFP; SEQ ID NO:152; AATGATACGGCGACCACCGAGATCTACACGTCATGCAGGACCAGAGAATTC GAATACA) and full length reverse (CAC3_FLRP; SEQ ID NO:153; CAAGCAGAAGACGGCATACGAGATGTGACTGGCACGGGAGTTGATCCTGGT TTTCAC) primers.

Following amplification and purification, the resulting product masses were measured and equal masses were pooled for sequencing.

### RESULTS - MODIFIED 40MER PRIMERS

The capture probe performance as a function of length and wash temperature is shown graphically in Figure 3. Overall, the 40 mer capture probes performed as well as the 60 mer capture probes with 44° C and 47° C washes. With the 50° C wash, the 40 mer capture probes exhibit sporadic behavior. These data empirically validate the use of 40 mer capture probes and wash temperatures in the 44° C to 47° C range when using these reagents.

### METHODS - HIGH DENSITY 40 MERS

In general, sequence capture probes are designed using specific "rules." For example, regions of redundant sequence or that exhibit extreme base composition biases are generally avoided. One key implication of the requirement for high probe density and close spacing of probes along target regions is that there is little or no latitude to move probes in order to accommodate any such probe design rules. In this study, probes were designed based solely on their position relative to one another with no consideration of probe binding sequences; thus, use of this high density approach required empirically validating that the hybridization and processing methods would accommodate such a collection of probes.

The human ALK gene encodes a protein kinase important in early development, but normal ALK gene expression is essentially undetectable in normal adults. Oncogenic ALK fusions are created when intron 19 of ALK undergoes illegitimate recombination to fuse the kinase encoding portion of ALK to the 5' end of another gene. Such gene fusions often cause ectopic expression of the ALK kinase, which in turn is important in driving the inappropriate cell proliferation observed in pulmonary tumors. In lung cancer, this "other gene" is often EML4, but other fusion partners have also been detected. To create an assay that can detect any possible ALK gene fusion event, 40 nucleotide probes were designed that were placed at 80 nucleotide intervals in intron 19 of ALK. These probes were oriented such that they are antisense relative to the gene (Figure 4). This means that their 3" terminus extends and copies genic regions that are 5' to their hybridization site. When fusion genes are present, probe extension from probes positioned near the fusion junction copy junction sequences. The DNA sequences resulting from these junction clones have fusion partner sequences at their 5' end, the fusion junction sequence, and ALK intron 19 sequences at their 3' ends (Figure 4B).

Another important diagnostic target in cancer is the TP53 gene. It encodes a tumor suppressor, and it is often inactivated by mutations in cancers. Mutations that can inactivate gene function are scattered throughout the gene, and hence conclusive sequence-based assays for TP53 inactivating mutations must address the entire coding region and untranslated regions (UTRs) of the gene. Because circulating DNA fragments are short, high density probes were used to interrogate all target regions of the TP53 gene. Unlike ALK, probes for TP53 are placed in both possible orientations (Figure 5). At high probe densities, the cumulative coverage from multiple probes provides uniform deep sequencing coverage of target regions.

The collection of 105 probes used in this study is shown in Table 6. In addition to probes that target the fusion-prone region of ALK and the coding regions of TP53, probes that cover known SNVs in the cell line DNA were also included.

**Table 6.**

| **Name_target region** | **SEQ ID NO**: | **Probe sequence** |
|---|---|---|
| ALK_chr2:29446208_ fusion_f | 154 | ATGTGACTGGCACGGGAGTTGATCCTGGTTTTCACCGAAT GAGGGTGATGTTTTTCCGCGGCACCTCCTTCAGGT |
| ALK_chr2:29446288_ fusion_f | 155 | ATGTGACTGGCACGGGAGTTGATCCTGGTTTTCACGTTGT AGTCGGTCATGATGGTCGAGGTGCGGAGCTTGCTC |
| ALK_chr2:29446368_ fusion_f | 156 | ATGTGACTGGCACGGGAGTTGATCCTGGTTTTCACGCAGC TCCTGGTGCTTCCGGCGGTACACTGCAGGTGGGTG |
| ALK_chr2:29446448_ fusion_f | 157 | ATGTGACTGGCACGGGAGTTGATCCTGGTTTTCACCTACA CAGGCCACTTCCTACAGGAAGCCTCCCTGGATCTC |
| ALK_chr2:29446528_ fusion_f | 158 | ATGTGACTGGCACGGGAGTTGATCCTGGTTTTCACGAAAT ACTAATAAAATGATTAAAGAAGGTGTGTCTTTAAT |
| ALK_chr2:29446608_ fusion_f | 159 | ATGTGACTGGCACGGGAGTTGATCCTGGTTTTCACTATAT GGAAAATAATTATTTGTATTATATAGGGCAGAGTC |
| ALK_chr2:29446688_ fusion_f | 160 | ATGTGACTGGCACGGGAGTTGATCCTGGTTTTCACATTAG ACCCAATATGGTCTGCAGATTTTATTAGAAGAAAT |
| ALK_chr2:29446768_ fusion_f | 161 | ATGTGACTGGCACGGGAGTTGATCCTGGTTTTCACGTGAA CCAGCAGACTGTGTTGCAAGTATAACCCCACGTGA |
| ALK_chr2:29446848_ fusion_f | 162 | ATGTGACTGGCACGGGAGTTGATCCTGGTTTTCACGCCAT GGAGCCTAAGGAAGTTTCAGCAAGGCCCTAAGGG |
| ALK_chr2:29446928_ fusion_f | 163 | ATGTGACTGGCACGGGAGTTGATCCTGGTTTTCACCCCAG GAATTGGCCTGCCTTAGTATTTCTGCTGTGCTCAG |
| ALK_chr2:29447008_ fusion_f | 164 | ATGTGACTGGCACGGGAGTTGATCCTGGTTTTCACTTTGA GGGTGCAGCTGGGATCTTGGTCAGTTGTGTTTCCT |
| ALK_chr2:29447088_ fusion_f | 165 | ATGTGACTGGCACGGGAGTTGATCCTGGTTTTCACCACAT CATGAAAAGATCTCTGAATTGGTGTCTGGGGATCT |
| ALK_chr2:29447168_ fusion_f | 166 | ATGTGACTGGCACGGGAGTTGATCCTGGTTTTCACTGAGG ACCAGGTCACAGGACCTCTTTGGACTGCAGTTTCC |
| ALK_chr2:29447248_ fusion_f | 167 | ATGTGACTGGCACGGGAGTTGATCCTGGTTTTCACTAACC ACTGCCACTCCCCACCCTCTAGGGTTGTCAATGAA |
| ALK_chr2:29447328_ fusion_f | 168 | ATGTGACTGGCACGGGAGTTGATCCTGGTTTTCACGAGCT CTACCAATGTGAGTGACCATTATCACTCCTACATG |
| ALK_chr2:29447408_ fusion_f | 169 | ATGTGACTGGCACGGGAGTTGATCCTGGTTTTCACAAAAT TGTGATTCAGTGGGTAGATTCTGTGTGTAAAGCCC |
| ALK_chr2:29447488_ fusion_f | 170 | ATGTGACTGGCACGGGAGTTGATCCTGGTTTTCACTATGT GCTCAGTTCCCTCCTCTATGCAATGGACCGACCGT |
| ALK_chr2:29447568_fusion_f | 171 | ATGTGACTGGCACGGGAGTTGATCCTGGTTTTCACGTGTA |
| | | AATTGCCGAGCACGTAGTAACCATGCAACAAGTGT |
| ALK_chr2:29447648_ fusion_f | 172 | ATGTGACTGGCACGGGAGTTGATCCTGGTTTTCACTGGGG ACACAGTGTGTGCTGCCATCTCCCTTCTACCGGCA |
| ALK_chr2:29447728_ fusion_f | 173 | ATGTGACTGGCACGGGAGTTGATCCTGGTTTTCACAAGAG CCTTTCCCTCTGCCCTTTTCAAGCCTCTGCCCATC |
| ALK_chr2:29447808_ fusion_f | 174 | ATGTGACTGGCACGGGAGTTGATCCTGGTTTTCACGACCA CACTGAGTTCTCTGTGACCTGCAGGTCAGCTCACC |
| ALK_chr2:29447888_ fusion_f | 175 | ATGTGACTGGCACGGGAGTTGATCCTGGTTTTCACTTTCCT ATCTCTCTGCCTGGAGGGTGGTGGAGGGCTGGTT |
| ALK_chr2:29447968_ fusion_f | 176 | ATGTGACTGGCACGGGAGTTGATCCTGGTTTTCACAAACA GGAGCTGCGCCGGTGGAAGCATGTGGGAGCTAGAA |
| ALK_chr2:29448048_ fusion_f | 177 | ATGTGACTGGCACGGGAGTTGATCCTGGTTTTCACGGACA CTGAAGGAGCTCCCCACCCCCTGATCAGCCAGGAG |
| ALK_chr2:29448128_ fusion_f | 178 | ATGTGACTGGCACGGGAGTTGATCCTGGTTTTCACGGGAA CTGCAGCTGCTCTGGTGGGGGGAAGGTTGGGAGCT |
| ALK_chr2:29448208_ fusion_f | 179 | ATGTGACTGGCACGGGAGTTGATCCTGGTTTTCACACCCA ATTCCAGGGACTAGCATAACGAAGTGACACCTTGG |
| ALK_chr2:29448288_ fusion_f | 180 | ATGTGACTGGCACGGGAGTTGATCCTGGTTTTCACCCTGC CCCCTTGGGAGTCCCTGGGGCTCTGTGCACTCACC |
| MYCNr1f_40 | 181 | ATGTGACTGGCACGGGAGTTGATCCTGGTTTTCACGGAAG CACCCCCGGTATTAAAACGAACGGGGCGGAAAGAA |
| MYCNr1r_40 | 182 | ATGTGACTGGCACGGGAGTTGATCCTGGTTTTCACCTAAC AAAGGGGACGCGACCCGGGGTCCAGTGCCCCAGGG |
| MYCNr1f2_40 | 183 | ATGTGACTGGCACGGGAGTTGATCCTGGTTTTCACCCTGG GGGGACTGGGTGGCCTCACCCCCAACCCGGTCATC |
| MYCNr1r2_40 | 184 | ATGTGACTGGCACGGGAGTTGATCCTGGTTTTCACCGCGC TCCAGCTTCTCGCGGGCGGAGAAGCCGCTCCACAT |
| MYCNr1f3_40 | 185 | ATGTGACTGGCACGGGAGTTGATCCTGGTTTTCACCCCAC CCGGCCGCCGAGTGCGTGGATCCCGCCGTGGTCTT |
| MYCNr1r3_40 | 186 | ATGTGACTGGCACGGGAGTTGATCCTGGTTTTCACGGGCA CGGGCGCTGGCTCGCGCTTGTTCACGGGAAAGGGG |
| MYCNr2f_40 | 187 | ATGTGACTGGCACGGGAGTTGATCCTGGTTTTCACAACAT GGATATATATGTGAATTTCATTCAAATGGTTCTCA |
| MYCNr2r_40 | 188 | ATGTGACTGGCACGGGAGTTGATCCTGGTTTTCACTAAAC CAACATTCTTAATGTCAACACAATGTTTGTTTAAA |
| MYCNr2f2_40 | 189 | ATGTGACTGGCACGGGAGTTGATCCTGGTTTTCACCCCTA CGTGGAGAGTGAGGATGCACCCCCACAGAAGAAGA |
| MYCNr2r2_40 | 190 | ATGTGACTGGCACGGGAGTTGATCCTGGTTTTCACATGAC ACTCTTGAGCGGACGTGGGGACGCCTCGCTCTTTA |
| MYCNr2f3_40 | 191 | ATGTGACTGGCACGGGAGTTGATCCTGGTTTTCACTCTCA CGCTCAGGGACCACGTGCCGGAGTTGGTAAAGAAT |
| MYCNr2r3_40 | 192 | ATGTGACTGGCACGGGAGTTGATCCTGGTTTTCACCAGTG GCCTTTTTCAAAATGACCACCTTGGCGGCCTTCTC |
| TP53_chr17:7579779: region_1:75nt:-59:-20:f | 193 | ATGTGACTGGCACGGGAGTTGATCCTGGTTTTCACGCTAG GGGGCTGGGGTTGGGGTGGGGGTGGTGGGCCTGCC |
| TP53_chr17:7579838: region_1:75nt:1:40:f | 194 | ATGTGACTGGCACGGGAGTTGATCCTGGTTTTCACCAGTT TCCATAGGTCTGAAAATGTTTCCTGACTCAGAGGG |
| TP53_chr17:7579878: region_1:75nt:41:+5:r | 195 | ATGTGACTGGCACGGGAGTTGATCCTGGTTTTCACCTGCC ATGGAGGAGCCGCAGTCAGATCCTAGCGTCGAGCC |
| TP53_chr17:7579932: region_1:75nt:+20:+59 :r | 196 | ATGTGACTGGCACGGGAGTTGATCCTGGTTTTCACTCATG CTGGATCCCCACTTTTCCTCTTGCAGCAGCCAGAC |
| TP53_chr17:7579640: region_2:23nt:-59:-20:f | 197 | ATGTGACTGGCACGGGAGTTGATCCTGGTTTTCACAGCCC CCCAGCCCTCCAGGTCCCCAGCCCTCCAGGTCCCC |
| TP53_chr17:7579741: | 198 | ATGTGACTGGCACGGGAGTTGATCCTGGTTTTCACGCAGA |
| region_2:23nt:+20:+59 :r | | GACCTGTGGGAAGCGAAAATTCCATGGGACTGACT |
| TP53_chr17:7579252: region_3:280nt:-59:-20:f | 199 | ATGTGACTGGCACGGGAGTTGATCCTGGTTTTCACGCAGG GGGATACGGCCAGGCATTGAAGTCTCATGGAAGCC |
| TP53_chr17:7579311: region_3:280nt:1:40:f | 200 | ATGTGACTGGCACGGGAGTTGATCCTGGTTTTCACCCGTG CAAGTCACAGACTTGGCTGTCCCAGAATGCAAGAA |
| TP53_chr17:7579351: region_3:280nt:41:80:r | 201 | ATGTGACTGGCACGGGAGTTGATCCTGGTTTTCACCCAGA AAACCTACCAGGGCAGCTACGGTTTCCGTCTGGGC |
| TP53_chr17:7579391: region_3:280nt:81:120 :f | 202 | ATGTGACTGGCACGGGAGTTGATCCTGGTTTTCACGAAGG GACAGAAGATGACAGGGGCCAGGAGGGGGCTGGTG |
| TP53_chr17:7579431: region_3:280nt: 121:16 0:r | 203 | ATGTGACTGGCACGGGAGTTGATCCTGGTTTTCACGTGGC CCCTGCACCAGCAGCTCCTACACCGGCGGCCCCTG |
| TP53_chr17:7579471: region_3:280nt: 161:20 0:f | 204 | ATGTGACTGGCACGGGAGTTGATCCTGGTTTTCACGGGGG GAGCAGCCTCTGGCATTCTGGGAGCTTCATCTGGA |
| TP53_chr17:7579511: region_3:280nt:201:24 0:r | 205 | ATGTGACTGGCACGGGAGTTGATCCTGGTTTTCACCCCCG GACGATATTGAACAATGGTTCACTGAAGACCCAGG |
| TP53_chr17:7579610: region_3:280nt:+20:+5 9:r | 206 | ATGTGACTGGCACGGGAGTTGATCCTGGTTTTCACCTGGG GGGCTGGGGGGCTGAGGACCTGGTCCTCTGACTGC |
| TP53_chr17:7578327: region_4:185nt:-43:-4:f | 207 | ATGTGACTGGCACGGGAGTTGATCCTGGTTTTCACCCTGG GCAACCAGCCCTGTCGTCTCTCCAGCCCCAGCTGC |
| TP53_chr17:7578370: region_4:185nt:1:40:f | 208 | ATGTGACTGGCACGGGAGTTGATCCTGGTTTTCACCCATC GCTATCTGAGCAGCGCTCATGGTGGGGGCAGCGCC |
| TP53_chr17:7578410: region_4:185nt:41:80:r | 209 | ATGTGACTGGCACGGGAGTTGATCCTGGTTTTCACGCCAT CTACAAGCAGTCACAGCACATGACGGAGGTTGTGA |
| TP53_chr17:7578450: region_4:185nt: 81:120 :f | 210 | ATGTGACTGGCACGGGAGTTGATCCTGGTTTTCACCATGG CGCGGACGCGGGTGCCGGGCGGGGGTGTGGAATCA |
| TP53_chr17:7578490: region_4:185nt:121:16 0:r | 211 | ATGTGACTGGCACGGGAGTTGATCCTGGTTTTCACTTTGC CAACTGGCCAAGACCTGCCCTGTGCAGCTGTGGGT |
| TP53_chr17:7578574: region_4:185nt:+20:+5 9:r | 212 | ATGTGACTGGCACGGGAGTTGATCCTGGTTTTCACTGCTTT ATCTGTTCACTTGTGCCCTGACTTTCAACTCTGT |
| TP53_chr17:7578117: region_5:114nt:-59:-20:f | 213 | ATGTGACTGGCACGGGAGTTGATCCTGGTTTTCACGAGGG CCACTGACAACCACCCTTAACCCCTCCTCCCAGAG |
| TP53_chr17:7578176: region_5:114nt:1:40:f | 214 | ATGTGACTGGCACGGGAGTTGATCCTGGTTTTCACCCTCA GGCGGCTCATAGGGCACCACCACACTATGTCGAAA |
| TP53_chr17:7578216: region_5:114nt:41:80:r | 215 | ATGTGACTGGCACGGGAGTTGATCCTGGTTTTCACAGGAA ATTTGCGTGTGGAGTATTTGGATGACAGAAACACT |
| TP53_chr17:7578292: region_5:114nt:+3 :+42 :r | 216 | ATGTGACTGGCACGGGAGTTGATCCTGGTTTTCACCCAGG GTCCCCAGGCCTCTGATTCCTCACTGATTGCTCTT |
| TP53_chr17:7577439: region_6:111nt:-59: - 20:1 | 217 | ATGTGACTGGCACGGGAGTTGATCCTGGTTTTCACGAGGC AAGCAGAGGCTGGGGCACAGCAGGCCAGTGTGCAG |
| TP53_chr17:7577498: region_6:111nt:1:40:f | 218 | ATGTGACTGGCACGGGAGTTGATCCTGGTTTTCACCCTGG AGTCTTCCAGTGTGATGATGGTGAGGATGGGCCTC |
| TP53_chr17:7577538: region_6:111nt:41:80:r | 219 | ATGTGACTGGCACGGGAGTTGATCCTGGTTTTCACACTAC ATGTGTAACAGTTCCTGCATGGGCGGCATGAACCG |
| TP53_chr17:7577628: region_6:111nt:+20:+5 9:r | 220 | ATGTGACTGGCACGGGAGTTGATCCTGGTTTTCACCTTGC CACAGGTCTCCCCAAGGCGCACTGGCCTCATCTTG |
| TP53_chr17:7576974: region_7:138nt-44:-5:f | 221 | ATGTGACTGGCACGGGAGTTGATCCTGGTTTTCACCTGCA CCCTTGGTCTCCTCCACCGCTTCTTGTCCTGCTTG |
| TP53_chr17:7577018: region_:138nt:1:40:f | 222 | ATGTGACTGGCACGGGAGTTGATCCTGGTTTTCACCCTCG CTTAGTGCTCCCTGGGGGCAGCTCGTGGTGAGGCT |
| TP53_chr17:7577058: region_7:138nt:41:80:r | 223 | ATGTGACTGGCACGGGAGTTGATCCTGGTTTTCACGACCG GCGCACAGAGGAAGAGAATCTCCGCAAGAAAGGGG |
| TP53_chr17:7577098: region_7:138nt:81:120 :f | 224 | ATGTGACTGGCACGGGAGTTGATCCTGGTTTTCACTCTCC CAGGACAGGCACAAACACGCACCTCAAAGCTGTTC |
| TP53_chr17:7577138: region_7:13 8nt: 121:+2 2:r | 225 | ATGTGACTGGCACGGGAGTTGATCCTGGTTTTCACTCTCTT TTCCTATCCTGAGTAGTGGTAATCTACTGGGACG |
| TP53_chr17:7577175: region_7:138nt:+20:+5 9:r | 226 | ATGTGACTGGCACGGGAGTTGATCCTGGTTTTCACGGACA GGTAGGACCTGATTTCCTTACTGCCTCTTGCTTCT |
| TP53_chr17:7576793: region_8:75nt:-59:-20:f | 227 | ATGTGACTGGCACGGGAGTTGATCCTGGTTTTCACGGCAT TTTGAGTGTTAGACTGGAAACTTTCCACTTGATAA |
| TP53_chr17:7576852: region_8:75nt:1:40:f | 228 | ATGTGACTGGCACGGGAGTTGATCCTGGTTTTCACCCTGA AGGGTGAAATATTCTCCATCCAGTGGTTTCTTCTT |
| TP53_chr17:7576892: region_8:75nt:41:+5:r | 229 | ATGTGACTGGCACGGGAGTTGATCCTGGTTTTCACCCTAG CACTGCCCAACAACACCAGCTCCTCTCCCCAGCCA |
| TP53_chr17:7576931: region_8:75nt:+5:+44: r | 230 | ATGTGACTGGCACGGGAGTTGATCCTGGTTTTCACTGCCT CAGATTCACTTTTATCACCTTTCCTTGCCTCTTTC |
| TP53_chr17:7573867: region_9:108nt:-59:-20:f | 231 | ATGTGACTGGCACGGGAGTTGATCCTGGTTTTCACATGGC TTTCCAACCTAGGAAGGCAGGGGAGTAGGGCCAGG |
| TP53_chr17:7573926: region_9:108nt:1:40:f | 232 | ATGTGACTGGCACGGGAGTTGATCCTGGTTTTCACCCTGG AGTGAGCCCTGCTCCCCCCTGGCTCCTTCCCAGCC |
| TP53_chr17:7573966: region_9:108nt:41:80:r | 233 | ATGTGACTGGCACGGGAGTTGATCCTGGTTTTCACTCCGA GAGCTGAATGAGGCCTTGGAACTCAAGGATGCCCA |
| TP53_chr17:7574053: region_9:108nt:+20:+5 9:r | 234 | ATGTGACTGGCACGGGAGTTGATCCTGGTTTTCACCATCT TTTAACTCAGGTACTGTGTATATACTTACTTCTCC |
| TP53_chr17:7572867: region_10:83nt:-59:-20:f | 235 | ATGTGACTGGCACGGGAGTTGATCCTGGTTTTCACGGCAG GGGAGGGAGAGATGGGGGTGGGAGGCTGTCAGTGG |
| TP53_chr17:7572926: region_10:83nt:1:40:f | 236 | ATGTGACTGGCACGGGAGTTGATCCTGGTTTTCACGTCAG TCTGAGTCAGGCCCTTCTGTCTTGAACATGAGTTT |
| TP53_chr17:7572966: region_10:83nt:41:80:r | 237 | ATGTGACTGGCACGGGAGTTGATCCTGGTTTTCACCCTGA AGTCCAAAAAGGGTCAGTCTACCTCCCGCCATAAA |
| TP53_chr17:7573028: region_10:83nt:+20:+5 9:r | 238 | ATGTGACTGGCACGGGAGTTGATCCTGGTTTTCACGGCAC AGACCCTCTCACTCATGTGATGTCATCTCTCCTCC |
| ALDH4A1_chr1:1919 9369_rs61757683:G:T :f | 239 | ATGTGACTGGCACGGGAGTTGATCCTGGTTTTCACCAGGG GCTTATGTGTCTCCTTGATGACCTGCGGCGACGTC |
| ALDH4A1_chr1:1919 9488_rs61757683:G:T :r | 240 | ATGTGACTGGCACGGGAGTTGATCCTGGTTTTCACCCATC ATCTCCTCCCTTCCCCTTCTGCCCAGGCTGTTGCA |
| BRCA1_ chr17:412230 15_rs1799966:T:A,C:f | 241 | ATGTGACTGGCACGGGAGTTGATCCTGGTTTTCACAATTC TGGCTTCTCCCTGCTCACACTTTCTTCCATTGCAT |
| BRCA1_chr17:412231 34_rs1799966:T:A,C:r | 242 | ATGTGACTGGCACGGGAGTTGATCCTGGTTTTCACGTCAG CTCGTGTTGGCAACATACCATCTTCAACCTCTGCA |
| BRCA1_chr17:412439 21_rs16942:T:C:f | 243 | ATGTGACTGGCACGGGAGTTGATCCTGGTTTTCACTAATT TCTTGGCCCCTCTTCGGTAACCCTGAGCCAAATGT |
| BRCA1_chr17:412440 40_rs16942:T:C:r | 244 | ATGTGACTGGCACGGGAGTTGATCCTGGTTTTCACGGTGA AATAAAGGAAGATACTAGTTTTGCTGAAAATGACA |
| BRCA2_chr13:329066 70_rs144848:A:C:f | 245 | ATGTGACTGGCACGGGAGTTGATCCTGGTTTTCACACTCA TTTGTATCTGAAGTGGAACCAAATGATACTGATCC |
| BRCA2_chr13:329067 69_rs144848:A:C:r | 246 | ATGTGACTGGCACGGGAGTTGATCCTGGTTTTCACAGTTG AGACCATTCACAGGCCAAAGACGGTACAACTTCCT |
| CDKN2A_chr9:21970 837_rs3731249:C:T:f | 247 | ATGTGACTGGCACGGGAGTTGATCCTGGTTTTCACGAAAA TGAATGCTCTGAGCTTTGGAAGCTCTCAGGGTACA |
| CDKN2A_chr9:21970 956_rs3731249:C:T:r | 248 | ATGTGACTGGCACGGGAGTTGATCCTGGTTTTCACGGGCC ATCGCGATGTCGCACGGTACCTGCGCGCGGCTGCG |
| DPYD_chr1:97981316 _rs1801159:T:C:f | 249 | ATGTGACTGGCACGGGAGTTGATCCTGGTTTTCACCCCCA TCCAGCTTCAAAAGCTCTTCGAATCATTGATGTGC |
| DPYD_chr1:97981435 _rs1801159:T:C:r | 250 | ATGTGACTGGCACGGGAGTTGATCCTGGTTTTCACTGCCA AGCCTGAACTACCCCTCTTTTACACTCCTATTGAT |
| EPHX1_chr1:2260263 27_rs2234922:A:G:f | 251 | ATGTGACTGGCACGGGAGTTGATCCTGGTTTTCACCCACC CTGACTGTGCTCTGTCCCCCCAGGGCTGGACATCC |
| EPHX1_chr1:2260264 46_rs2234922:A:G:r | 252 | ATGTGACTGGCACGGGAGTTGATCCTGGTTTTCACAGTCA GGAGTGGGATGATCTTATAAAACTCGTAGAAAGAG |
| MYC_chr8:12875075 2_G123E:f | 253 | ATGTGACTGGCACGGGAGTTGATCCTGGTTTTCACCTTCG GGGAGACAACGACGGCGGTGGCGGGAGCTTCTCCA |
| MYC_chr8:12875087 1_G123E:r | 254 | ATGTGACTGGCACGGGAGTTGATCCTGGTTTTCACCATAC AGTCCTGGATGATGATGTTTTTGATGAAGGTCTCG |
| RB1 _chr13:49039115 _rs121913297:G:T:f | 255 | ATGTGACTGGCACGGGAGTTGATCCTGGTTTTCACTTTTAC TGTTCTTCCTCAGACATTCAAACGTGTTTTGATC |
| RB1_chr13:49039204 _rs121913297:G:T:r | 256 | ATGTGACTGGCACGGGAGTTGATCCTGGTTTTCACGTGGA AGCATACTGCAAAATATTTGTTTTCAGTCTCTGCA |
| TNFRSF14_chr1:2491 227 _rs2234163:G:A:f | 257 | ATGTGACTGGCACGGGAGTTGATCCTGGTTTTCACACGTA CCCCTCTCAGCCCCTCCTCTTGGACTCCAGCCATG |
| TNFRSF14_chr1:2491 346 _rs2234163:G:A:r | 258 | ATGTGACTGGCACGGGAGTTGATCCTGGTTTTCACGTGGC GTAAGCGCGGCACGCGGCGCAGTGGTCCCCGTCCT |

### Genomic samples

Three samples of genomic DNA were analyzed::
1) Germline sample NA 06994 - a normal human sample obtained from the Coriell repository;
2) Cancer cell line NCI-H69 - a cell line known to harbor a mutation in TP53, an amplification of the MYCN locus, and SNVs in ALDH4A1, BRCA1, BRCA2, CDKN2A, DPYD, EPHX1, MYC, RB1 and TNFRSF14 that were included in the target probe set;
3) Cancer cell line ZR-75-1, which was reported to harbor an EML4-ALK fusion gene (Lin et al., Mol. Cancer Res. 7(9): 1466, 2009).

DNA sequencing libraries are generally constructed from sheared DNA fragments. Acoustic disruption was used to generate DNA fragments that ranged in size from 200 to >500 bp. Enzymatic fragmentation of the acoustically fragmented DNA was performed in an effort to emulate circulating DNA, which is reputed to be composed of nucleosomal, -150 bp fragments. Briefly, DNA at 20-40 ng/µl was sonicated on the 200 bp setting, which yields fragments that range in size from 150 bp to 400 bp in a broad smear. The DNA was further fragmented by the addition of 0.01 and 0.02 µl of DNAse enzyme (New England Biolabs recombinant bovine DNAse) to 50 µl aliquots of DNA in DNAse buffer (10 mM Tris pH 8.0, 2.5 mM MnCl₂, 0.5 mM CaCl₂). The DNAse reaction was incubated at 37° C for 10 min and stopped with the addition of 0.5 M EDTA to a final concentration of 25 mM. DNA with an average size of 150 bp was purified by "two-sided" bead selection by first adding 0.9 volumes of beads to 1 volume of DNA. The beads bind unwanted larger fragments and are discarded, and an additional 1.6 volumes of beads are added to the supernatant. The bound material is then purified and quantified. An agarose gel of the resulting, highly fragmented, short DNA used for library construction is shown in Figure 6.

Fragmented DNA was end-repaired using the Quick Blunt kit form NEB and blended in the ratios shown in Table 7. Ten nanograms of blended DNA were then ligated to adaptors with the sequences shown in Table 7. For mixes 9 and 15, two ligation reactions with 10 ng each were performed and subsequently pooled. For mix 16, four reactions were done. An estimate of genomic inputs into each library using a qPCR assay is also shown in Table 7.

**Table 7. Samples and admixture ratios**

| **Sample** | **Admix ratio** | **Code** | **SEQ ID NO:** | **Genomic inputs** |
|---|---|---|---|---|
| 1)NA06994 = GL | pure | NNNNNAAGATCTTAGTGGCAC | 259 | 202 |
| 2)NCI-H69 = N | pure | NNNNNCGACAGAACTATTGCC | 260 | 149 |
| 3)ZR-75-1 = Z | pure | NNNNNACTATCTTAGTGGCAC | 261 | 242 |
| 4)GL:N | 1:1 | NNNNNCTCCAGAACTATTGCC | 262 | 200 |
| 5)GL:N | 2:1 | NNNNNAGCATCTTAGTGGCAC | 263 | 83 |
| 6)GL:N | 4:1 | NNNNNCATCAGAACTATTGCC | 264 | 186 |
| 7)GL:N | 10:1 | NNNNNATAATCTTAGTGGCAC | 265 | 264 |
| 8)GL:N | 20:1 | NNNNNAAGAAGGTAGACCCTC | 266 | 203 |
| 9)GL:N | 100:1 | NNNNNTTTCTCTACTCGTGAC | 267 | 436 |
| 10)GL:Z | 1:1 | NNNNNACTAAGGTAGACCCTC | 268 | 297 |
| 11)GL:Z | 2:1 | NNNNNGAAGCTACGAGTATCC | 269 | 224 |
| 12)GL:Z | 4:1 | NNNNNAGCAAGGTAGACCCTC | 270 | 73 |
| 13)GL:Z | 10:1 | NNNNNCATTGACGTCTAGAGC | 271 | 181 |
| 14)GL:Z | 20:1 | NNNNNTCACTCTACTCGTGAC | 272 | 224 |
| 15)GL:Z | 100:1 | NNNNNATAAAGGTAGACCCTC | 273 | 580 |
| 16)GL:N:Z | 500:1:1 | NNNNNTACCTCTACTCGTGAC | 274 | 1324 |

### Targeted sequencing

One microgram of each of the sixteen DNA libraries shown in Table 7 were pooled and adjusted to a final volume of 160 µL. Eight identical 20 µL aliquots were denatured at 98° C, cooled on ice, and 20 µL of probes (Table 6) at 1 nM/probe and 50 µL of CF hyb buffer were added. The samples were annealed for 24 hours from 80° C to 50° C, washed, and amplified. Following amplification of the resulting captured and processed fragments, the final sequencing library was size selected using the Pippin Prep^{™} instrument with a size selection of 175 - 400 bp. The library was sequenced on an Illumina MiSeq using a 150 read V3 kit.

### RESULTS

Capture probe performance of high density capture probes that were chosen based on their position with target sequences were monitored. A graphical display of the performance of each capture probe is shown in Figure 7. These data demonstrate that:
1) all capture probes chosen strictly by positional constraint provided on-target sequence information;
2) most capture probes exhibit very little off target and unmappable read capture; and
3) the yield of useful, on-target reads was substantially uniform.
Capture probes that captured a high proportion of off-target and unmappable reads were analyzed further. These capture probes were generally positioned in regions of low sequence complexity/high sequence redundancy. Here, however, such capture probes had no significant deterimental impact on the sequencing depth because the high level of probe redundancy (high density probes) means that all regions are covered by reads derived from several probes. The net effect was excellent uniformity of coverage. See, *e.g*., Figure 8, probe coverage for the TP53 gene using the 40 mer capture probes.

### CONCLUSION

Taken together, these data demonstrate that capture probe length can be reduced from 60 nucleotides to 40 nucleotides with little or no discernible loss of probe performance (once capture wash temperatures are adjusted). They also show that probe design can follow positional constraints and can generally ignore sequence context or composition. Even though this methodology produces the occasional poor-performing probe, the high redundancy at close probe spacing more than compensates for individual probe deficiencies.

### EXAMPLE 3

### GENETIC ANALYSIS OF CIRCULATING DNA

### PURPOSE

The purpose of this example was to benchmark the genetic analysis of cfDNA using an efficient cloning procedure for cfDNA and target retrieval system.

### BACKGROUND

While there is tremendous enthusiasm in the scientific and health-care community for "liquid biopsies" - analysis of circulating DNA (cfDNA) for markers associated with potential disease states, there is remarkably little practical information about this potential analyte.

### SUMMARY

Plasma samples collected from healthy donors and individuals suffering from either ovarian or colon cancers were used to perform the genetic analysis of circulating DNA. The amount and the overall character of circulating cfDNA can vary widely from individual to individual. Surprisingly, the present inventors found that cfDNA is readily clonable with an efficiency indistinguishable from highly purified and fragmented genomic DNA; that the fragment size was remarkably consistent, with an average clone insert size of 170 ± 10 bp (in 7/8 samples); and that the genome representation from such samples was uniform and comparable to experiments performed using purified gDNA. It was further established that by counting unique reads, the depth of representation in each library provided an estimate of minor allele frequency for tumor markers present in the cfDNA of diseased patients. This study established that construction and target retrieval systems contemplated herein were effectively applied to the quantitative genetic analysis of cfDNA.

### METHODS

### DNA purification

Eight sets of plasma samples were purchased from Proteogenex, Inc., Culver City, CA (Table 8). Circulating DNA was extracted from the samples (on two separate occasions) using the Circulating Nucleic Acid Purification kit from Qiagen. Samples were passed through DNA mini-columns using centrifugation. The specimen IDs and yield of DNA are shown in Table 8.

**Table 8. Plasma samples and cfDNA yields**

| **Sample ID** | **Patient diagnosis** | **Specimen type** | **Volume** | **DNA yield (ng per mL of input)** |
|---|---|---|---|---|
| D5930P | Healthy donor | plasma | 4 mL | 11 |
| D5942P | Healthy donor | plasma | 4 mL | 68 |
| 023407P | Colorectal cancer | plasma | 4 mL | 10 |
| 023406P | Colorectal cancer | plasma | 4 mL | 63 |
| 023185P | Colorectal cancer | plasma | 4 mL | 171 |
| 023149P | Colorectal cancer | plasma | 4 mL | 36 |
| 032667P | Ovarian cancer | plasma | 4 mL | 24 |
| 032676P | Ovarian cancer | plasma | 4 mL | 13 |

### Library construction

Purified DNA from 4 mL of plasma was collected in 100 µl elution buffer. For the four samples collected from colon cancer patients (CRC), the DNA was split in half and one 50 µl aliquot from each patient was sonicated to 200 bp. One 50 µl aliquot of untreated cfDNA and one 50 µl fragmented cfDNA from each patient (the entire sample from each patient) was end repaired by adding (per sample):
- 6 µl of 10X quick blunt buffer (New England Biolabs (NEB))
- 0.6 µl of 10 mM dNTPs
- 2.4 µl of quick blunt enzyme mix
- 1.2 µl of PreCR enzyme mix.

Samples were incubated at 20° C for 30 min and at 70° C for 10 min. Ligations with adaptors (Table 2) were performed by combining:
- 60 µl end-repaired cfDNA
- 12 µl adaptor duplex (10 µM)
- 10 µl 10X ligase buffer (NEB)
- 15 µl 50% PEG₈₀₀₀
- 3 µl HC T4 DNA ligase

**Table 9. Samples and codes used for four CRC plasma samples**

| **Progenex ID** | **Sample #** | **Pretreatment** | **Adapter** |
|---|---|---|---|
| 23149 | 1 | none | NNNNNTTTTGTGTGTGTGTG (SEQ ID NO:275) |
| 23407 | 2 | none | NNNNNACTACACACACACAC (SEQ ID NO:276) |
| 23406 | 3 | none | NNNNNCTCGTGTGTGTGTGT (SEQ ID NO:277) |
| 23185 | 4 | none | NNNNNGAACACACACACACA (SEQ ID NO:278) |
| 23149 | 5 | frag | NNNNNCATGTGTGTGTGTGT (SEQ ID NO:279) |
| 23407 | 6 | frag | NNNNNGTGCACACACACACA (SEQ ID NO:280) |
| 23406 | 7 | frag | NNNNNATAACACACACACAC (SEQ ID NO:281) |
| 23185 | 8 | frag | NNNNNTACTGTGTGTGTGTG (SEQ ID NO:282) |

Reactions were incubated at 22° C for one hour and 65° C for 10 min. Ligation products were purified by the addition of 100 µl beads, washing, and elution in 40 µl TEzero. All 40 µl of ligation product was amplified by PCR with primer ACA2 (SEQ ID NO:283) and the samples were combined in equal mass for targeted capture.

### Targeted sequence capture and sequencing

The four unfragmented and four fragmented colon plasma samples (Figure 9C) were hybridized with our high-density, 40 nucleotide probe set that targets TP53, ALK, among others. The capture complexes were processed as described above in Example 2.

### RESULTS

### Library appearance

A false-color picture of a 2% agarose gel loaded with 50 ng of each library is shown in Figure 9A. The average fragment size was in a tight range of 260 ± 20 bp. These data indicated that the clonable fraction of cfDNA is present predominantly as nucleosomal fragments. In addition, the size of the cfDNA libraries had the same basic superficial appearance as cfDNA in kidney dialysis patients (Atamaniuk et al., Clinical Chemistry 52(3):pp. 523-26 (2006)) except that the cfDNA libraries were shifted to higher mass by the addition of adaptor sequences (Figure 9B). In contrast, the cfDNA libraries differed dramatically from sonicated gDNA libraries, which appear as broad smears.

Four additional sets of cfDNA libraries were constructed from the two ovarian cancer patient plasma samples and two plasma samples from healthy volunteers. 38 µl aliquots of cfDNA were end-repaired in 50 µl total volume.. Ligations included 40 µl of end-repaired fragment, 16 µl of adaptor (10 µM), 8 ul of 10X ligase buffer, 16 µl of 50% PEG and 4 µl of HC T4 DNA ligase in a total volume of 80 µl . The ligation reaction was incubated at 20° C for 1 hour and 65° C for 10 min. For purification, 20 µl of TEzero and 150 µl of beads were added. The purified ligation products were resuspended in 40 µl , all of which was used in a subsequent 200 µl library amplification by PCR. The resulting amplified libraries are shown in Figure 9C.

### Sequencing data analysis

The average unique read count observed in each of the eight libraries ranges from ~700 unique reads to >3000 unique reads, defining a range of sensitivities from -0.15% to ~0.03%. Figure 10. A rare mutant read will likely be observed more than once, meaning minimum sensitivities are less than those calculated above. In preferred embodiments, unique reads provide the lower bound on statistically defensible observation frequencies.

### cfDNA cloning efficiency

Sample 23407 was used as a benchmark. 10 ng/mL of cfDNA was recovered from the plasma sample and 20 ng of the isolated cfDNA was used in each of two library construction efforts. The unique read counts indicated that we recovered an average of 700 unique reads (genome equivalents) from unfragmented DNA ("23407" in Figure 10). Given that each genome contains 0.003 ng of gDNA, 2.1 ng of input DNA in this library (10% cloning efficiency) was recovered.

Fragmentation prior to library construction with this sample increased the library yield by more than two-fold ("23407 frag" in Figure 10). This indicates that much of the DNA present in the 23407 sample was high molecular weight DNA that required fragmentation in order to be clonable. Thus, the library cloning efficiency was likely far greater than 10% and was likely in the range of 20% for input cfDNA. This cloning efficiency is comparable to highly purified genomic DNA and indicates that cfDNA was not likely modified in any way that is deleterious to downstream cloning efforts.

### Library coverage

The cfDNA libraries resembled a set of discrete bands with random coverage of target regions. Figure 11 shows a random sampling of sequence data. A random set of reads from sample 23407 that was not fragmented prior to cloning (see Figure 10), and that were captured by the TP53 probe "chr17:7579351:region_3:280nt:41:80:r" (SEQ ID NO:201) were aligned using BLAT. Given the way that the sample was prepared, these are likely a reflection of cfDNA fragments in general because the left hand portion of these reads (the read start sites) are randomly distributed across the target region. This random distribution indicates the random breakage of genomic DNA, and it demonstrates that despite the band-like appearance of cfDNA libraries, the sequencing output was a random coverage of the target region. The random distribution is important for effective genetic analysis using technology contemplated herein.

Figure 12 provides a more high resolution overview of TP53 coding region sequencing for a typical cfDNA library. The elements of targeted sequencing - coverage across all target regions and uniform depth at each sequenced base - are readily apparent. At this depth of >4000 unique reads per base, and with a requirement that legitimate candidate base changes must be encountered at least twice, it is possible to estimate that the mutation detection sensitivity for this particular library was about 1 mutation in 2000 sequences, or 0.05%. This level of sensitivity represents a surprising and unexpected outstanding technical achievement.

### CONCLUSION

cfDNA was isolated and cloned from plasma clones with an efficiency comparable to highly purified gDNA isolated from cell lines (the gold standard). The cfDNA libraries resembled circulating nucleosomal-sized DNA fragments + adaptors and the ends possessed sufficiently random character, which enabled efficient genetic analysis. In addition, the highly uniform size characteristic of plasma cfDNA libraries allows designing capture strategies and underlying probe sequences to maximize reliable coverage of targets as far as 120 bp (= 160 - 40) from the ends of probes.

### EXAMPLE 4

### MEASUREMENT OF GENOME EQUIVALENTS IN CIRCULATING DNA LIBRARIES

### PURPOSE AND BACKGROUND

One of the major challenges in the analysis of circulating, cell-free DNA is achieving sufficient assay sensitivity. If sufficient sensitivity is not achieved then analysis of the cfDNA libraries is confounded: if a sample is sequenced and no mutational events are detected, that result could be interpreted to mean that no mutations are present, or that significant events were missed because the sampling depth was too small. The sensitivity of an assay is defined in statistical terms as the false negative rate. In the context of sequencing circulating, cell-free DNA, a significant obstacle is the detection of a rare sequence that is blended in a large excess of reference sequence.

One method for determining assay sensitivity is to measure the occurrences of mutant sequence in a set of samples where mutant sequence is progressively diluted into non-mutant, reference sequence. The dilution at which mutant sequences are no longer detected defines assay sensitivity. This method is adequate if both the identity of the mutant sequence and the extent of dilution are known. Unfortunately, clinical samples do not generally provide either parameter. Often the identity of the mutant sequence is not known, and the extent of dilution varies from sample to sample. In this context, assay sensitivity is established on a sample-by-sample basis.

To assign a sensitivity value on a sample-by-sample basis, the numbers of different and distinct sequences that are present in each sample are measured, by measuring the number of genome equivalents that are present in a sequencing library. By way of a non-limiting example, if a DNA sequencing library is known to contain 3 ng (3000 pg) of human genomic DNA and each human genome has a mass of 3 pg, then the library possesses 3000 ÷ 3 = 1000 genome equivalents of DNA. If a mutant DNA sequence must be detected twice to be statistically significant, then an estimate of the best possible sensitivity of detection for this particular library is 2 mutant sequences ÷ 1000 total sequences = 0.002 = 0.2%. To establish sensitivity, the number of genome equivalents must be measured for each sample library.

### SUMMARY

Two methods were used to measure genome equivalents. The first method is based on quantitative PCR (qPCR). A genomic library was constructed using ligation of adaptors to genomic fragments and a pair of PCR primers, one that is specific to a common genomic sequence (*e.g*., Alu I repeat) and one that is specific to the adaptor. The abundance of ligated adaptor: fragment sequences of these cfDNA libraries was measured. A standard library of known concentration was used to construct a standard curve and the measurements were fit to the resulting standard curve and a value for genome equivalents was derived from the fit.

The second method to measure genome equivalents used bioinformatics counting after sequencing was performed. Each unique sequence in a library was identified by its random sequence label and the starting nucleotide of the genomic sequence. Moreover, each unique sequence must be derived from an independent genome. Therefore, the sum of unique sequences present in sequence data established a precise quantitative measurement of the number of genome equivalents present in a sample.

### METHODSAND RESULTS

### qPCR assay development

The first version of a qPCR-based genome equivalence assay used the ACA2 primer (Table 10), but this assay chronically under-reports the number of genome equivalents that are present in a cfDNA library (Figure 13).

**Table 10. PCR primers used in the development of the genome equivalent qPCR assay**

| **Name** | **SEQ ID NO:** | **Sequence** |
|---|---|---|
| ACA2 | 283 | TGCAGGACCAGAGAATTCGAATACA |
| ACA2_FL FP | 284 | AATGATACGGCGACCACCGAGATCTACACGTCATGCAGGACCAG AGAATTCGAATACA |
| Alu_F1 | 285 | CGGTGGCTCACGCCTGTA |
| Alu_R1 | 286 | GCCTCGGCCTCCCAAAGT |
| Alu_F2 | 287 | GAGGCTGAGGCAGGAGAATCG |
| Alu_R2 | 288 | GTCGCCCAGGCTGGAGTG |

The improved version of the assay was based on endogenous repeats (e.g., Alu repeats) that are found at very high frequency throughout the human genome. By coupling an Alu-specific primer with an adaptor-specific primer, the frequency with which adaptors are joined to genomic fragments was reliably measured. Standard curves using libraries of known genome equivalents were generated, and the number of genome equivalents in cloned libraries was measure by fitting to the curve.

The PCR primers used to develop an Alu + adaptor-based qPCR assay are shown in Table 10. The PCR primers for Alu amplification were designed from consensus a consensus human Alu sequence (Batzer & Deininger, *Nat Rev Genet.* 3(5):370-9 (2002)) using PRIMER3 (Alu_F1 & Alu_R1, SEQ ID NOs:285 and 286, respectively). The remaining two Alu primers (Alu_F2 and Alu_R2, SEQ ID NOs:287 and 288, respectively) were reported in the literature (Marullo *et al., Genome Biology* 11:R9 (2010)).

A schematic of the assay design is provided in Figure 14. Because a single PCR primer can used to amplify the genomic DNA libraries (Figure 14A), a primer that recognizes the adaptor sequence but that cannot amplify genomic clones was used. The 58 nucleotide ACA2-FLFP primer (henceforth abbreviated AF, SEQ ID NO:284) fills these criteria because its length induces strong stem-loop PCR suppression (Figure 14B). Additionally, a functional pair of Alu primers were used (Figure 14C). Moreover, a primer pair consisting of one Alu primer and the long ACA2 primer that did not amplify genomic DNA used (Figure 14D). These same primers also amplified genomic library clones (Figure 14E).

All of the required elements for a functional Alu-based assay were validated. Figure 15. Specifically, the long primer alone was inert, both sets of Alu primer pairs recognized human genomic DNA, and any combination of one Alu primer and the long ACA2 primer amplified genomic library clones (Figure 15A). Finally, the ability of Alu primer plus long ACA2 primer pair to discriminate between genomic DNA and genomic library clones is shown in Figure 15B. The combination of Alu_R1 and AF primers were used for measuring genome equivalents in the newly constructed libraries.

A direct comparison between the ACA2-based and the Alu-based qPCR assays is shown in Figure 16. An 8-fold difference in genome equivalents was found. In addition the Alu-based assays provided a more consistent performance library-to-library and a better alignment between qPCR derived equivalents and bioinformatically counted tag equivalents in sequencing runs (Table 11).

**Table 11. qPCR vs counted sequencing tags**

| **Sample** | **Alu-based qPCR** | **counted tags** |
|---|---|---|
| Run_68 50to1 | 6962 | 3459 |
| Run_68 1000to1 | 10937 | 4641 |

### High-sensitivity library adaptors for sequence-based counting of genome equivalents

As discussed above, the reality of disease surveillance using cfDNA is that mutant sequences may be rare constituents in an otherwise vast excess of "normal" (meaning germline) DNA sequences. Thus, highly sensitive and quantifiable sequencing assays are needed. Assay sensitivity could be made by counting the number of unique sequences present in a sequencing library. However, such counting would lead to a *false underestimate* of sensitivity because cfDNA fragments are rather short (~165 bp) and may lead to identical reads that were actually derived from independent cloning events. One solution to this problem is to mark each independent sequencing clone during library construction by including, for example, a set of DNA tags in the adaptors used to construct libraries.

A set of such library construction adaptors was specifically designed to measure the number of genome equivalents present in cfDNA libraries, and, by extension, the sensitivity of sequencing assays used to monitor mutant sequences.

The architecture of high-sensitivity library adaptors that were configured to accommodate large numbers of genome equivalents in cfDNA libraries is shown in Figure 17. There is a substantial amount of molecular engineering within the 45 nucleotide ligation strand, which is the strand that becomes attached to end repaired cfDNA fragments. The adaptors comprise at least five elements.

Element 1 is a PCR primer binding site for the single-primer library amplification primer ACA2 (Table 12).

**Table 12**

| **Element** | **Function** | **Number of sequences** | **Sequences (5' -> 3')** | **SEQ ID NO:** |
|---|---|---|---|---|
| Element 1 | PCR primer binding site | 1 | TGCAGGACCAGAGAATTCGAATACA | 289 |

Element 2 is a 5 nucleotide read code. The combination of this code with the genomic DNA sequence constitutes the DNA tag that was used to uniquely identify each read. The 5 nucleotide codes consist of 256 possible unique sequences that were chosen to be 2 base changes different from every other code in the set. This feature enabled unique and distinct reads to be differentiated from reads that appeared to be unique owing to a sequencing error in the code region. Seven codes in which G residues are over-represented and that were shown empirically to interfere with adaptor function were removed, leaving 249 random codes. Table 13.

**Table 13**

| **Element** | | **Function** | | **Number of sequences** | | | |
|---|---|---|---|---|---|---|---|
| Element 2 | | Distinct sequence labels | | 249 | | | |
| **Seqences** (**5' -> 3')** | **SEQ ID NO:** | **Sequences (5' -> 3')** | **SEQ ID NO:** | **Sequences (5' -> 3')** | **SEQ ID NO:** | **Sequences (5' -> 3')** | **SEQ ID NO:** |
| CGGGT | 290 | GGGTC | 354 | AGAGA | 418 | CCGGA | 482 |
| CGGTG | 291 | GGTCG | 355 | AGCCG | 419 | CGACG | 483 |
| CGTGG | 292 | GGTGC | 356 | AGCGC | 420 | CGAGC | 484 |
| GCGGT | 293 | GTCGG | 357 | AGGAA | 421 | CGCAG | 485 |
| GCGTG | 294 | GTGCG | 358 | AGGCC | 422 | CGCGA | 486 |
| GCTGG | 295 | GTGGC | 359 | AGGTT | 423 | CGGAC | 487 |
| GGCGT | 296 | TGCGG | 360 | AGTGT | 424 | CGGCA | 488 |
| GGCTG | 297 | TGGCG | 361 | AGTTG | 425 | GAAAG | 489 |
| GGGCT | 298 | TGGGC | 362 | ATGGT | 426 | GAAGA | 490 |
| TTAAA | 299 | AAAGG | 363 | ATGTG | 427 | GACCG | 491 |
| TTACC | 300 | AAGAG | 364 | ATTGG | 428 | GACGC | 492 |
| TTATT | 301 | AAGGA | 365 | CACGG | 429 | GAGAA | 493 |
| TTCAC | 302 | ACCGG | 366 | CAGCG | 430 | GAGCC | 494 |
| TTCCA | 303 | ACGCG | 367 | CAGGC | 431 | GAGTT | 495 |
| TTTAT | 304 | ACGGC | 368 | CCAGG | 432 | GATGT | 496 |
| TTTTA | 305 | AGAAG | 369 | CCGAG | 433 | GATTG | 497 |
| GCACG | 306 | GTGTA | 370 | AACTG | 434 | AGTCA | 498 |
| GCAGC | 307 | GTTAG | 371 | AAGCT | 435 | ATACG | 499 |
| GCCAG | 308 | GTTGA | 372 | AAGTC | 436 | ATAGC | 500 |
| GCCGA | 309 | TAGGT | 373 | AATCG | 437 | ATCAG | 501 |
| GCGAC | 310 | TAGTG | 374 | AATGC | 438 | ATCGA | 502 |
| GCGCA | 311 | TATGG | 375 | ACAGT | 439 | ATGAC | 503 |
| GGAAA | 312 | TGAGT | 376 | ACATG | 440 | ATGCA | 504 |
| GGACC | 313 | TGATG | 377 | ACGAT | 441 | CAAGT | 505 |
| GGATT | 314 | TGGAT | 378 | ACGTA | 442 | CAATG | 506 |
| GGCAC | 315 | TGGTA | 379 | ACTAG | 443 | CAGAT | 507 |
| GGCCA | 316 | TGTAG | 380 | ACTGA | 444 | CAGTA | 508 |
| GGTAT | 317 | TGTGA | 381 | AGACT | 445 | CATAG | 509 |
| GGTTA | 318 | TTAGG | 382 | AGATC | 446 | CATGA | 510 |
| GTAGT | 319 | TTGAG | 383 | AGCAT | 447 | CCCGT | 511 |
| GTATG | 320 | TTGGA | 384 | AGCTA | 448 | CCCTG | 512 |
| GTGAT | 321 | AACGT | 385 | AGTAC | 449 | CCGCT | 513 |
| CCGTC | 322 | CTGTT | 386 | GTAAC | 450 | TCCGC | 514 |
| CCTCG | 323 | CTTGT | 387 | GTACA | 451 | TCGAA | 515 |
| CCTGC | 324 | CTTTG | 388 | GTCAA | 452 | TCGCC | 516 |
| CGAAT | 325 | GAACT | 389 | GTCCC | 453 | TCGTT | 517 |
| CGATA | 326 | GAATC | 390 | GTCTT | 454 | TCTGT | 518 |
| CGCCT | 327 | GACAT | 391 | GTTCT | 455 | TCTTG | 519 |
| CGCTC | 328 | GACTA | 392 | GTTTC | 456 | TGAAC | 520 |
| CGTAA | 329 | GATAC | 393 | TAACG | 457 | TGACA | 521 |
| CGTCC | 330 | GATCA | 394 | TAAGC | 458 | TGCAA | 522 |
| CGTTT | 331 | GCAAT | 395 | TACAG | 459 | TGCCC | 523 |
| CTAAG | 332 | GCATA | 396 | TACGA | 460 | TGCTT | 524 |
| CTAGA | 333 | GCCCT | 397 | TAGAC | 461 | TGTCT | 525 |
| CTCCG | 334 | GCCTC | 398 | TAGCA | 462 | TGTTC | 526 |
| CTCGC | 335 | GCTAA | 399 | TCAAG | 463 | TTCGT | 527 |
| CTGAA | 336 | GCTCC | 400 | TCAGA | 464 | TTCTG | 528 |
| CTGCC | 337 | GCTTT | 401 | TCCCG | 465 | TTGCT | 529 |
| TTGTC | 338 | ACTTC | 402 | CCACC | 466 | TATAA | 530 |
| TTTCG | 339 | ATAAT | 403 | CCATT | 467 | TATCC | 531 |
| TTTGC | 340 | ATATA | 404 | CCCAC | 468 | TATTT | 532 |
| AAAAA | 341 | ATCCT | 405 | CCCCA | 469 | TCACT | 533 |
| AAACC | 342 | ATCTC | 406 | CCTAT | 470 | TCATC | 534 |
| AAATT | 343 | ATTAA | 407 | CCTTA | 471 | TCCAT | 535 |
| AACAC | 344 | ATTCC | 408 | CTACT | 472 | TCCTA | 536 |
| AACCA | 345 | ATTTT | 409 | CTATC | 473 | TCTAC | 537 |
| AATAT | 346 | CAAAC | 410 | CTCAT | 474 | TCTCA | 538 |
| AATTA | 347 | CAACA | 411 | CTCTA | 475 | | |
| ACAAC | 348 | CACAA | 412 | CTTAC | 476 | | |
| ACACA | 349 | CACCC | 413 | CTTCA | 477 | | |
| ACCAA | 350 | CACTT | 414 | TAAAT | 478 | | |
| ACCCC | 351 | CATCT | 415 | TAATA | 479 | | |
| ACCTT | 352 | CATTC | 416 | TACCT | 480 | | |
| ACTCT | 353 | CCAAA | 417 | TACTC | 481 | | |

Element 3 is a 3 nucleotide sample code that differ by at least two base changes. This element was used to identify different samples and enabled sample multiplexing within a sequencing run. Table 14.

**Table 14**

| **Element** | **Function** | **Number of sequences** | **Sequences (5' -> 3')** | **SEQ ID NO:** |
|---|---|---|---|---|
| Element 3 | Distinct sample labels; sample multiplexing | 16 | AAG | 539 |
| | | | CTC | 540 |
| | | | GGT | 541 |
| | | | TCA | 542 |
| | | | ACT | 543 |
| | | | CGA | 544 |
| | | | GTG | 545 |
| | | | TAC | 546 |
| | | | AGC | 547 |
| | | | CCG | 548 |
| | | | GAA | 549 |
| | | | TTT | 550 |
| | | | ATA | 551 |
| | | | CAT | 552 |
| | | | GCC | 553 |
| | | | TGG | 554 |

Element 4 is a 12 nucleotide anchor sequence with three important characteristics with respect to library construction and downstream sequencing. Table 15. These are A) each 12 base extension is part of a family of four 12 base extensions that collectively represent each of the four possible DNA bases at each site within extension. This feature, balanced base representation, is required by the Illumina sequencing instrument in order to calibrate proper base calling in sequencing reads. B) Each extension is composed of only two of four possible bases, and these are specifically chosen to be either 6 A's + 6 C's or 6 G's + 6 T's. This extension formed from only two bases greatly reduces the possibility that the extension sequence will participate in secondary structure formation that would preclude proper adaptor function. C) Because each extension is composed of equal numbers of A+C or G+T, each extension shares roughly the same melting temperature and duplex stability as every other extension in a set of four.

**Table 15.**

| **Element** | **Function** | **Number of sequences** | **Sequences (5' -> 3')** | **SEQ ID NO:** |
|---|---|---|---|---|
| Element 4 | 12 nucleotide extension that provides a duplexing site for the partner oligonucleotide | 4 | ACCCACACCAAA | 555 |
| | | | CAAACACAACCC | 556 |
| | | | GTGTGGGTTGTT | 557 |
| | | | TGTGTTTGGTGG | 558 |

Element 5 is the two base sequence found at the 3' end of Element 4. The particular two base extensions were chosen based on empirical data that shows that these two base sequences are efficient substrates for ligation. Table 15. 4.

The adaptor module is hybrized to a partner oligonucleotide. Table 16. The hybridization takes place between the sequence within Element 4 and the partner oligonucleotide. The double-stranded adaptor was ligated to end-repaired cfDNA.

**Table 16.**

| **Element** | **Function** | **Number of sequences** | **Sequences (5' -> 3')** | **SEQ ID NO:** |
|---|---|---|---|---|
| Element 4 | 12 nucleotide extension that provides a duplexing site for the partner oligonucleotide | 4 | TTTGGTGTGGGT | 559 |
| | | | GGGTTGTGTTTG | 560 |
| | | | AACAACCCACAC | 561 |
| | | | CCACCAAACACA | 562 |

To convert a set of 256 independently synthesized and pooled ligation strands (each of which shares a common sample code and therefore constitutes a single sample adaptor set) to duplexes suitable for ligation, the 45 nucleotide ligation strand was combined with the appropriate complementary 12 nucleotide partner strand, heated to 95° C, cooled to 65° C for 5 min, then cooled to room temperature. This duplex formed a blunt end ligation substrate as shown in Figure 17B. Following ligation and DNA purification, a DNA polymerase-mediated step that occurs prior to PCR amplification displaced the partner strand and copied the ligation strand to form a double-strand adaptor that was suitable for exponential amplification by single-primer PCR.

The quantitative analysis of genome equivalents derived from targeted sequencing data was then performed. Each unique read was considered a unique ligation event and the sum of unique reads was considered equivalent to the number of genome equivalents analyzed.

A rough, "back-of-the-envelope", "rule-of-thumb" calculation was performed to determine the number of genome equivalents that could be analyzed. Each cfDNA clone was approximately 150 base pairs, 50 base pairs of which were required for binding to capture probes. This left roughly 100 possible sequence start sites within any captured cfDNA clone. The attachment of 249 random codes to each of the 100 possible start sites created a total repertoire of -249,000 possible unique clones. As the number of unique clones approaches the total number of possible sequence combinations, probability dictates that the same code and start site combinations will be created by independent events and that these independent events will be inappropriately grouped within single families. The net result will be an underestimate of genome equivalents analyzed, and rare mutant reads may be discarded as sequencing errors because they overlap with wild-type reads bearing the same identifiers. To avoid this, efforts were made using the qPCR assay to constrain genomic inputs to one tenth or less the number of possible unique clones. For example, a single adaptor has 24,900 possible clones and thus, has a reliable capacity to provide accurate analysis for libraries consisting of 2500 or fewer genome equivalents.

The procedure that is outlined is provided as an example and the methods contemplated herein are not meant to be bound by this example. In some cases, the number of genome equivalents to be analyzed may well exceed the 2500 limit illustrated in the preceding paragraph. To expand the depth of genome equivalents, two solutions to this problem are readily available. The first solution is to use more than one adaptor set per sample. By combining adaptors, it is possible to expand the total number of possible clones and therefore, expand the comfortable limits of genomic input. As a non-limiting example, the combination of four adaptor sets used for one sample would expand the analysis to 24,900 x 4 = 99,600 possible sequences and -10,000 reasonably analyzed genome equivalents. The second solution is to expand the code in Element 2 of Figure 17A to 6, 7, or more bases. The number of possible codes that differ by at least 2 bases from every other code scales as 4⁽ⁿ⁻¹⁾ where n is the number of bases within Element 2. Thus, in the non-limiting example presented here, n = 5 and 4⁽⁵⁻¹⁾ = 256; therefore, the inclusion of additional bases expands the available repertoire by a factor of four for each additional base.

### CONCLUSION

The results from this example showed that two independent methods for the determination of genome equivalents have utility in sample processing workflow. The first method, qPCR, was implemented during the library construction phase of cfDNA analysis and it was used as a quality control step to ensure that adequate numbers of genome equivalents are moved through library amplification, targeted sequence capture, and DNA sequencing. The other method use explicit counting of unique reads as a more direct measure of the actual number of genome equivalents that fell under informatics consideration.

### EXAMPLE 5

### QUANTITATIVE GENETIC ANALYSIS

### PURPOSE

The purpose of this example was to apply quantitative genetic analysis to normal DNA admixed cancer genomes and to uncharacterized cfDNA isolated from the plasma of cancer patients.

### BACKGROUND

Three types of genomic events are prevalent in human cancers. These are somatic mutations that alter the function of the affected gene and its expressed protein product(s); genomic rearrangements that create chimeric gene fusions and therefore expressed fusion proteins with novel biological properties; and changes in gene copy number that lead to gene loss and under expression of gene product(s), or, conversely, amplification of genes and over-representation of the corresponding gene product(s). In the circulating DNA of a cancer patient, these aberrant loci, many of which have critical significance in guiding patient care, are admixed (blended) with the patient's normal, germline DNA.

### SUMMARY

In the previous examples, technology has been described that was configured for the analysis of circulating, cell-free DNA (cfDNA), with an aim toward cancer surveillance. However, the technology is widely applicable to any analytical, diagnostic and monitoring paradigm including, but not limited to r genetic diseases; fetal testing; mendelian disorders; pathogen screening; and organ transplant monitoring in which circulating DNA is a potential analyte. In this example, the technical features highlighted in previous examples are applied to the analysis of admixed cancer samples. In the first phase of this validation, cancer-derived cell lines were admixed with normal human DNA at defined dilutions, and quantitative genetic analysis was performed. In the second phase of this study, uncharacterized cfDNA was isolated from the plasma of cancer patients and subsequently examined using quantitative genetic analysis.

### METHODS

### Admixtures of cell line genomic DNA with normal human DNA

The following DNA samples were used:
- NA06994 - normal human genomic DNA (Coriell repository);
- NCI-H2228 - non-small cell lung cancer cell line (ATCC), harbors mutation in TP53 (Q331*) and EML4-ALK gene fusion (breakpoint unknown); and
- NCI-H69 - small cell lung cancer cell line (ATCC), harbors amplification of the MYCN gene (-100 copies).

***Library preparation:*** Genomic DNA isolated from cell lines (all three above) is high molecular weight material that is dissimilar to the small size of cfDNA. To mimic cfDNA in these validation experiments, genomic DNAs were first fragmented on the "150 bp" setting using a Covaris Acoustic Sonicator. The sonication generally produces a broad smear, and the DNA was further processed using "two sided" bead selection. A dilute solution of DNA purification beads were added to the sample and the higher molecular mass fragments that adhere to the beads were discarded (the size of purified DNA is proportional to the amount of beads added). An additional aliquot of beads are added to the remaining supernatant and in this second round, DNA that adheres to the beads that (in a higher overall concentration of binding buffer) are purified. This "two-sided" purification produces a narrow size distribution that is a reasonable proxy for cfDNA (Figure 18).

Fragmented genomic DNA was end repaired, quantified, and mixed in the various ratios shown in Table 17 and described in the results section below.

**Table 17. Samples and admixtures used for validation studies**

| **Sample, Admixture** | **genome equivalents (qPCR)** |
|---|---|
| Pure H2228 | 2248 |
| NA06994:H2228 4:1 | 2616 |
| NA06994:H2228 10:1 | 2600 |
| NA06994:H2228 20:1 | 2968 |
| NA06994:H2228 50:1 | 5000 |
| NA06994:H2228 1000:1 | 10000 |

| **Sample, Admixture** | **genome equivalents (qPCR)** |
|---|---|
| Pure H69 | 2472 |
| NA06994:H69 4:1 | 2768 |
| NA06994:H69 10:1 | 3088 |
| NA06994:H69 20:1 | 2944 |
| NA06994:H69 40:1 | 1616 |
| NA06994:H69 100:1 | 1920 |
| NA06994:H69 200:1 | 2920 |
| NA06994:H69 500:1 | 17520 |

cfDNA libraries may have limited DNA inputs. The amount of cfDNA obtained per mL of patient plasma is widely variable, but the lower limits (e.g., Example 3) are generally ~10 ng/mL, which is equivalent to 3300 human genomes. To guard against limited cfDNA quantities, the admixture experiments were modeled to reflect the lower limits of cfDNA that were routinely collected from patients. This constraint was applied to all but the most extreme admixtures. In these latter admixtures, libraries were made to mimic inputs from 4 mLs (NA06994:H2228 1000:1) or 8 mLs (NA06994:H69 500:1) of low yield patient cfDNA. Admixed samples were then ligated to the adaptor sets described in Example 4. Measurement of the genome equivalents in each purified library using qPCR (Example 4) is also shown in Table 17. Libraries were amplified, quantified, and equivalent masses of each library were pooled (500 ng of each). The pooled sample was hybridized with the proof of concept, high-density 40 mer capture probes listed in Table 6 of Example 2. The resulting complexes were captured on streptavidin-coated beads, washed, processed, amplified, purified and size-selected as described in previous examples. The resulting library was analyzed using an Illumina 150bp-V3 Miseq sequencing kit on the Illumina MiSeq instrument.

For bioinformatics analysis, a rare somatic variant caller was used to detect mutations, a split read aligner was used to detect fusion genes, and in-house analysis that quantifies and statistically fits tags was used to call copy number variation (CNV).

The detection of an admixed point mutation in the TP53 gene is shown in Figure 19. The "expected" frequency deviates from the admix ratio because it is known that TP53 is hemizygous in the NCI-H2228 cell line. Automated software was able to call the mutant allele in the 50:1 admixture. Manual curation was required to call the mutant event at 1000:1. With respect to specificity, the tag filtering described in Example 1 was applied to the analysis, and no other mutation calls in TP53 were detected after applying this tag filter.

Cell line NCI-H2228 is known to harbor a fusion gene between EML4 and ALK; the cell line serves as a positive control in both fluorescence *in situ* hybridization assays and in detection of fusion gene transcripts using RT-PCR. There are no published reports of the exact location of the gene fusion junction. Using dense probe coverage of the intron 19 region of ALK, sequence analysis revealed precise location and sequence of the junction formed when the two genes fused (Figure 20). The frequency of normal reads versus junction reads in the NCI-H2228 cell line (378 vs 249, respectively) suggests that the fusion gene is heterozygous with a normal copy of ALK.

Detection of junction reads as a function of admixture is shown in Figure 21. As with point mutation detection, the expected values were adjusted to reflect the fact that the mutant allele is found in one copy per diploid genome. No fusion reads were detected in the 1000: 1 admixed sample.

Figure 22 shows the results of CNV determination for the MYCN gene as a function of admixture. The NCI-H69 cell line harbors a highly amplified MYCN gene. MYCN is normally found as a single copy gene, two per diploid genome, and thus the expected result for progressively more dilute admixtures is that the tag-calculated CNV should asymptotically approach 2 copies (the asymptote is highlighted in the figure). The validation experiment shown here indicated that the assay system described in this invention is robustly sensitive to highly amplified genes.

### Variant discovery in cfDNA from cancer patients

The most rigorous validation of the technology contemplated herein is to apply it to cfDNA samples in which the spectrum of mutations is unknown. An analysis was performed by sequencing matched cfDNA, tumor and normal adjacent tissue (NAT) samples from two ovarian cancer patients. In addition, two cfDNA samples from colorectal cancer (CRC) patients and two cfDNA samples from healthy volunteers were analyzed. In no case, were mutations, fusions or abnormal CNV detected in the healthy volunteer samples.

Libraries of cfDNA from the four cancer patients were initially screened using the targeted probes described in Table 6 of Example 2. These probes were primarily configured to detect point mutations in the TP53 gene, gene fusions with ALK and amplifications of the MYCN. The results of this initial sequencing screen are shown in Figure 23. A point mutation occurring at the same base position was found in the cfDNA, tumor, and NAT of one ovarian patient. No TP53 mutations were found in the other set of ovarian cancer patient matched samples. Point mutations were also detected in the two CRC cfDNA libraries for which matching tissues were unavailable. All of these point mutations have been previously identified in tumors and all are known to be causal drivers of tumorogenesis. The mutant sequence detection in cfDNA library CRC406 of 0.9% was well within the scope of assay sensitivity. Sensitivity is defined by the presence of multiple families of tagged reads, all of which possess the mutant sequence. These data highlight the clinical utility of the system contemplated herein.

To further explore the detection of cancer-related changes in cfDNA libraries and associated tissues, the same libraries were hybridized to a set of 679 probes that are directed to a total of 20 different cancer-related genes (Table 18). In this probe set, all of the coding regions of 14 genes were targeted while select loci were targeted in the remaining 6 genes.

**Table 18. Cancer genes targeted**

| **Genes: coding region targeted** | **Genes: select regions targeted** |
|---|---|
| BRCA1 | ALK |
| BRCA2 | DPYD |
| BRAF | EPHX1 |
| CDH1 | MYC |
| ERBB2 | TNFRSF14 |
| JAK2 | ALDH4A1 |
| NF2 | |
| PIK3CA | |
| RB1 | |
| CDKN2A | |
| KRAS | |
| MYCN | |
| PTEN | |
| TP53 | |

As shown in Figure 24, the OVA1 sample, which lacked any detectable changes in TP53, carried a mutation in KRAS that was found in both cfDNA and in the corresponding tumor. This observation highlights a significant feature of the assay system described here. Libraries created from cfDNA can be interrogated with hundreds (as in this example), and even thousands, of targeting probes. The sequencing of the resulting targeted libraries revealed somatic mutations that reside within the tumor and not within the germline of the affected individual. These tumor-associated somatic markers can also be used to quantify the amount of circulating DNA that is shed from the tumor (as opposed to cfDNA that has germline sequence). Thus, the discovery of mutations, regardless of their biological significance, also provides an estimate of tumor content in admixed cfDNA.

Many targeted therapies are most successful in the presence of normal genes (e.g., EGFR inhibitors work only in the presence of wild-type KRAS). A quantitative assessment of circulating tumor DNA levels becomes especially significant in these cases where mutations in genes are not found. In other words, the demonstrable presence of circulating tumor DNA coupled with a wild-type sequencing result at a particular target gene suggests that the target gene is normal within the tumor, and such results can have a significant influence in guiding the choice of therapies. Such is the case with the OVA1 sample highlighted in Figure 24. The presence of the KRAS mutation in the cfDNA library suggested that this patient's tumor harbors a wild-type TP53 gene(s).

Another example of aberrant gene discovery is shown in Figure 25. The targeted quantitative genetic analysis system revealed the presence of a significant amplification in the ERBB2 gene, otherwise referred to as HER-2/neu. While this type of amplification has been much publicized in breast cancer, it is occasionally identified in colorectal carcinomas as well.

### CONCLUSION

Validation experiments with cell line DNA revealed the thresholds of detection of three types of genetic variation that are central to driving neoplastic growth in cancers. Characterization of cfDNA derived from cancer patients revealed tumor-associated genetic changes that were well above the thresholds set by reconstruction experiments in all four samples analyzed. These data indicated that the quantitative analysis system contemplated herein may have significant clinical utility, especially in settings where liquid biopsies are most appropriate.

## Claims

1. A method for targeted genetic analysis of cell-free DNA (cfDNA) comprising:
(a) treating the cfDNA with one or more end-repair enzymes to generate end-repaired cfDNA and ligating an adaptor module to each end of the end-repaired cfDNA to generate a cfDNA library;
(b) amplifying the cfDNA library to generate cfDNA library clones and determining the number of genome equivalents in the cfDNA clone library; and
(c) performing a quantitative genetic analysis of a target genetic locus in the cfDNA library clones comprising:
(i) hybridizing one or more capture probe modules to a target genetic locus in the cfDNA clone library to form one or more capture probe module-cfDNA clone complexes, wherein the target genetic locus is a region of a gene, wherein the capture probe modules comprise capture probes that are 25 to 45 nucleotides in length and hybridize comparably, as well as, or better than capture probes that are 60 nucleotides in length;
(ii) isolating the one or more isolated capture probe module-cfDNA clone complexes from (i);
(iii) enzymatically processing the one or more isolated capture probe module cfDNA clone complexes from (ii), wherein the enzymatic processing comprises performing 5'-3' DNA polymerase extension of the capture probe using the isolated cfDNA clone in the complex as a template;
(iv) performing PCR on the enzymatically processed complex of (iii) to generate amplified hybrid nucleic acid molecules, wherein the amplified hybrid nucleic acid molecules comprise a target sequence capable of hybridizing to the capture probe and the complement of the capture probe module tail sequence; and
(v) sequencing the amplified hybrid nucleic acid molecules of (iv) to generate a plurality of sequencing reads and performing bioinformatics analysis on the plurality of sequencing reads.

2. The method of claim 1, wherein the adaptor module comprises a primer binding site for amplification of the cfDNA library.

3. The method of claim 1 or claim 2, wherein the adaptor module comprises one or more unique read codes.

4. The method of any one of claims 1 to 3, wherein the adaptor module comprises one or more sequences for DNA sequencing.

5. The method of any one of claims 1 to 4, wherein the adaptor module comprises one or more sample codes for sample multiplexing.

6. The method of claim 1, wherein a plurality of capture probe modules are hybridized to the target genetic locus in the cfDNA clone library.

7. The method of claim 1, wherein the targeted genetic analysis is performed on a plurality of target genetic loci.

8. The method of any one of claims 1 to 7, wherein the capture probes are 25 nucleotides, 26 nucleotides, 27 nucleotides, 28 nucleotides, 29 nucleotides, 30 nucleotides, 31 nucleotides, 32 nucleotides, 33 nucleotides, 34 nucleotides, 35 nucleotides, 36 nucleotides, 37 nucleotides, 38 nucleotides, 39 nucleotides, 40 nucleotides, 41 nucleotides, 42 nucleotides, 43 nucleotides, 44 nucleotides, or 45 nucleotides in length.

9. The method of any one of claims 1 to 8, wherein the capture probes are 35 to 45, 36 to 44, 37 to 43, or 38 to 42, or 39 to 41 nucleotides in length.

10. The method of any one of claims 1 to 8, wherein the capture probes are 40 nucleotides.

11. The method of claim 1, wherein the bioinformatics analysis is used to detect one or more genetic lesions within the target genetic locus.

12. The method of claim 11, wherein the one or more genetic lesions comprise a nucleotide transition or transversion, a nucleotide insertion or deletion, a genomic rearrangement, or a change in copy number.

13. The method of any one of claims 1 to 12, wherein the cfDNA is isolated from a biological sample of a subject selected from the group consisting of amniotic fluid, blood, plasma, serum, semen, lymphatic fluid, cerebral spinal fluid, ocular fluid, urine, saliva, stool, mucous, and sweat.

14. The method of any one of claims 1 to 13, wherein the quantitative genetic analysis is used to identify or detect one or more genetic lesions that cause or are associated with a genetic disease.

15. The method of claim 14, wherein the genetic disease is cancer.

16. The method of any one of claims 1 to 15, wherein at least one capture probe module hybridizes downstream of a target region within the target genetic locus and at least one capture probe module hybridizes upstream of the target region.

17. The method of any one of claims 1 to 16, wherein a plurality of capture probes hybridize to the target genetic locus, wherein each of the plurality of capture probes hybridizes to the target genetic locus within about 200 bp of any other capture probe.

## Patentansprüche

1. Verfahren für eine gezielte genetische Analyse zellfreier DNA (cfDNA), umfassend:
(a) Behandeln der cfDNA mit einem oder mehreren Endreparaturenzymen, um endreparierte cfDNA zu erzeugen, und Ligieren eines Adaptormoduls an jedes Ende der endreparierten cfDNA, um eine cfDNA-Bibliothek zu erzeugen;
(b) Amplifizieren der cfDNA-Bibliothek, um cfDNA-Bibliotheksklone zu erzeugen, und Bestimmen der Anzahl von Genomäquivalenten in der cfDNA-Klonbibliothek; und
(c) Durchführen einer quantitativen genetischen Analyse eines genetischen Zielgenlocus in den Klonen der cfDNA-Bibliothek, umfassend:
(i) Hybridisieren eines oder mehrerer Erfassungssondenmodule an einem genetischen Zielgenlocus in der cfDNA-Klonbibliothek zu hybridisieren, um einen oder mehrere Erfassungssondenmodul-cfDNA-Klonkomplexe auszubilden, wobei der genetische Zielgenlocus eine Region eines Gens ist, wobei die Erfassungssondenmodule Erfassungssonden umfassen, die 25 bis 45 Nukleotide lang sind und vergleichbar, so gut wie oder besser als Erfassungssonden hybridisieren, die 60 Nukleotide lang sind;
(ii) Isolieren des einen oder der mehreren isolierten Erfassungssondenmodul-cfDNA-Klonkomplexe aus (i);
(iii) enzymatisches Verarbeiten des einen oder der mehreren isolierten Erfassungssondenmodul-cfDNA-Klonkomplexe aus (ii), wobei das enzymatische Verarbeiten das Durchführen einer 5'-3'-DNA-Polymeraseverlängerung der Erfassungssonde unter Verwendung des isolierten cfDNA-Klons in dem Komplex als eine Vorlage umfasst;
(iv) Durchführen von PCR an dem enzymatisch verarbeiteten Komplex von (iii), um amplifizierte hybride Nukleinsäuremoleküle zu erzeugen, wobei die amplifizierten hybriden Nukleinsäuremoleküle eine Zielsequenz umfassen, die in der Lage ist, an der Erfassungssonde und dem Komplement der Schwanzsequenz des Erfassungssondenmoduls zu hybridisieren; und
(v) Sequenzieren der amplifizierten hybriden Nukleinsäuremoleküle von (iv), um eine Vielzahl von Sequenzierungs-Reads zu erzeugen, und Durchführen einer bioinformatischen Analyse an der Vielzahl von Sequenzierungs-Reads.

2. Verfahren nach Anspruch 1, wobei das Adaptormodul eine Primerbindungsstelle zur Amplifikation der cfDNA-Bibliothek umfasst.

3. Verfahren nach Anspruch 1 oder 2, wobei das Adaptormodul einen oder mehrere eindeutige Read-Codes umfasst.

4. Verfahren nach einem der Ansprüche 1 bis 3, wobei das Adaptormodul eine oder mehrere Sequenzen zur DNA-Sequenzierung umfasst.

5. Verfahren nach einem der Ansprüche 1 bis 4, wobei das Adaptormodul einen oder mehrere Probencodes für eine Proben-Multiplexierung umfasst.

6. Verfahren nach Anspruch 1, wobei eine Vielzahl von Erfassungssondenmodulen an den genetischen Zielgenlocus in der cfDNA-Klonbibliothek hybridisiert werden.

7. Verfahren nach Anspruch 1, wobei die gezielte genetische Analyse an einer Vielzahl von genetischen Zielgenloci durchgeführt wird.

8. Verfahren nach einem der Ansprüche 1 bis 7, wobei die Erfassungssonden 25 Nukleotide, 26 Nukleotide, 27 Nukleotide, 28 Nukleotide, 29 Nukleotide, 30 Nukleotide, 31 Nukleotide, 32 Nukleotide, 33 Nukleotide, 34 Nukleotide, 35 Nukleotide, 36 Nukleotide, 37 Nukleotide, 38 Nukleotide, 39 Nukleotide, 40 Nukleotide, 41 Nukleotide, 42 Nukleotide, 43 Nukleotide, 44 Nukleotide oder 45 Nukleotide lang sind.

9. Verfahren nach einem der Ansprüche 1 bis 8, wobei die Erfassungssonden 35 bis 45, 36 bis 44, 37 bis 43 oder 38 bis 42 oder 39 bis 41 Nukleotide lang sind.

10. Verfahren nach einem der Ansprüche 1 bis 8, wobei die Erfassungssonden 40 Nukleotide sind.

11. Verfahren nach Anspruch 1, wobei die Bioinformatikanalyse verwendet wird, um eine oder mehrere genetische Läsionen innerhalb des genetischen Zielgenlocus zu erkennen.

12. Verfahren nach Anspruch 11, wobei die eine oder mehrere genetischen Läsionen einen Nukleotidübergang oder eine Nukleotidtransversion, eine Nukleotidinsertion oder -deletion, eine genomische Umlagerung oder eine Änderung einer Kopienzahl umfassen.

13. Verfahren nach einem der Ansprüche 1 bis 12, wobei die cfDNA aus einer biologischen Probe eines Subjekts isoliert wird, ausgewählt aus der Gruppe bestehend aus Fruchtwasser, Blut, Plasma, Serum, Sperma, Lymphflüssigkeit, Rückenmarksflüssigkeit, Augenflüssigkeit, Urin, Speichel, Stuhl, Schleim und Schweiß.

14. Verfahren nach einem der Ansprüche 1 bis 13, wobei die quantitative genetische Analyse verwendet wird, um eine oder mehrere genetische Läsionen zu identifizieren oder erkennen, die eine genetische Krankheit verursachen oder mit einer solchen verbunden sind.

15. Verfahren nach Anspruch 14, wobei die genetische Krankheit Krebs ist

16. Verfahren nach einem der Ansprüche 1 bis 15, wobei mindestens ein Erfassungssondenmodul stromabwärts einer Zielregion innerhalb des genetischen Zielgenlocus hybridisiert und mindestens ein Erfassungssondenmodul stromaufwärts der Zielregion hybridisiert.

17. Verfahren nach einem der Ansprüche 1 bis 16, wobei eine Vielzahl von Erfassungssonden an den genetischen Zielgenlocus hybridisiert wird, wobei jede der Vielzahl von Erfassungssonden an dem genetischen Zielgenlocus innerhalb von etwa 200 bp von einer beliebigen anderen Erfassungssonde hybridisiert.

## Revendications

1. Procédé d'analyse génétique ciblée d'ADN acellulaire (ADNcf) comprenant :
(a) le traitement de l'ADNcf avec une ou plusieurs enzymes de réparation des extrémités pour générer un ADNcf à extrémité réparée et la ligature d'un module adaptateur à chaque extrémité de l'ADNcf à extrémité réparée pour générer une banque d'ADNcf ;
(b) l'amplification de la banque d'ADNcf pour générer des clones de banque d'ADNcf et la détermination du nombre d'équivalents génomiques dans la banque de clones d'ADNcf ; et
(c) la réalisation d'une analyse génétique quantitative d'un locus génétique cible dans les clones de banque d'ADNcf comprenant :
(i) l'hybridation d'un ou plusieurs modules de sonde de capture à un locus génétique cible dans la banque de clones d'ADNcf pour former un ou plusieurs complexes de module de sonde de capture-clone d'ADNcf, dans lequel le locus génétique cible est une région d'un gène, dans lequel les modules de sonde de capture comprennent des sondes de capture d'une longueur comprise entre 25 et 45 nucléotides et s'hybridant de manière comparable, aussi bien ou mieux que les sondes de capture d'une longueur de 60 nucléotides ;
(ii) l'isolement du ou des complexes isolés de module de sonde de capture - clone d'ADNcf de (i) ;
(iii) le traitement par voie enzymatique du ou des complexes isolés de module de sonde de capture-clone d'ADNcf à partir de (ii), dans lequel le traitement enzymatique comprend la réalisation d'extension 5'-3' de l'ADN polymérase de la sonde de capture à l'aide du clone d'ADNcf isolé dans le complexe en tant que modèle ;
(iv) la réalisation d'une PCR sur le complexe traité par voie enzymatique de (iii) pour générer des molécules d'acide nucléique hybrides amplifiées, dans lequel les molécules d'acide nucléique hybrides amplifiées comprennent une séquence cible capable de s'hybrider à la sonde de capture et au complément de la séquence de queue du module de sonde de capture ; et
(v) le séquençage des molécules d'acide nucléique hybride amplifiées de (iv) pour générer une pluralité de lectures de séquençage et la réalisation d'une analyse bioinformatique sur la pluralité de lectures de séquençage.

2. Procédé selon la revendication 1, dans lequel le module adaptateur comprend un site de liaison d'amorce pour l'amplification de la banque ADNcf.

3. Procédé selon la revendication 1 ou la revendication 2, dans lequel le module adaptateur comprend un ou plusieurs codes de lecture uniques.

4. Procédé selon l'une quelconque des revendications 1 à 3, dans lequel le module adaptateur comprend une ou plusieurs séquences pour le séquençage de l'ADN.

5. Procédé selon l'une quelconque des revendications 1 à 4, dans lequel le module adaptateur comprend un ou plusieurs codes d'échantillons pour le multiplexage des échantillons.

6. Procédé selon la revendication 1, dans lequel une pluralité de modules de sondes de capture sont hybridés au locus génétique cible dans la banque de clones d'ADNcf.

7. Procédé selon la revendication 1, dans lequel l'analyse génétique ciblée est réalisée sur une pluralité de loci génétiques cibles.

8. Procédé selon l'une quelconque des revendications 1 à 7, dans lequel les sondes de capture ont une longueur de 25 nucléotides, 26 nucléotides, 27 nucléotides, 28 nucléotides, 29 nucléotides, 30 nucléotides, 31 nucléotides, 32 nucléotides, 33 nucléotides, 34 nucléotides, 35 nucléotides, 36 nucléotides, 37 nucléotides, 38 nucléotides, 39 nucléotides, 40 nucléotides, 41 nucléotides, 42 nucléotides, 43 nucléotides, 44 nucléotides ou 45 nucléotides.

9. Procédé selon l'une quelconque des revendications 1 à 8, dans lequel les sondes de capture ont une longueur de 35 à 45, 36 à 44, 37 à 43, ou 38 à 42, ou 39 à 41 nucléotides.

10. Procédé selon l'une quelconque des revendications 1 à 8, dans lequel les sondes de capture sont 40 nucléotides.

11. Procédé selon la revendication 1, dans lequel l'analyse bioinformatique est utilisée pour détecter une ou plusieurs lésions génétiques dans le locus génétique cible.

12. Procédé selon la revendication 11, dans lequel la ou les lésions génétiques comprennent une transition ou une transversion nucléotidique, une insertion ou une délétion nucléotidique, un réarrangement génomique ou un changement du nombre de copies.

13. Procédé selon l'une quelconque des revendications 1 à 12, dans lequel l'ADNcf est isolé à partir d'un échantillon biologique d'un sujet choisi dans le groupe constitué de liquide amniotique, sang, plasma, sérum, sperme, liquide lymphatique, liquide céphalo-rachidien, liquide oculaire, urine, salive, selles, muqueuses et sueur.

14. Procédé selon l'une quelconque des revendications 1 à 13, dans lequel l'analyse génétique quantitative est utilisée pour identifier ou détecter une ou plusieurs lésions génétiques qui causent une maladie génétique ou sont associées à celle-ci.

15. Procédé selon la revendication 14, dans lequel la maladie génétique est le cancer.

16. Procédé selon l'une quelconque des revendications 1 à 15, dans lequel au moins un module de sonde de capture s'hybride en aval d'une région cible dans le locus génétique cible et au moins un module de sonde de capture s'hybride en amont de la région cible.

17. Procédé selon l'une quelconque des revendications 1 à 16, dans lequel une pluralité de sondes de capture s'hybride avec le locus génétique cible, dans lequel chacune de la pluralité des sondes de capture s'hybride avec le locus génétique cible à environ 200 pb d'une autre sonde de capture quelconque.
